# EUROPÄISCHE PATENTSCHRIFT

(11) **EP 2 029 586 B1**
(45) Veröffentlichungstag und Bekanntmachung des Hinweises auf die Patenterteilung: **24.11.2010**
(21) Anmeldenummer: 07725602.2
(22) Anmeldetag: 26.05.2007
(51) Int. Cl.: C07D 413/14

(54) **ISOINDOLIN-1-ON-, ISOINDOLIN-3-ON- UND ISOINDOLIN-1,3-DION-DERIVATE UND IHRE VERWENDUNG**
ISOINDOLIN-1-ONE-, ISOINDOLIN-3-ONE- AND ISOINDOLIN-1,3-DIONE-DERIVATIVES AND USE THEREOF
DÉRIVÉS D'ISOINDOLIN-1-ONE, D'ISOINDOLIN-3-ONE ET D' ISOINDOLIN-1,3-DIONE ET LEUR UTILISATION

(30) Priorität: 31.05.2006 DE 102006025317
(43) Veröffentlichungstag der Anmeldung: 04.03.2009
(73) Patentinhaber: Bayer Schering Pharma Aktiengesellschaft, 13353 Berlin (DE)
(72) Erfinder: RÖHRIG, Susanne, 40724 Hilden (DE); JESKE, Mario, 42699 Solingen (DE); PERZBORN, Elisabeth, 42327 Wuppertal (DE); GNOTH, Mark Jean, 40822 Mettmann (DE)
(86) Internationale Anmeldenummer: PCT/EP2007/004706
(87) Internationale Veröffentlichungsnummer: WO 2007/137800

(56) Entgegenhaltungen:
- WO-A-03/007942
- WO-A-03/011858

## Beschreibung

Die Erfindung betrifft neue Isoindolin-1-on-, Isoindolin-3-on- und Isoindolin-1,3-dion-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet. Die aktivierte Serinprotease Xa spaltet Prothrombin zu Thrombin. Das entstandene Thrombin wiederum spaltet seinerseits Fibrinogen zu Fibrin. Durch anschließende Quervernetzung der Fibrin-Monomere kommt es zur Bildung von Blutgerinnseln und damit zur Blutstillung. Darüber hinaus ist Thrombin ein potenter Auslöser der Thrombozytenaggregation, die ebenfalls einen erheblichen Beitrag bei der Hämostase leistet.

Die Hämostase unterliegt einem komplexen Regulationsmechanismus. Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thromboembolischen Erkrankungen führen. Darüber hinaus kann eine Hyperkoagulabilität - systemisch - bei einer Verbrauchskoagulopathie zur disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen.

Thromboembolische Erkrankungen sind die häufigste Ursache von Morbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prophylaxe von thromboembolischen Erkrankungen erweist sich in der Praxis deshalb als sehr schwierig und unbefriedigend.

Für die Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"].

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21S; J. Ansell, J. Hirsh, J. Dalen et al., "Managing oral anticoagulant therapy" Chest 2001, 119, 22S-38S; P.S. Wells, A.M. Holbrook, N.R. Crowther et al., "Interactions of warfarin with drugs and food" Ann. Intern. Med. 1994, 121, 676-683].

In jüngster Zeit ist ein neuer Therapieansatz für die Behandlung und Prophylaxe von thromboembolischen Erkrankungen beschrieben worden. Ziel dieses neuen Therapieansatzes ist die Inhibierung von Faktor Xa. Entsprechend der zentralen Rolle, die Faktor Xa in der Blutgerinnungskaskade spielt, stellt Faktor Xa eines der wichtigsten Targets für antikoagulatorische Wirkstoffe dar [J. Hauptmann, J. Stürzebecher, Thrombosis Research 1999, 93, 203; S.A.V. Raghavan, M. Dikshit, "Recent advances in the status and targets of antithrombotic agents" Drugs Fut. 2002, 27, 669-683; H.A. Wieland, V. Laux, D. Kozian, M. Lorenz, "Approaches in anticoagulation: Rationales for target positioning" Curr. Opin. Investig. Drugs 2003, 4, 264-271; U.J. Ries, W. Wienen, "Serine proteases as targets for antithrombotic therapy" Drugs Fut. 2003, 28, 355-370; L.-A. Linkins, J.I. Weitz, "New anticoagulant therapy" Annu. Rev. Med. 2005, 56, 63-77; A. Casimiro-Garcia et al., "Progress in the discovery of Factor Xa inhibitors" Expert Opin. Ther. Patents 2006, 15, 119-145].

Dabei ist gezeigt worden, dass verschiedene, sowohl peptidische wie nicht-peptidische Verbindungen in Tiermodellen als Faktor Xa-Inhibitoren wirksam sind. Eine große Anzahl von direkten Faktor Xa-Inhibitoren ist bislang bekannt [J.M. Walenga, W.P. Jeske, D. Hoppensteadt, J. Fareed, "Factor Xa Inhibitors: Today and beyond" Curr. Opin. Investig. Drugs 2003, 4, 272-281; J. Ruef, H.A. Katus, "New antithrombotic drugs on the horizon" Expert Opin. Investig. Drugs 2003, 12, 781-797; M.L. Quan, J.M. Smallheer, "The race to an orally active Factor Xa inhibitor: Recent advances" Curr. Opin. Drug Discovery & Development 2004, 7, 460-469]. Weiterhin sind nicht-peptidische, niedermolekulare Faktor Xa-Inhibitoren beispielsweise auch in WO 03/099276, WO 03/011858 und WO 03/007942 beschrieben.

Eine Aufgabe der vorliegenden Erfindung ist die Bereitstellung neuer alternativer Verbindungen mit vergleichbarer oder verbesserter Wirkung und besserer Löslichkeit in wässrigen Lösungen, zur Bekämpfung von Erkrankungen, insbesondere von thromboembolischen Erkrankungen, bei Menschen und Tieren.

Gegenstand der Erfindung sind Verbindungen der Formel 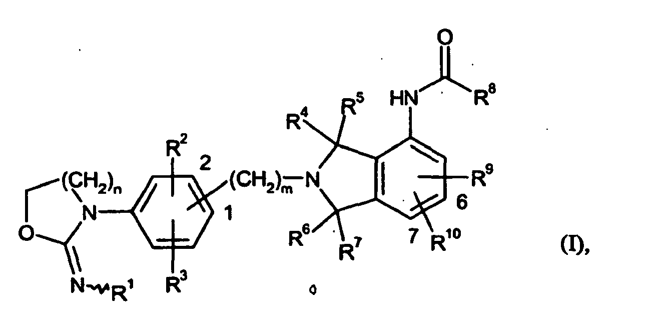 in welcher
- n: für die Zahl 1, 2 oder 3 steht,
- m: für die Zahl 0, 1 oder 2 steht,
- R¹: und die (CH₂)ₘ-Gruppe in 1 oder 2 Position an den Phenyl-Ring gebunden ist, für Wasserstoff, Cyano, Hydroxy, C₁-C₄-Alkyl, C₁-C₄-Alkylcarbonyl, C₃-C₆-Cycloalkyl- carbonyl, Phenylcarbonyl, 4- bis 7-gliedriges Heterocyclylcarbonyl oder 5- oder 6-glie- driges Heteroarylcarbonyl steht,
- R²: für Wasserstoff, Fluor, Chlor, Cyano, Hydroxy, Amino, Trifluormethyl, Trifluormethoxy, C₁-C₄-Alkyl, C₁-C₄-Alkoxy, C₁-C₄-Alkoxymethyl, C₁-C₄-Alkylamino, C₃-C₆-Cycloalkyl, Aminocarbonyl, C₁-C₄-Alkoxycarbonyl oder C₁-C₄-Alkylaminocarbonyl steht,
- R³: für Wasserstoff, Fluor, Chlor, Cyano, Hydroxy, Amino, Trifluormethyl, Trifluormethoxy, C₁-C₄-Alkyl, C₁-C₄-Alkoxy, C₁-C₄-Alkoxymethyl, C₁-C₄-Alkylamino, C₃-C₆-Cycloalkyl, Aminocarbonyl, C₁-C₄-Alkoxycarbonyl oder C₁-C₄-Alkylaminocarbonyl steht,
- R⁴ und R⁵: für Wasserstoff stehen, und
und
- R⁶ und R⁷: zusammen mit dem Kohlenstoffatom, an das sie gebunden sind, eine Carbonyl- gruppe bilden,
oder
- R⁴ und R⁵: zusammen mit dem Kohlenstoffatom, an das sie gebunden sind, eine Carbonyl- gruppe bilden,
und
- R⁶ und R⁷: für Wasserstoff stehen,
oder
- R⁴ und R⁵: zusammen mit dem Kohlenstoffatom, an das sie gebunden sind, eine Carbonyl- gruppe bilden,
und
- R⁶ und R⁷: zusammen mit dem Kohlenstoffatom, an das sie gebunden sind, eine Carbonyl- gruppe bilden,
- R⁸: für Phenyl, Pyridyl, Pyrazinyl, Pyrimidinyl, Pyridazinyl oder Thienyl steht,
wobei Phenyl, Pyridyl, Pyrazinyl, Pyrimidinyl und Pyridazinyl substituiert sind mit einem Substituenten R¹¹ und/oder einem Substituenten R¹² oder mit zwei verschiedenen Substituenten R¹¹ oder mit zwei verschiedenen Substituenten R¹²,
wobei
R¹¹ an ein Kohlenstoffatom gebunden ist, das nicht einem Stickstoffatom im Ring benachbart ist, und für Wasserstoff, Fluor, Chlor, Cyano, Ethinyl, C₁- C₄-Alkyl, C₁-C₄-Alkoxy oder C₃-C₆-Cycloalkyl steht,
R¹² an ein Kohlenstoffatom gebunden ist, das einem Stickstoffatom im Ring benachbart ist, und für Wasserstoff, Amino, C₁-C₄-Alkyl, C₁-C₄- Alkylamino oder C₃-C₆-Cycloalkyl steht,
und wobei
Thienyl substituiert ist mit einem Substituenten R¹³ und einem Substituenten R¹⁴,
wobei
R¹³ an ein Kohlenstoffatom gebunden ist, das dem Schwefelatom im Ring benachbart ist, und für Wasserstoff, Fluor, Chlor, Cyano, Ethinyl, C₁-C₄- Alkyl, C₁-C₄-Alkoxy oder C₃-C₆-Cycloalkyl steht, R¹⁴ für Wasserstoff, Fluor, Chlor, Amino, C₁-C₄-Alkyl, C₁-C₄-Alkylamino oder C₃-C₆-Cycloalkyl steht,
- R⁹: für Wasserstoff, Fluor, Chlor, Cyano, Hydroxy, Amino, Trifluormethyl, Trifluormethoxy, C₁-C₄-Alkyl, C₁-C₄-Alkoxy, C₁-C₄-Alkylamino, C₃-C₆-Cycloalkyl, Aminocarbonyl, C₁-C₄- Alkoxycarbonyl oder C₁-C₄-Alkylaminocarbonyl steht,
- R¹⁰: für Wasserstoff, Fluor, Chlor, Cyano, Trifluormethyl, Trifluormethoxy, C₁-C₄-Alkyl, C₁-C₄-Alkoxy oder C₃-C₆-Cycloalkyl steht,
und
wobei R⁹ an die 6-Position und R¹⁰ an die 7-Position des Isoindolin-Rings gebunden ist,
oder
wobei R⁹ an die 7-Position und R¹⁰ an die 6-Position des Isoindolin-Rings gebunden ist,
und ihre Salze, ihre Solvate und die Solvate ihrer Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassen, nachfolgend als Ausfuhrungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfmdungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkoxycarbonyl und Alkylaminocarbonyl steht für einen linearen oder verzweigten Alkylrest mit in der Regel 1 bis 4, bevorzugt 1 oder 2 Kohlenstoffatomen, beispielhaft und vorzugsweise für Methyl, Ethyl, n-Propyl, Isopropyl und *tert*.-Butyl.

Alkoxy steht beispielhaft und vorzugsweise für Methoxy, Ethoxy, n-Propoxy, Isopropoxy und *tert*.-Butoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*.-Butylamino, *N,N*-Dimethylamino, *N,N*-Diethylamino, *N*-Ethyl-*N-*methylamino, *N*-Methyl-*N*-n-propylamino, *N*-Isopropyl-*N*-n-propylamino und *N*-*tert.*-Butyl-*N-*methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkoxycarbonyl steht beispielhaft und vorzugsweise für Methoxycarbonyl, Ethoxycarbonyl, n-Propoxycarbonyl, Isopropoxycarbonyl und *tert*.-Butoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, *tert*.-Butylaminocarbonyl, *N,N*-Dimethylaminocarbonyl, *N,N*-Diethylaminocarbonyl, *N*-Ethyl-*N*-methylaminocarbonyl, *N-*Methyl-*N*-n-propylaminocarbonyl, *N*-iso-Propyl-*N*-n-propylaminocarbonyl und *N*-*tert.*-Butyl-*N-*methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Cycloalkyl steht für eine Cycloalkylgruppe mit in der Regel 3 bis 6 Kohlenstoffatomen, bevorzugt mit 3 bis 5 Kohlenstoffatomen, beispielhaft und vorzugsweise für Cyclopropyl, Cyclobutyl, Cyclopentyl und Cyclohexyl.

Heterocyclyl steht für einen monocyclischen, heterocyclischen Rest mit in der Regel 4 bis 7 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- bis 7-gliedrige, monocyclische gesättigte Heterocyclylreste mit bis zu zwei Heteroatomen aus der Reihe O, N und S, wie beispielhaft und vorzugsweise Tetrahydrofuranyl, Pyrrolidinyl, Pyrrolinyl, Piperidinyl, Tetrahydropyranyl, Piperazinyl, Morpholinyl und Perhydroazepinyl.

Heteroaryl steht für einen aromatischen, monocyclischen Rest mit 5 oder 6 Ringatomen und bis zu 4 Heteroatomen aus der Reihe S, O und N, beispielhaft und vorzugsweise für Thienyl, Furyl, Pyrrolyl, Thiazolyl, Oxazolyl, Isoxazolyl, Isothiazolyl, Imidazolyl, Pyrazolyl, Pyridyl, Pyrimidinyl, Pyridazinyl und Pyrazinyl.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

In den Formeln der Gruppe, die für R⁸ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R⁸ gebunden ist.

Bevorzugt sind Verbindungen der Formel (I), in welcher
- n: für die Zahl 1, 2 oder 3 steht,
- m: für die Zahl 0, 1 oder 2 steht, und die (CH₂)ₘ-Gruppe in 1 oder 2 Position an den Phenyl-Ring gebunden ist,
- R¹: für Wasserstoff, Cyano, Hydroxy oder C₁-C₄-Alkyl steht.
- R² für: Wasserstoff, Fluor, Chlor, Cyano, Hydroxy, C₁-C₄-Alkyl oder C₁-C₄-Alkoxy steht,
- R³ für: Wasserstoff, Fluor, Chlor, Cyano, Hydroxy, C₁-C₄-Alkyl, C₁-C₄-Alkoxy, C₁-C₄- Alkoxymethyl, Cyclopropyl, Aminocarbonyl, C₁-C₄-Alkoxycarbonyl oder C₁-C₄-Alkyl- aminocarbonyl steht,
- R⁴ und R⁵: für Wasserstoff stehen, und
- R⁶ und R⁷ oder: zusammen mit dem Kohlenstoffatom, an das sie gebunden sind, eine Carbonyl- gruppe bilden,
- R⁴ und R⁵: zusammen mit dem Kohlenstoffatom, an das sie gebunden sind, eine Carbonyl- gruppe bilden, und
- R⁶ und R⁷: für Wasserstoff stehen, oder
- R⁴ und R⁵: zusammen mit dem Kohlenstoffatom, an das sie gebunden sind, eine Carbonyl- gruppe bilden, und
- R⁶ und R⁷: zusammen mit dem Kohlenstoffatom, an das sie gebunden sind, eine Carbonyl- gruppe bilden,
- R⁸: für eine Gruppe der Formel 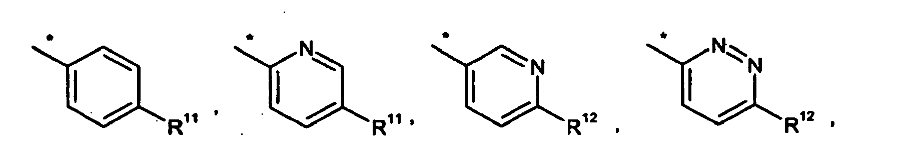 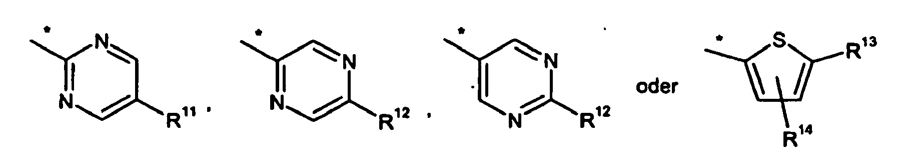 steht,
wobei
* die Anknüpfstelle an die Carbonylgruppe ist,
R¹¹ für Fluor, Chlor, Ethinyl, Methyl, Ethyl, Methoxy oder Ethoxy steht,
R¹² für Amino, Methyl, Methylamino oder Dimethylamino steht,
R¹³ und für Fluor, Chlor, Ethinyl, Methyl, Ethyl, Methoxy oder Ethoxy steht,
R¹⁴ für Wasserstoff steht,
- R⁹: für Wasserstoff, Fluor, Chlor, Cyano, Methyl, Methoxy, Aminocarbonyl, Methylamino- carbonyl oder Dimethylaminocarbonyl steht,
- R¹⁰: für Wasserstoff, Fluor, Chlor, Cyano, Trifluormethyl, Trifluormethoxy, Methyl oder Methoxy steht,
und
wobei R⁹ an die 6-Position und R¹⁰ an die 7-Position des Isoindolin-Rings gebunden ist,
oder
wobei R⁹ an die 7-Position und R¹⁰ an die 6-Position des Isoindolin-Rings gebunden ist,
und ihre Salze, ihre Solvate und die Solvate ihrer Salze.

Bevorzugt sind auch Verbindungen der Formel (I), in welcher
- n: für die Zahl 1 oder 2 steht,
- m: für die Zahl 1 steht,
und die (CH₂)ₘ-Gruppe in 1 oder 2 Position an den Phenyl-Ring gebunden ist,
- R¹: für Wasserstoff steht,
- R²: für Wasserstoff steht,
- R³: fürWasserstoff, Fluor, Chlor, Cyano, Methyl, Ethyl, n-Propyl, Methoxy, Ethoxy oder Methoxymethyl steht,
- R⁴ und R⁵: für Wasserstoff stehen,
und
- R⁶ und R⁷: zusammen mit dem Kohlenstoffatom, an das sie gebunden sind, eine Carbonyl- gruppe bilden,
oder
- R⁴ und R⁵: zusammen mit dem Kohlenstoffatom, an das sie gebunden sind, eine Carbonyl- gruppe bilden,
und
- R⁶ und R⁷: für Wasserstoff stehen,
oder
- R⁴ und R⁵: zusammen mit dem Kohlenstoffatom, an das sie gebunden sind, eine Carbonyl- gruppe bilden,
und
- R⁶ und R⁷: zusammen mit dem Kohlenstoffatom, an das sie gebunden sind, eine Carbonyl- gruppe bilden,
- R⁸: für eine Gruppe der Formel 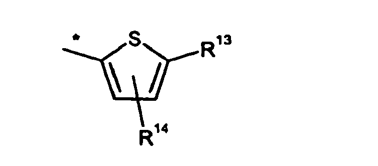 steht,
wobei
* die Anknüpfstelle an die Carbonylgruppe ist,
R¹³ für Fluor, Chlor oder Methyl steht,
und
R¹⁴ für Wasserstoff steht,
- R⁹: für Wasserstoff steht,
- R¹⁰: für Wasserstoff steht,
und
wobei R⁹ an die 6-Position und R¹⁰ an die 7-Position des Isoindolin-Rings gebunden ist,
oder
wobei R⁹ an die 7-Position und R¹⁰ an die 6-Position des Isoindolin-Rings gebunden ist,
und ihre Salze, ihre Solvate und die Solvate ihrer Salze.

Bevorzugt sind auch Verbindungen der Formel (I), in welcher
- n: für die Zahl 1 steht,
- m: für die Zahl 1 steht, und die (CH₂)ₘ-Gruppe in 1 oder 2 Position an den Phenyl-Ring gebunden ist,
- R¹: für Wasserstoff steht,
- R²: für Wasserstoff steht,
- R³: für Wasserstoff, Fluor, Chlor, Cyano oder Methyl steht,
- R⁴ und R⁵: für Wasserstoff stehen, und
- R⁶ und R⁷: zusammen mit dem Kohlenstoffatom, an das sie gebunden sind, eine Carbonyl- gruppe bilden,
oder
- R⁴ und R⁵: zusammen mit dem Kohlenstoffatom, an das sie gebunden sind, eine Carbonyl- gruppe bilden,
und
- R⁶ und R⁷: für Wasserstoff stehen,
oder
- R⁴ und R⁵: zusammen mit dem Kohlenstoffatom, an das sie gebunden sind, eine Carbonylgruppe bilden,
und
- R⁶ und R⁷: zusammen mit dem Kohlenstoffatom, an das sie gebunden sind, eine Carbonylgruppe bilden,
- R⁸: für eine Gruppe der Formel 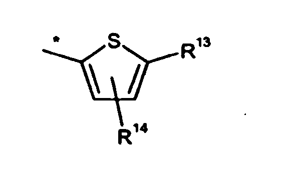 steht,
wobei
* die Anknüpfstelle an die Carbonylgruppe ist,
R¹³ für Chlor steht,
und
R¹⁴ für Wasserstoff steht,
- R⁹: für Wasserstoff steht,
- R¹⁰: für Wasserstoff steht,
und
wobei R⁹ an die 6-Position und R¹⁰ an die 7-Position des Isoindolin-Rings gebunden ist,
oder
wobei R⁹ an die 7-Position und R¹⁰ an die 6-Position des Isoindolin-Rings gebunden ist,
und ihre Salze, ihre Solvate und die Solvate ihrer Salze.

Bevorzugt sind auch Verbindungen der Formel (I), in welcher n für die Zahl 1 steht.

Bevorzugt sind auch Verbindungen der Formel (I), in welcher m für die Zahl 1 steht.

Bevorzugt sind auch Verbindungen der Formel (I), in welcher R¹ für Wasserstoff steht.

Bevorzugt sind auch Verbindungen der Formel (I), in welcher R² für Wasserstoff steht.

Bevorzugt sind auch Verbindungen der Formel (I), in welcher R³ für Wasserstoff, Fluor, Chlor, Cyano oder Methyl steht.

Bevorzugt sind auch Verbindungen der Formel (I), in welcher R³ für Wasserstoff steht.

Bevorzugt sind auch Verbindungen der Formel (I), in welcher R² und R³ für Wasserstoff stehen.

Bevorzugt sind auch Verbindungen der Formel (I), in welcher R⁸ für eine Gruppe der Formel 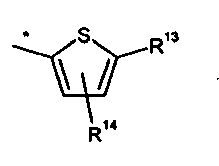 steht, wobei * die Anknüpfstelle an die Carbonylgruppe ist, R¹³ für Chlor steht und R¹⁴ für Wasserstoff steht.

Bevorzugt sind auch Verbindungen der Formel (I), in welcher R⁹ und R¹⁰ für Wasserstoff stehen.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 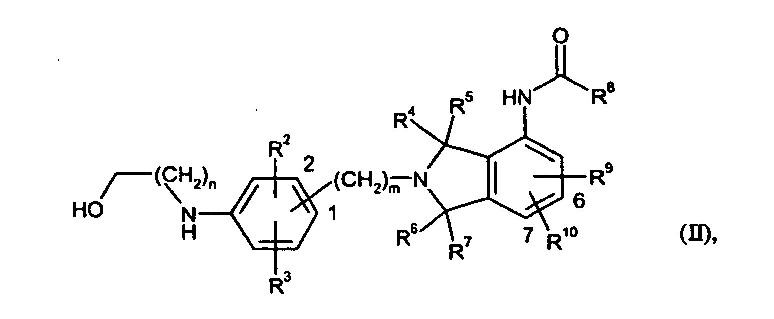 in welcher n, m, R², R³, R⁴, R⁵, R⁶, R⁷, R⁸, R⁹ und R¹⁰ die oben angegebene Bedeutung haben,
   in einem inerten Lösungsmittel in Gegenwart einer Säure mit Bromcyan zu Verbindungen der Formel (I), in welcher R¹ für Wasserstoff steht, umgesetzt werden,
   oder
[B] die Verbindungen der Formel 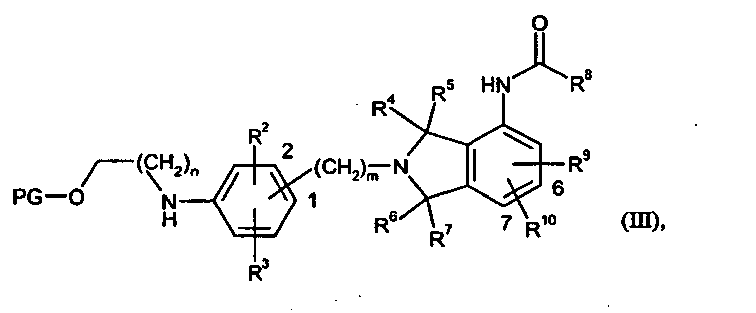 in welcher n, m, R², R³, R⁴, R⁵, R⁶, R⁷, R⁸, R⁹ und R¹⁰ die oben angegebene Bedeutung haben, und
   - PG: für eine Hydroxy-Schutzgruppe, vorzugsweise für Trimethylsilyl oder *tert*.-Butyldimethylsilyl, steht,
   in einem dreistufigen Verfahren zuerst in einem inerten Lösungsmittel mit Bromcyan, vorzugsweise in Gegenwart einer Base, zu Verbindungen der Formel 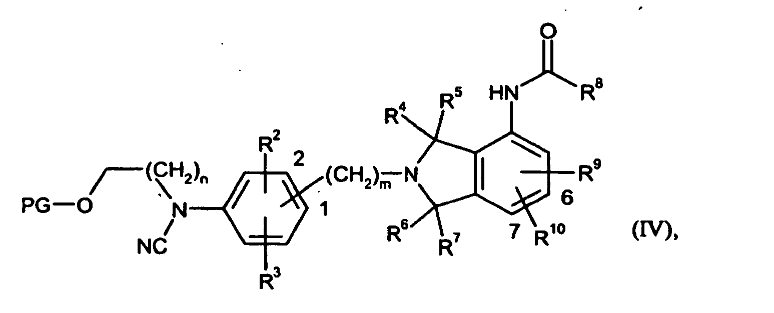 in welcher n, m, R², R³, R⁴, R⁵, R⁶, R⁷, R⁸, R⁹ und R¹⁰ die oben angegebene Bedeutung haben, und
   - PG: für eine Hydroxy-Schutzgruppe, vorzugsweise für Trimethylsilyl oder tert.-Butyldimethyl- silyl, steht,
   und anschließend durch Abspaltung der Schutzgruppe PG zu Verbindungen der Formel 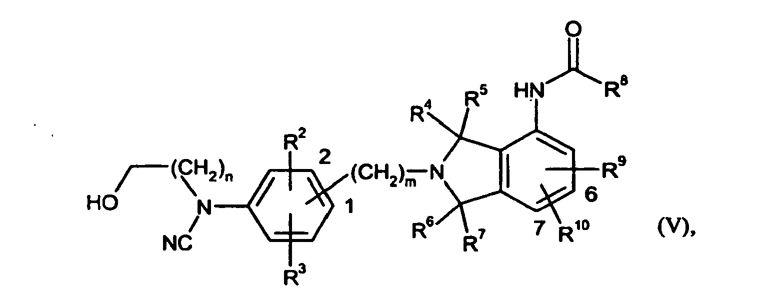 in welcher n, m, R², R³, R⁴, R⁵, R⁶, R⁷, R⁸, R⁹ und R¹⁰ die oben angegebene Bedeutung haben,
   umgesetzt werden und in der dritten Stufe die Verbindungen der Formel (V) in einem inerten Lösungsmittel in Gegenwart einer Säure zu Verbindungen der Formel (I), in welcher R¹ für Wasserstoff steht, cyclisiert werden,
   oder
[C] die Verbindungen der Formel (II) in der ersten Stufe mit Verbindungen der Formel 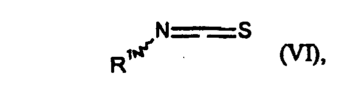 in welcher
   - R¹: für C₁-C₄-Alkyl, C₁-C₄-Alkylcarbonyl, C₃-C₆-Cycloalkylcarbonyl, Phenylcarbonyl, 4- bis 7- gliedriges Heterocyclylcarbonyl oder 5- oder 6-gliedriges Heteroarylcarbonyl steht,
   umgesetzt werden und in der zweiten Stufe cyclisiert werden,
   oder
[D] die Verbindungen der Formel (II) mit Verbindungen der Formel 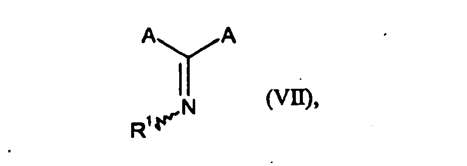 in welcher
   - R¹: für Cyano oder C₁-C₄-Alkyl steht, und
   - A: für eine Abgangsgruppe, bevorzugt Phenoxy oder Methylthio, steht,
   umgesetzt werden,
   oder
[E] die Verbindungen der Formel (I), in welcher R¹ für Wasserstoff steht, mit Hydroxylamin-Hydrochlorid zu Verbindungen der Formel (I), in welcher R¹ für Hydroxy steht, umgesetzt werden.

Die Verbindungen der Formel (I), in welcher R¹ für Wasserstoff steht, können gegebenenfalls mit den entsprechenden Lösungsmitteln und/oder Basen oder Säuren zu ihren Salzen, ihren Solvaten und/oder den Solvaten ihrer Salze umgesetzt werden.

Die freie Base der Salze kann zum Beispiel durch Chromatographie an einer Reversed Phase Säule mit einem Acetonitril-Wasser-Gradienten unter Zusatz einer Base erhalten werden, insbesondere durch Verwendung einer RP18 Phenomenex Luna C18(2) Säule und Diethylamin als Base, oder durch Lösen der Salze in einem organischen Lösungsmittel und Ausschütteln mit wässrigen Lösungen von basischen Salzen wie Natriumhydrogencarbonat.

Weiterer Gegenstand der Erfindung ist ein Verfahren zur Herstellung der Verbindungen der Formel (I) oder ihrer Solvate, bei dem Salze der Verbindungen oder Solvate der Salze der Verbindungen durch Chromatographie unter Zusatz einer Base in die Verbindungen überführt werden.

Die Umsetzung nach Verfahren [A] erfolgt im Allgemeinen in inerten Lösungsmitteln, bevorzugt in einem Temperaturbereich von -20°C bis 50°C bei Normaldruck.

Inerte Lösungsmittel sind beispielsweise Tetrahydrofuran, Dichlormethan oder Acetonitril oder Gemische dieser Lösungsmittel.

Säuren sind beispielsweise starke anorganische oder organische Säuren wie Fluorwasserstoff, Chlorwasserstoff, Bromwasserstoff, Methansulfonsäure, Trifluormethansulfonsäure oder Trifluoressigsäure.

Die Umsetzung der ersten Stufe nach Verfahren [B] erfolgt im Allgemeinen in inerten Lösungsmitteln, bevorzugt in einem Temperaturbereich von -20°C bis 50°C bei Normaldruck.

Inerte Lösungsmittel sind beispielsweise Tetrahydrofuran, Dichlormethan oder Acetonitril oder Gemische dieser Lösungsmittel.

Basen sind beispielsweise anorganische Basen wie Alkali- oder Erdalkalicarbonate oder -hydrogencarbonate wie Lithium-, Natrium-, Kalium-, Calcium- oder Cäsiumcarbonat oder Natrium- oder Kaliumhydrogencarbonat, oder Alkalihydride wie Natriumhydrid.

Die Abspaltung von Trimethylsilyl oder *tert*.-Butyldimethylsilyl als bevorzugt verwendete Hydroxy-Schutzgruppen (PG) in der zweiten Stufe nach Verfahren [B] erfolgt im Allgemeinen in Tetrahydrofuran als Lösungsmittel, vorzugsweise mit Hilfe von Tetra-n-butylammoniumfluorid (TBAF), bevorzugt in einem Temperaturbereich von 0°C bis 40°C bei Normaldruck.

Die Umsetzung der dritten Stufe nach Verfahren [B] erfolgt im Allgemeinen in inerten Lösungsmitteln, bevorzugt in einem Temperaturbereich von -20°C bis 50°C bei Normaldruck.

Inerte Lösungsmittel sind beispielsweise Tetrahydrofuran, Dichlormethan oder Acetonitril oder Gemische dieser Lösungsmittel.

Säuren sind beispielsweise starke anorganische oder organische Säuren wie Fluorwasserstoff, Chlorwasserstoff, Bromwasserstoff, Methansulfonsäure, Trifluormethansulfonsäure oder Trifluoressigsäure.

Die Umsetzung der zweiten und dritten Stufe nach Verfahren [B] erfolgt besonders bevorzugt unter Verwendung einer säurelabilen Hydroxy-Schutzgruppe, wie beispielsweise Trimethylsilyl oder *tert*.-Butyldimethylsilyl, in Gegenwart eines Überschusses einer Säure als Eintopf-Reaktion, in inerten Lösungsmitteln, bevorzugt in einem Temperaturbereich von -20°C bis 50°C bei Normaldruck, ohne Isolierung der Zwischenstufe der Verbindungen der Formel (V).

Inerte Lösungsmittel sind beispielsweise Tetrahydrofuran, Dichlormethan oder Acetonitril oder Gemische dieser Lösungsmittel.

Säuren sind beispielsweise starke anorganische oder organische Säuren wie Fluorwasserstoff, Chlorwasserstoff, Bromwasserstoff, Methansulfonsäure, Trifluormethansulfonsäure oder Trifluoressigsäure.

Die Umsetzung der ersten Stufe nach Verfahren [C] erfolgt im Allgemeinen in Analogie zu literaturbekannten Verfahren, wie beschrieben in z. B. A. Hetenyi et al., J. Org. Chem. 2003, 68, 2175-2182; D. Douglass, J. Am. Chem. Soc. 1934, 56, 719; F.B. Dains et al., J. Am. Chem. Soc. 1925, 47, 1981-1989 oder F.B. Dains et al., J. Am. Chem. Soc. 1922, 44, 2637-2643.

Die Umsetzung der zweiten Stufe nach Verfahren [C] erfolgt im Allgemeinen in Analogie zu literaturbekannten Verfahren, wie beschrieben in z. B. T. Shibanuma, M. Shiono, T. Mukaiyama, Chem. Lett. 1977, 575-576.

Die Umsetzung nach Verfahren [D] erfolgt im Allgemeinen in Analogie zu literaturbekannten Verfahren, wie beschrieben in z. B. N. Maezaki, A. Furusawa, S. Uchida, T. Tanaka, Tetrahedron 2001, 57, 9309-9316; G. Berecz, J. Reiter, G. Argay, A. Kalman, J. Heterocycl. Chem. 2002, 39, 319-326; R. Evers, M. Michalik, J. Prakt. Chem. 1991, 333, 699-710; R. Mohr, A. Buschauer, W. Schunack, Arch. Pharm. (Weinheim Ger.) 1988, 321, 221-227; P. J. Garratt et al., Tetrahedron 1989, 45, 829-834 oder V.A. Vaillancourt et al., J. Med. Chem. 2001, 44, 1231-1248.

Die Umsetzung nach Verfahren [E] erfolgt im Allgemeinen in Analogie zu literaturbekannten Verfahren, wie beschrieben in z. B. G. Zinner, G. Nebel, Arch. Pharm. Ber. Dtsch. Ges. 1970, 303, 385-390.

Die Verbindungen der Formeln (VI) und (VII) sind bekannt oder lassen sich nach bekannten Verfahren aus den entsprechenden Ausgangsverbindungen synthetisieren.

Die Verbindungen der Formel (III) sind bekannt oder können hergestellt werden, aus den Verbindungen der Formel (II) durch Einführung der Schutzgruppe PG nach dem Fachmann bekannten Bedingungen.

Die Einführung von Trimethylsilyl oder *tert*.-Butyldimethylsilyl als bevorzugt verwendete Hydroxy-Schutzgruppen (PG) erfolgt im Allgemeinen durch Umsetzung mit Trimethylsilylchlorid oder tert.-Butyldimethylsilylchlorid in Tetrahydrofuran oder Dimethylformamid als Lösungsmittel, vorzugsweise in Gegenwart von Imidazol, bevorzugt in einem Temperaturbereich von 0°C bis 40°C bei Normaldruck.

Die Verbindungen der Formel (IIa), in denen R⁴ und R⁵ zusammen mit dem Kohlenstoffatom, an das sie gebunden sind, eine Carbonylgruppe bilden, und R⁶ und R⁷ zusammen mit dem

Kohlenstoffatom, an das sie gebunden sind, eine Carbonylgruppe bilden, sind bekannt oder können hergestellt werden, indem Verbindungen der Formel 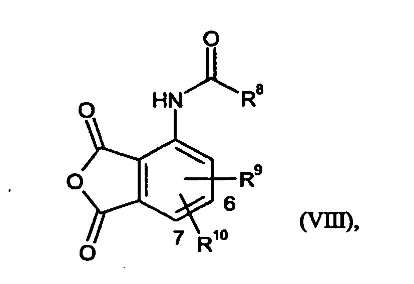 in welcher R⁸, R⁹ und R¹⁰ die oben angegebene Bedeutung haben,
mit Verbindungen der Formel 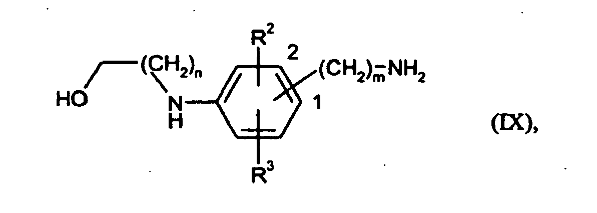 in welcher n, m, R² und R³ die oben angegebene Bedeutung haben,
umgesetzt werden.

Die Umsetzung erfolgt im Allgemeinen in inerten Lösungsmitteln, in Gegenwart einer Base, bevorzugt in einem Temperaturbereich von 60°C bis zum Rückfluss des Lösungsmittels bei Normaldruck.

Inerte Lösungsmittel sind beispielsweise Ether wie Dioxan oder Tetrahydrofuran, bevorzugt ist Dioxan.

Basen sind beispielsweise Aminbasen wie Triethylamin oder Diisopropylethylamin, bevorzugt ist Diisopropylethylamin.

Die Verbindungen der Formeln (VIII) und (IX) sind bekannt oder lassen sich nach bekannten Verfahren aus den entsprechenden Ausgangsverbindungen synthetisieren.

Die Verbindungen der Formel (IIIa), in denen R⁴ und R⁵ zusammen mit dem Kohlenstoffatom, an das sie gebunden sind, eine Carbonylgruppe bilden, und R⁶ und R⁷ zusammen mit dem

Kohlenstoffatom, an das sie gebunden sind, eine Carbonylgruppe bilden, sind bekannt oder können hergestellt werden, indem Verbindungen der Formel (VIII) mit Verbindungen der Formel 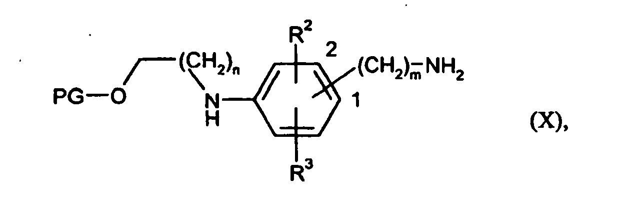 in welcher n, m, R², R³ und PG die oben angegebene Bedeutung haben,
umgesetzt werden.

Die Umsetzung erfolgt unter denselben Reaktionsbedingungen wie die Umsetzung der Verbindungen der Formel (VIII) mit Verbindungen der Formel (IX).

Die Verbindungen der Formel (X) sind bekannt oder lassen sich nach bekannten Verfahren aus den entsprechenden Ausgangsverbindungen synthetisieren.

Die Verbindungen der Formel (IIb), in denen R⁴ und R⁵ für Wasserstoff stehen und R⁶ und R⁷ zusammen mit dem Kohlenstoffatom, an das sie gebunden sind, eine Carbonylgruppe bilden, und die Verbindungen der Formel (IIc), in denen R⁴ und R⁵ zusammen mit dem Kohlenstoffatom, an das sie gebunden sind, eine Carbonylgruppe bilden und R⁶ und R⁷ für Wasserstoff stehen, sind bekannt oder können hergestellt werden, indem Verbindungen der Formel (IIa) in der ersten Stufe mit einem Borhydrid zu einem Gemisch der Verbindungen der Formeln 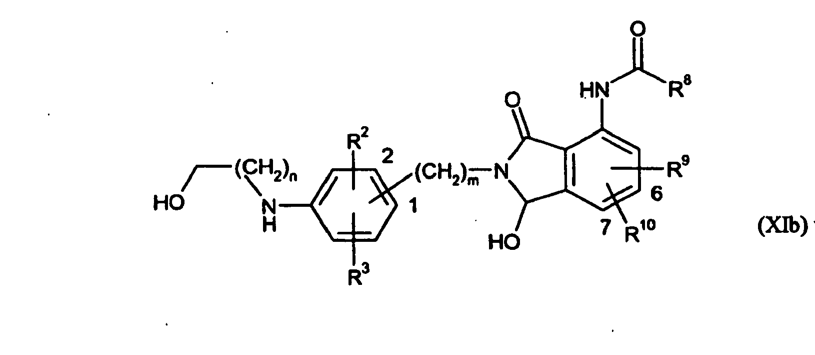 und 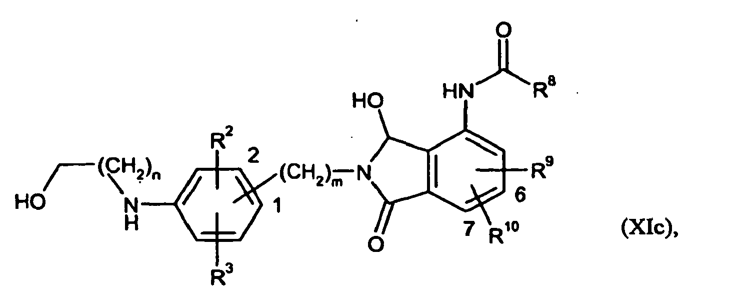 in welchen n, m, R², R³, R⁸, R⁹ und R¹⁰ die oben angegebene Bedeutung haben,
umgesetzt werden,
dieses Gemisch in der zweiten Stufe mit Trifluoressigsäure und Triethylsilan zu einem Gemisch der Verbindungen der Formeln 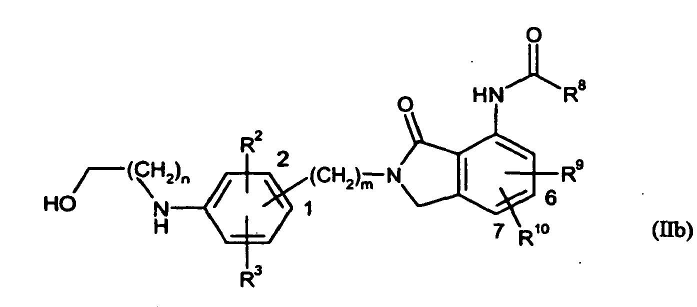 und 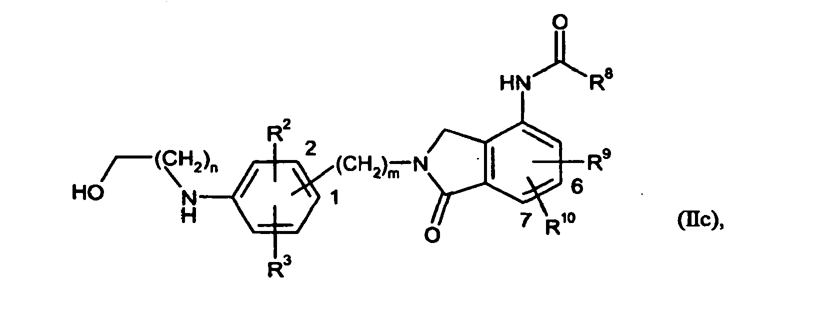 in welchen n, m, R², R³, R⁸, R⁹ und R¹⁰ die oben angegebene Bedeutung haben,
umgesetzt wird,
und die Isomere (IIb) und (IIc) anschließend durch Kristallisation oder Chromatographie getrennt werden.

Die Verbindungen der Formel (IIb) kristallisieren im Allgemeinen aus der Lösung aus und die Verbindungen der Formel (IIc) bleiben in der Mutterlauge zurück.

Die Trennung der Isomere kann auch schon nach der ersten Stufe durch Kristallisation oder Chromatographie erfolgen. In der zweiten Stufe wird dann das reine Isomer eingesetzt.

Die Umsetzung der ersten Stufe erfolgt im Allgemeinen in inerten Lösungsmitteln, bevorzugt in einem Temperaturbereich von -20°C bis 50°C bei Normaldruck.

Borhydride sind beispielsweise Natriumborhydrid oder Lithiumborhydrid, bevorzugt ist Natriumborhydrid.

Inerte Lösungsmittel sind beispielsweise Halogenkohlenwasserstoffe wie Methylenchlorid oder Trichlormethan, Alkohole wie Methanol, Ethanol, n-Propanol oder Isopropanol, oder Ether wie Dietylether, Dioxan oder Tetrahydrofuran, oder Gemische dieser Lösungsmittel, bevorzugt ist ein Gemisch aus Methanol und Methylenchlorid.

Die Umsetzung der zweiten Stufe erfolgt im Allgemeinen in inerten Lösungsmitteln, bevorzugt in einem Temperaturbereich von -20°C bis 50°C bei Normaldruck.

Inerte Lösungsmittel sind beispielsweise Halogenkohlenwasserstoffe wie Methylenchlorid oder Trichlormethan, bevorzugt ist Methylenchlorid.

In einem alternativen Verfahren können die Verbindungen der Formeln (IIb) und (IIc) hergestellt werden, indem in der ersten Stufe Verbindungen der Formel 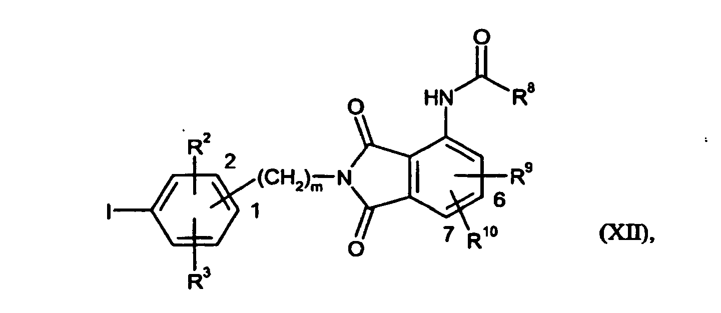 in welcher m, R², R³, R⁸, R⁹ und R¹⁰ die oben angegebene Bedeutung haben,
mit einem Borhydrid zu einem Gemisch der Verbindungen der Formeln 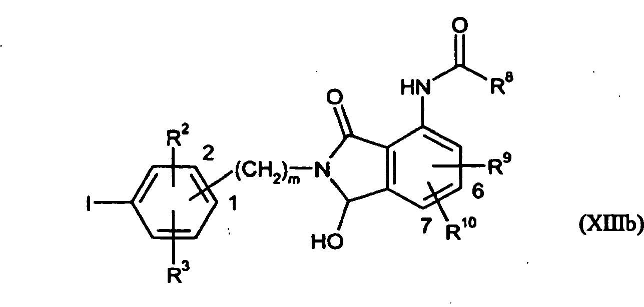 und 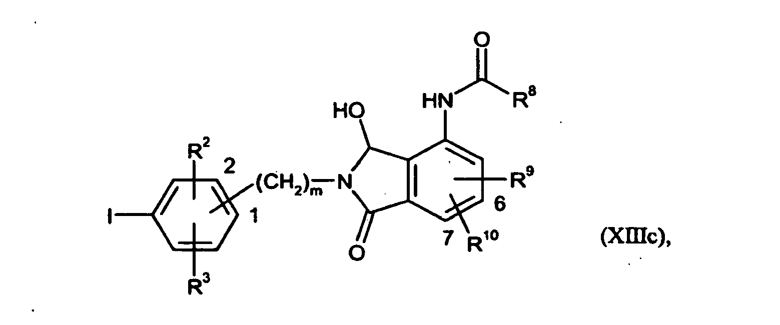 in welchen m, R², R³, R⁸, R⁹ und R¹⁰ die oben angegebene Bedeutung haben,
umgesetzt werden,
die Isomere (XIIIb) und (XIIIc) durch Kristallisation oder Chromatographie getrennt werden, und anschließend jedes Isomer einzeln in der zweiten Stufe mit Trifluoressigsäure und Triethylsilan und in der dritten Stufe mit Verbindungen der Formel 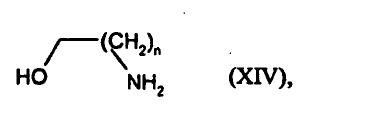 in welcher n die oben angegebene Bedeutung hat,
umgesetzt werden.

Die Umsetzung der ersten Stufe erfolgt unter denselben Reaktionsbedingungen wie die Umsetzung der Verbindungen der Formel (IIa) zu Verbindungen der Formeln (XIb) und (XIc).

Die Umsetzung der zweiten Stufe erfolgt unter denselben Reaktionsbedingungen wie die Umsetzung der Verbindungen der Formeln (XIb) und (XIc) zu Verbindungen der Formeln (IIb) und (IIc).

Die Umsetzung der dritten Stufe erfolgt im Allgemeinen in inerten Lösungsmitteln unter Zugabe eines Kupfer(I)-Salzes, einer Base und eines Diol-Liganden, bevorzugt in einem Temperaturbereich von 60°C bis zum Rückfluss des Lösungsmittels bei Normaldruck.

Inerte Lösungsmittel sind beispielsweise Alkohole wie iso-Propanol oder n-Butanol.

Kupfer(I)-Salze sind beispielsweise Kupfer(I)-iodid, Kupfer(I)-bromid, Kupfer(I)-chlorid oder Kupfer(I)-acetat, bevorzugt ist Kupfer(I)-iodid oder Kupfer(I)-acetat.

Basen sind beispielsweise Kaliumphosphat oder Cäsiumcarbonat, bevorzugt ist Kaliumphosphat.

Diol-Liganden sind beispielsweise 1,2-Diole wie Ethylenglycol.

Die Verbindungen der Formel (XIV) sind bekannt oder lassen sich nach bekannten Verfahren aus den entsprechenden Ausgangsverbindungen synthetisieren.

Die Verbindungen der Formel (XII) sind bekannt oder können hergestellt werden, indem Verbindungen der Formel (VIII) mit Verbindungen der Formel 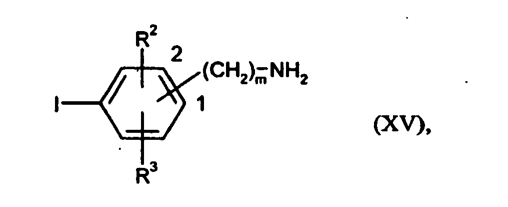 in welcher m, R² und R³ die oben angegebene Bedeutung haben,
umgesetzt werden.

Die Umsetzung erfolgt unter denselben Reaktionsbedingungen wie die Umsetzung der Verbindungen der Formel (VIII) mit Verbindungen der Formel (IX).

Die Verbindungen der Formel (XV) sind bekannt oder lassen sich nach bekannten Verfahren aus den entsprechenden Ausgangsverbindungen synthetisieren.

In einem alternativen Verfahren können die Verbindungen der Formel (XII) hergestellt werden, indem Verbindungen der Formel 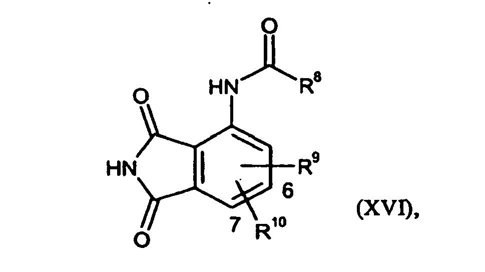 in welcher R⁸, R⁹ und R¹⁰ die oben angegebene Bedeutung haben,
mit Verbindungen der Formel 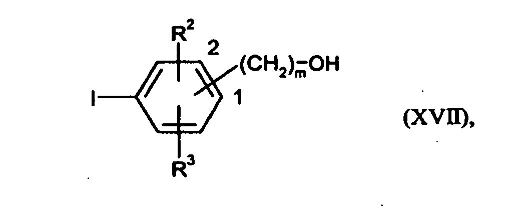 in welcher m, R² und R³ die oben angegebene Bedeutung haben,
unter Mitsunobu-Reaktionsbedingungen umgesetzt werden.

Die Umsetzung erfolgt im Allgemeinen in inerten Lösungsmitteln, bevorzugt in einem Temperaturbereich von -20°C bis 40°C bei Normaldruck.

Inerte Lösungsmittel sind beispielsweise Tetrahydrofuran, Dioxan, Dimethylformamid und Dichlormethan, bevorzugt ist Tetrahydrofuran.

Die Verbindungen der Formeln (XVI) und (XVII) sind bekannt oder lassen sich nach bekannten Verfahren aus den entsprechenden Ausgangsverbindungen synthetisieren.

In einem alternativen Verfahren können die Verbindungen der Formel (IIb) hergestellt werden, indem Verbindungen der Formel 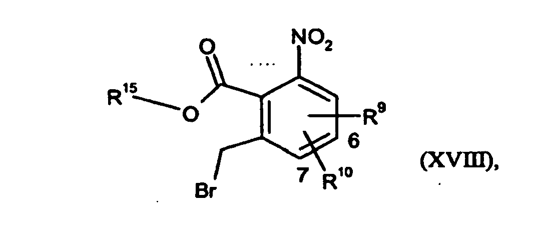 in welcher R⁹ und R¹⁰ die oben angegebene Bedeutung haben, und
- R¹⁵: für Methyl oder Ethyl steht,
in der ersten Stufe mit Verbindungen der Formel (XV) umgesetzt werden,
in der zweiten Stufe die Nitrogruppe reduziert wird und
in der dritten Stufe mit Verbindungen der Formel 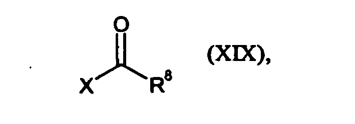 in welcher R⁸ die oben angegebene Bedeutung hat, und
- X: für Halogen, bevorzugt Brom oder Chlor, oder Hydroxy steht,
umgesetzt werden.

Die Umsetzung der ersten Stufe erfolgt unter denselben Reaktionsbedingungen wie die Umsetzung der Verbindungen der Formel (VIII) mit Verbindungen der Formel (IX).

Die Reduktion der Nitrogruppe in der zweiten Stufe erfolgt im Allgemeinen mit einem Reduktionsmitteln in inerten Lösungsmitteln, bevorzugt in einem Temperaturbereich von Raumtemperatur bis zum Rückfluß der Lösungsmittel bei Normaldruck bis 3 bar.

Reduktionsmittel sind beispielsweise Palladium auf Aktivkohle und Wasserstoff, Zinndichlorid oder Titantrichlorid, bevorzugt ist Palladium auf Aktivkohle und Wasserstoff oder Zinndichlorid.

Inerte Lösungsmittel sind beispielsweise Ether wie Diethylether, Methyl-*tert*.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Methanol, Ethanol, iso-Propanol oder im Falle von Zinndichlorid in Dimethylformamid.

Falls in der dritten Stufe X für Halogen steht, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Pyridin oder Dimethylformamid.

Basen sind beispielsweise Triethylamin, Diisopropylethylamin oder N-Methylmorpholin, bevorzugt ist Diisopropylethylamin.

Falls in der dritten Stufe X für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N,'*-Dipropyl-, *N,N'*-Diisopropyl-, *N,N'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N,N'*-tetra-methyluronium-hexafluorophosphat (HBTU), 2-(2-Oxo-1-(2H)-pyridyl)-1,1,3,3-tetramethyluroniumtetrafluoro-borat (TPTU) oder *O-*(7-Azabenzotriazol-1-yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formeln (XVIII) und (XIX) sind bekannt oder lassen sich nach bekannten Verfahren aus den entsprechenden Ausgangsverbindungen synthetisieren.

In einem alternativen Verfahren können die Verbindungen der Formel (IIc) hergestellt werden, wie im alternativen Verfahren für Verbindungen der Formel (IIb) beschrieben. Ausgangsverbindungen sind Verbindungen der Formel 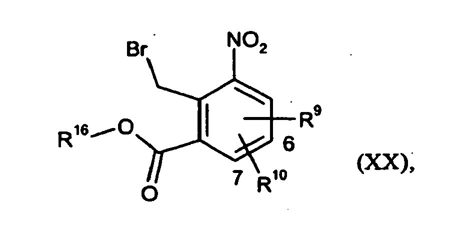 in welcher R⁹ und R¹⁰ die oben angegebene Bedeutung haben, und
- R¹⁶: für Methyl oder Ethyl steht.

Die Verbindungen der Formel (XX) sind bekannt oder lassen sich nach bekannten Verfahren aus den entsprechenden Ausgangsverbindungen synthetisieren.

Die Herstellung der erfindungsgemäßen Verbindungen kann durch die folgenden Syntheseschemata veranschaulicht werden:

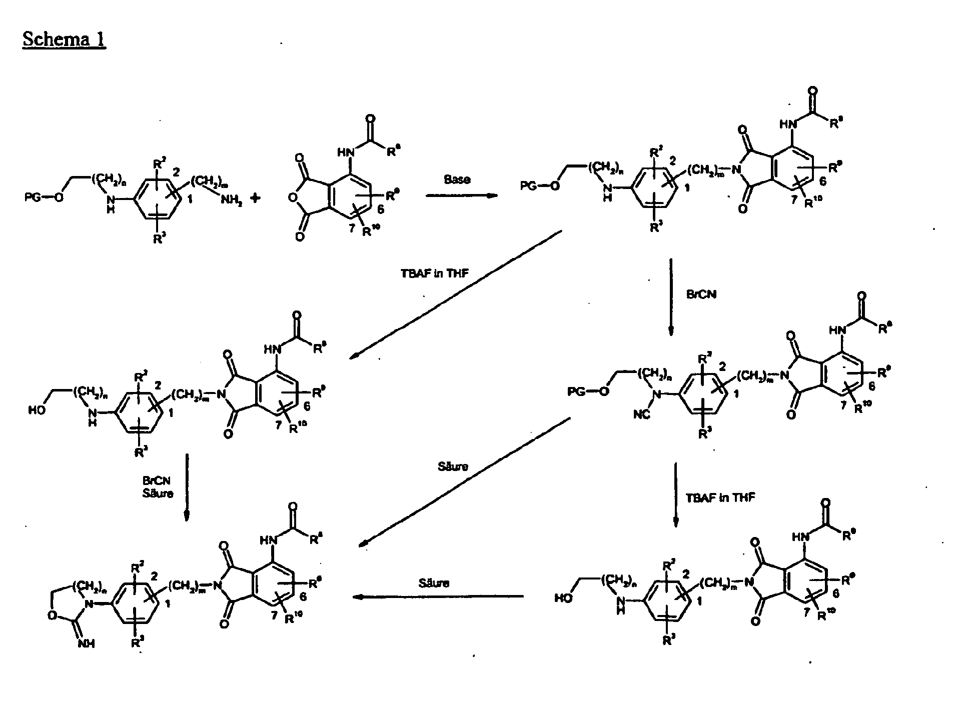

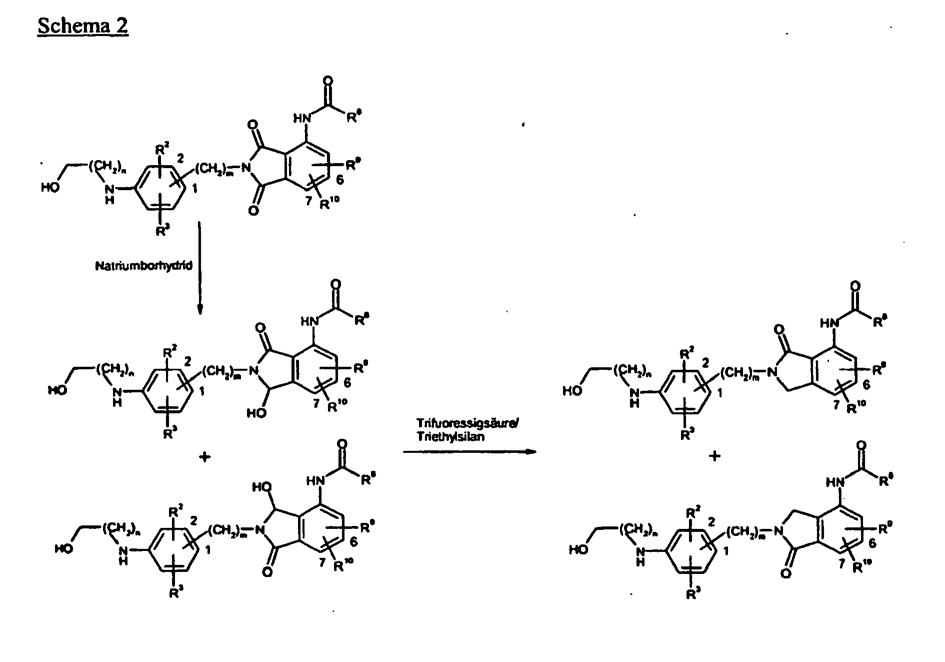

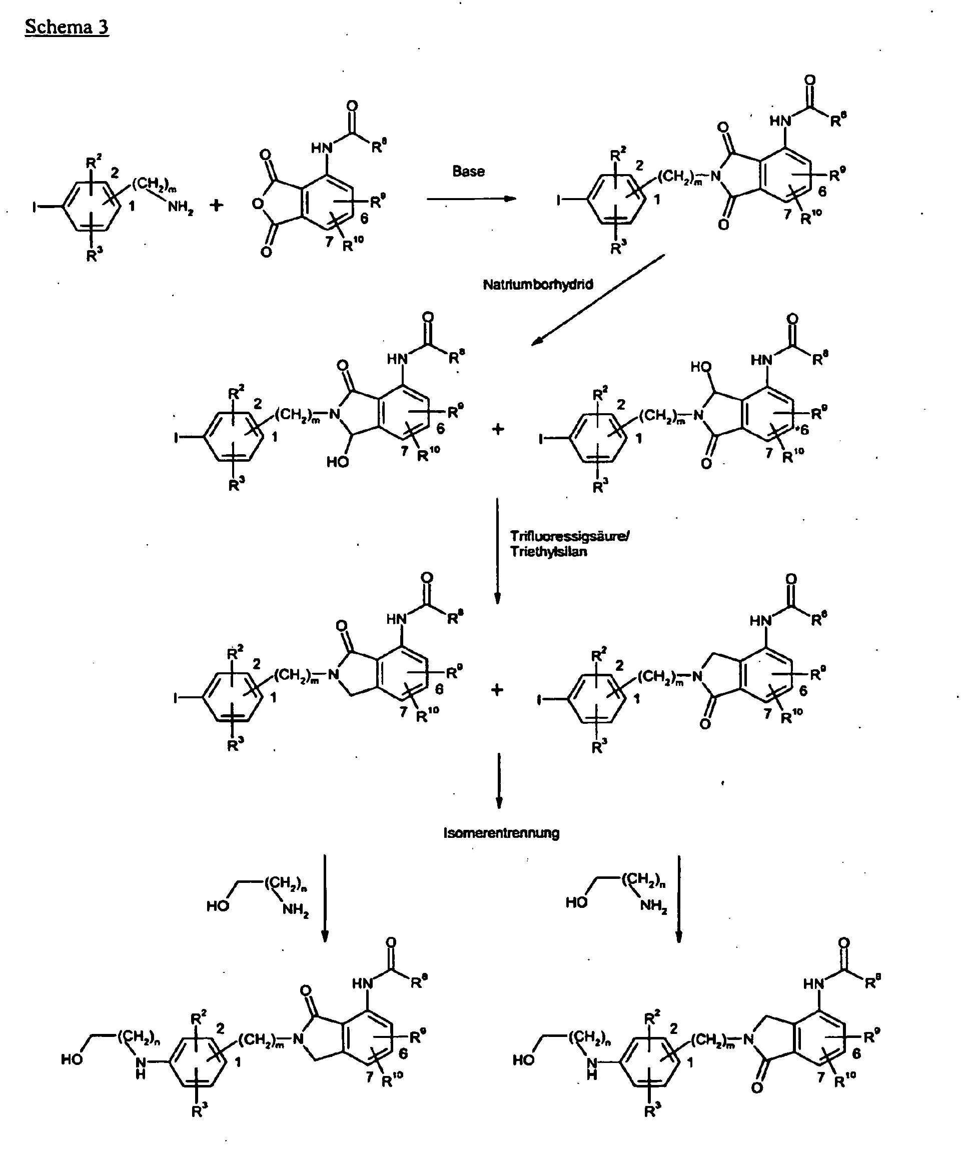

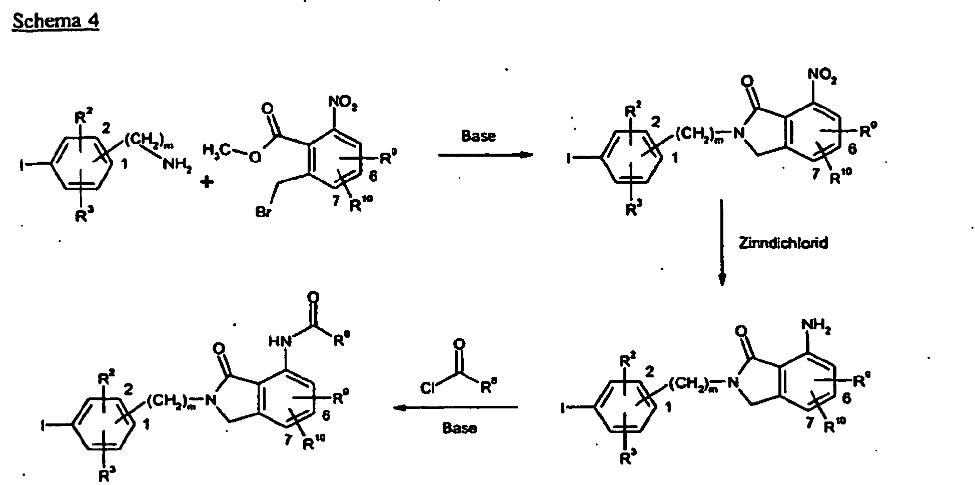

Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Bei den erfindungsgemäßen Verbindungen handelt es sich um selektive Inhibitoren des Blutgerinnungsfaktors Xa, die insbesondere als Antikoagulantien wirken.

Darüber hinaus verfügen die erfindungsgemäßen Verbindungen über günstige physikochemische Eigenschaften, wie beispielsweise eine gute Löslichkeit in Wasser und physiologischen Medien, was für ihre therapeutische Anwendung von Vorteil ist.

Weiterer Gegenstand der vorliegenden Erfindung ist der Einsatz der erfindungsgemäßen Verbindungen zur Herstelllung von Medikamenten zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Herstelllung von Medikamenten zur Prävention und Behandlung von kardiogenen Thromboembolien, wie beispielsweise Him-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus sind die erfindungsgemäßen Verbindungen zur Herstelllung von Medikamenten zur Behandlung der disseminierten intravasalen Gerinnung (DIC) geeignet.

Thromboembolische Komplikationen treten ferner auf bei mikroangiopathischen hämolytischen Anärnien, extrakorporalen Blutkreisläufen, wie Hämodialyse, sowie Herzklappenprothesen.

Außerdem kommen die erfindungsgemäßen Verbindungen auch zur Herstelllung von Medikamenten für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembotischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die erfindungsgemäßen Verbindungen können darüber hinaus auch zur Herstelllung von Medikamenten zür Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfmdungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Herstelllung von Medikamenten zur Behandlung und/oder Prophylaxe von Erkrankungen, insbesondere der zuvor genannten Erkrankungen, unter Verwendung einer antikoagulatorisch wirksamen Menge der erfindungsgemäßen Verbindung.

Weiterer Gegenstand der vorliegenden Erfindung ist die Herstelllung von Medikamenten für ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfmdungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasrninogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozytenaggregationshemmer);
- Fibrinogen-Rezeptor-Antagonisten (Glycoprotein-IIb/IIIa-Antagonisten);
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- DC: Dünnschicht-Chromatographie
- DCI: direkte chemische Ionisation (bei MS)
- DMF: *N,N*-Dimethylformamid
- DMSO: Dimethylsulfoxid
- d: Tag(e)
- d. Th.: der Theorie (bei Ausbeute)
- ee: Enantiomerenüberschuss
- eq.: Äquivalent(e)
- ESI: Elektrospray-Ionisation (bei MS)
- h: Stunde(n)
- HPLC: Hochdruck-, Hochleistungsflüssigchromatographie
- LC-MS: Flüssigchromatographie-gekoppelte Massenspektroskopie
- min: Minute(n)
- MS: Massenspektroskopie
- NMR: Kernresonanzspektroskopie
- RP: reverse phase (bei HPLC)
- RT: Raumtemperatur
- R₁: Retentionszeit (bei HPLC)
- TBTU: *O*-(Benzotriazol-1-yl)-*N,N,N',N'*-tetramethyluronium-tetrafluoroborat
- THF: Tetrahydrofuran

### LC-MS- und HPLC-Methoden

Methode 1: Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

Methode 2: Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

Methode 3: Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 208-400 nm.

Methode 4: Instrument: Micromass Platform LCZ mit HPLC Agilent Serie 1100; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

Methode 5: Instrument: Micromass Platform LCZ mit HPLC Agilent Serie 1100; Säule: Thermo HyPURTTY Aquastar 3µ 50 mm x 2.1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

Methode 6: Gerätetyp MS: Micromass ZQ; Gerätetyp HPLC: Waters Alliance 2795; Säule: Merck Chromolith SpeedROD RP-18e 50 mm x 4.6 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 10% B → 3.0 min 95% B → 4.0 min 95% B; Ofen: 35°C; Fluss: 0.0 min 1.0 ml/min → 3.0 min 3.0 ml/min → 4.0 min 3.0 ml/min; UV-Detektion: 210 nm.

Methode 7: Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%ige Ameisensäure, Eluent B: 1 l Acetonitril + 0.5 ml 50%ige Ameisensäure; Gradient: 0.0 min 900/ob → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 8: Instrument: Micromass Quattro LCZ mit HPLC Agilent Serie 1100; Säule: Phenomenex Gemini 3µ 30 mm x 3.00 mm; Eluent A: 1 l Wasser + 0.5 ml 50%ige Ameisensäure, Eluent. B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

Methode 9: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 9 min 0% B → 9.2 min 2% B → 10 min 2% B; Fluss: 0.75 ml/min; Säulentemperatur: 30°C; UV-Detektion: 210 nm.

Methode 10: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

Methode 11: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

Methode 12: Instrument: HP 1100 mit DAD-Detektion; Säule: Kromasil C18 60*2; Eluent A: 0.01 M Phosphorsäure, Eluent B: Acetonitril, Gradient: 0 min 90% A → 0.5 min 90% A, → 4.5 min 10% A, → 6.5 min 10% A; Fluss: 0.75 ml/min; Säulentemperatur: 30°C; UV-Detektion: 210 nm.

### Ausgangsverbindungen

### Beispiel 1A

### 4-Amino-2-benzofuran-1,3-dion

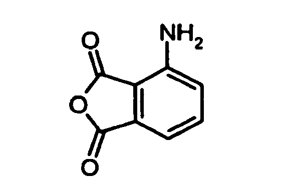

Die Darstellung der Titelverbindung erfolgt analog einem literaturbekannten Verfahren [E.L. Eliel et al., J. Am. Chem. Soc. 1955, 77, 5092-5094].

### Beispiel 2A

### 4-Amino-1H-isoindol-1,3(2H)-dion

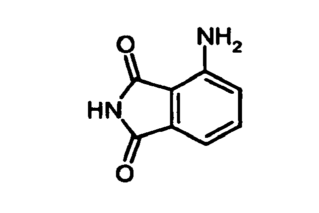

Die Darstellung der Titelverbindung erfolgt analog einem literaturbekannten Verfahren [H.D.K. Drew, F.H. Pearman, J. Chem. Soc. 1937, 26-33].

### Beispiel 3A

### 5-Chlor-N-(1,3-dioxo-1,3-dihydro-2-benzofuran-4-yl)thiophen-2-carboxamid

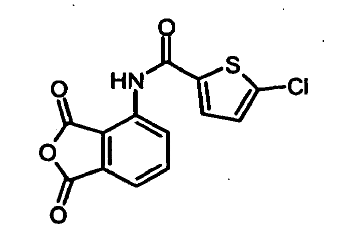

Die Darstellung der Titelverbindung erfolgt analog dem in WO 03/007942 beschriebenen Verfahren (Beispiel 1).

### Beispiel 4A

### 5-Chlor-N-(1,3-dioxo-2,3-dihydro-1H-isoindol-4-yl)thiophen-2-carboxamid

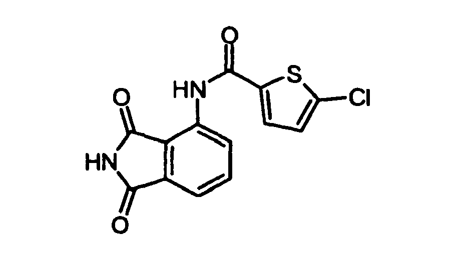

Die Darstellung der Titelverbindung erfolgt analog dem in WO 03/011858 beschriebenen Verfahren (Beispiel 1).

### Beispiel 5A

### 5-Chlor-N-[2-(4-iodbenzyl)-1,3-dioxo-2,3-dihydro-1H-isoindol-4-yl]thiophen-2-carboxamid

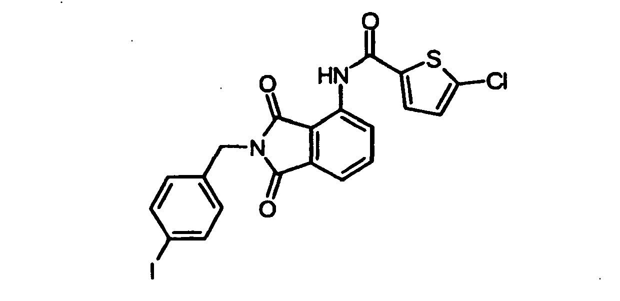

Eine Suspension aus 2.1 g (6.9 mmol) 5-Chlor-*N*-(1,3-dioxo-2,3-dihydro-1*H*-isoindol-4-yl)thiophen-2-carboxamid (Beispiel 4A) in 20 ml Tetrahydrofuran wird unter Argon bei RT mit einer Lösung aus 2.1 g (9.0 mmol, 1.3 eq.) (4-Iodphenyl)methanol in 10 ml Tetrahydrofuran und mit einer Lösung aus 2.3 g (9.0 mmol, 1.3 eq.) Triphenylphosphin in 10 ml Tetrahydrofuran versetzt. Die Reaktionssuspension wird auf 0°C gekühlt, mit einer Lösung aus 1.4 ml (9.0 mmol, 1.3 eq.) Azodicarbonsäurediethylester in 10 ml Tetrahydrofuran versetzt (wobei die Suspension in eine Lösung übergeht) und 1 h bei RT gerührt. Das Reaktionsgemisch wird im Vakuum eingeengt und der Rückstand mit Dichlormethan/Wasser verrührt.
Ausbeute: 2.5 g (70% Reinheit, 49% d. Th.)
LC-MS (Methode 1): Rₜ = 3.22 min;
MS (ESIpos): m/z = 521 [M+H]⁺.

### Beispiel 6A

### 5-Chlor-N-[2-(4-iodbenzyl)-3-oxo-2,3-dihydro-1H-isoindol-4-yl]thiophen-2-carboxamid

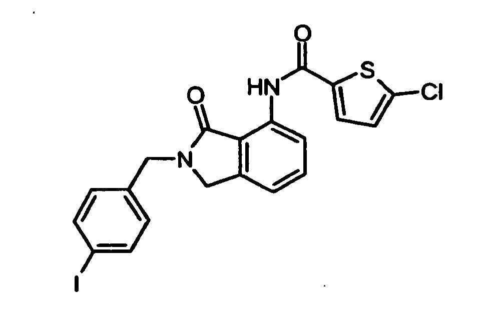

### Stufe a: 5-Chlor-N-[1-hydroxy-2-(4-iodbenzyl)-3-oxo-2,3-dihydro-1H-isoindol-4-yl]thiophen-2-carboxamid

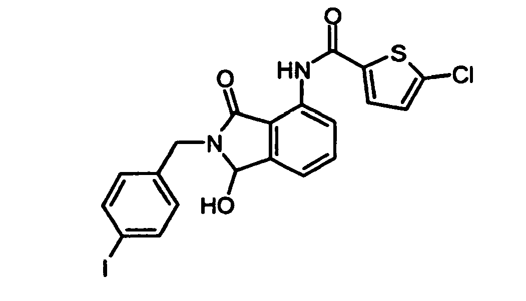

Eine Lösung aus 2.3 g (70% Reinheit, 3.0 mmol) 5-Chlor-*N*-[2-(4-iodbenzyl)-1,3-dioxo-2,3-dihydro-1*H*-isoindol-4-yl]thiophen-2-carboxamid (Beispiel 5A) in einem Gemisch aus 6 ml Methanol und 60 ml Dichlormethan wird unter Argon bei 0°C mit 247 mg (6.5 mmol, 2.2 eq.) Natriumborhydrid versetzt. Das Reaktionsgemisch wird 1.5 h bei RT gerührt und mit Salzsäure (1 N) auf pH 5 eingestellt. Der entstehende Niederschlag wird filtriert, mit Wasser und Dichlormethan gewaschen und im Vakuum getrocknet.
Ausbeute: 1.5 g (88% Reinheit, 57% d. Th.)
LC-MS (Methode 1): Rₜ = 2.93 min;
MS (ESIpos): m/z = 525 [M+H]⁺.

### Stufe b: 5-Chlor-N-[2-(4-iodbenzyl)-3-oxo-2,3-dihydro-1H-isoindol-4-yl]thiophen-2-carboxamid

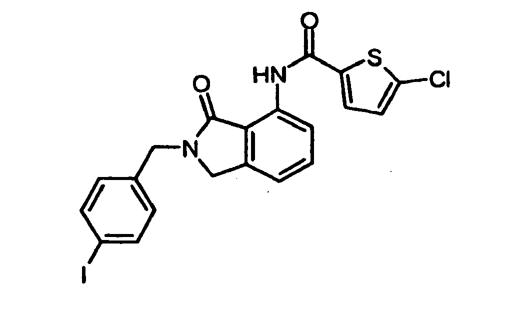

Eine Suspension aus 1.5 g (88% Reinheit, 2.6 mmol) 5-Chlor-*N*-[1-hydroxy-2-(4-iodbenzyl)-3-oxo-2,3-dihydro-1*H*-isoindol-4-yl]thiophen-2-carboxamid in 20 ml Dichlormethan wird unter Argon bei RT tropfenweise mit 2.7 ml (35.0 mmol, 13 eq.) Trifluoressigsäure und 0.93 ml (5.8 mmol, 2.2 eq.) Triethylsilan versetzt. Das Reaktionsgemisch wird 1 h bei RT gerührt und mit gesättigter, wässriger Natriumhydrogencarbonat-Lösung versetzt. Nach Zugabe von Dichlormethan und Phasentrennung wird die wässrige Phase mehrmals mit Dichlormethan extrahiert. Die vereinigten, organischen Phasen werden über Natriumsulfat getrocknet, filtriert und im Vakuum eingeengt.
Ausbeute: 1.4 g (95% d. Th.)
LC-MS (Methode 3): Rₜ = 3.32 min;
MS (ESIpos): m/z = 509 [M+H]⁺;
¹H-NMR (400 MHz, DMSO-d₆): δ = 11.27 (s, 1H), 8.25 (d, 1H), 7.72 (d, 2H), 7.64 (d, 1H), 7.59 (t, 1H), 7.33 (d, 1H), 7.28 (d, 1H), 7.13 (d, 2H), 4.71 (s, 2H), 4.43 (s, 2H).

### Beispiel 7A

### 5-Chlor-N-[2-(3-iodbenzyl)-1,3-dioxo-2,3-dihydro-1H-isoindol-4-yl]thiophen-2-carboxamid

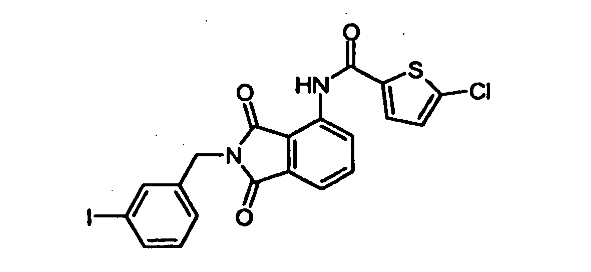

Eine Lösung aus 3.6 g (11.8 mmol) 5-Chlor-*N*-(1,3-dioxo-1,3-dihydro-2-benzofuran-4-yl)thiophen-2-carboxamid (Beispiel 3A) und 2.7 g (11.8 mmol, 1 eq.) 1-(3-Iodphenyl)methanamin in 50 ml Dioxan wird bei RT mit 10.3 ml (59.0 mmol, 5 eq.) *N,N*-Diisopropylethylamin versetzt. Das Reaktionsgemisch wird für 9 h unter Rückfluss gerührt und im Eisbad abgekühlt. Der entstehende Niederschlag wird filtriert, mit Dioxan gewaschen und im Vakuum getrocknet. Die vereinigten Mutterlaugen werden im Vakuum eingeengt. Der Rückstand wird mit Aceton verrührt und der Niederschlag filtriert, mit Aceton gewaschen und im Vakuum getrocknet.
Ausbeute: 3.9 g (62% d. Th.)
LC-MS (Methode 3): Rₜ = 3.36 min;
MS (ESIpos): m/z = 523 [M+H]⁺;
¹H-NMR (500 MHz, DMSO-d₆): δ = 10.40 (s, 1H), 8.33 (d, 1H), 7.87 (t, 1H), 7.80 (d, 1H), 7.72 (s, 1H), 7.67 (2xd, 2H), 7.37 (d, 1H), 7.34 (d, 1H), 7.15 (t, 1H), 4.73 (s, 2H).

### Beispiel 8A

### 5-Chlor-N-[2-(3-iodbenzyl)-3-oxo-2,3-dihydro-1H-isoindol-4-yl]thiophen-2-carboxamid

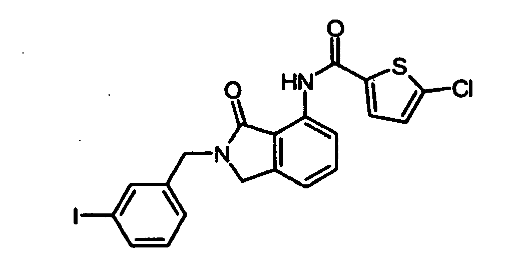

### stufe a): 5-Chlor-N-[1-hydroxy-2-(3-iodbenzyl)-3-oxo-2,3-dihydro-1H-isoindol-4-yl]thiophen-2-carboxamid

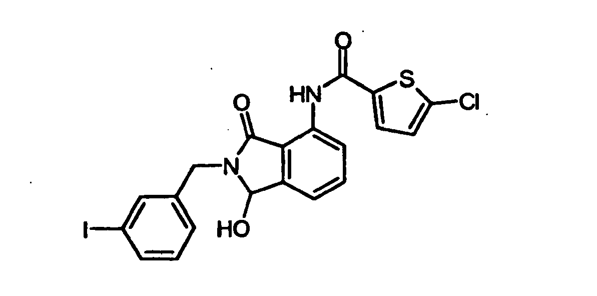

Eine Lösung aus 3.7 g (7.0 mmol) 5-Chlor-*N*-[2-(3-iodbenzyl)-1,3-dioxo-2,3-dihydro-1*H*-isoindol-4-yl]thiophen-2-carboxamid (Beispiel 7A) in einem Gemisch aus 8 ml Methanol und 80 ml Dichlormethan wird unter Argon bei 0°C mit 398 mg (10.5 mmol, 1.5 eq.) Natriumborhydrid versetzt. Das Reaktionsgemisch wird 1 h bei RT gerührt und mit Salzsäure (1 N) auf pH 5 eingestellt. Der entstehende Niederschlag wird filtriert, mit Wasser und Dichlormethan gewaschen und im Vakuum getrocknet.
Ausbeute: 2.3 g (6 1 % d. Th.)
LC-MS (Methode 3): Rₜ = 3.10 min;
MS (ESIpos): m/z = 525 [M+H]⁺;
¹H-NMR (500 MHz, DMSO-d₆): δ = 11.00 (s, 1H), 8.30 (d, 1H), 7.74 (s, 1H), 7.69-7.61 (m, 3H), 7.37 (d, 1H), 7.35-7.30 (m, 2H), 7.15 (t, 1H), 7.00 (d, 1H), 5.80 (d, 1H), 4.80 (d, 1H), 4.43 (d, 1H).

### Stufe b): 5-Chlor-N-[2-(3-iodbenzyl)-3-oxo-2,3-dihydro-1H-isoindol-4-yl]thiophen-2-carboxamid

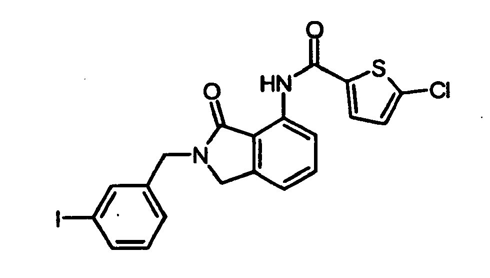

Eine Suspension aus 2.3 g (4.3 mmol) 5-Chlor-*N*-[1-hydroxy-2-(3-iodbenzyl)-3-oxo-2,3-dihydro-1*H*-isoindol-4-yl]thiophen-2-carboxamid in 30 ml Dichlormethan wird unter Argon bei RT tropfenweise mit 4.0 ml (51.5 mmol, 12 eq.) Trifluoressigsäure und 1.4 ml (8.6 mmol, 2 eq.) Triethylsilan versetzt. Das Reaktionsgemisch wird 20 h bei RT gerührt und mit gesättigter, wässriger Natriumhydrogencarbonat-Lösung versetzt. Nach Zugabe von Dichlormethan und Phasentrennung wird die wässrige Phase mehrmals mit Dichlormethan extrahiert. Die vereinigten, organischen Phasen werden mit gesättigter, wässriger Natriumchlorid-Lösung gewaschen, über Natriumsulfat getrocknet, filtriert und im Vakuum eingeengt.
Ausbeute: 1.5 g (97% d. Th.)
LC-MS (Methode 3): Rₜ = 3.33 min;
MS (ESIpos): m/z = 509 [M+H]⁺;
¹H-NMR (500 MHz, DMSO-d₆): δ = 11.23 (s, 1H), 8.27 (d, 1H), 7.71 (s, 1H), 7.68 (d, 1H), 7.65 (d, 1H), 7.61 (t, 1H), 7.33 (d, 2H), 7.29 (d, 1H), 7.19 (t, 1H), 4.73 (s, 2H), 4.46 (s, 2H).

### Beispiel 9A

### 4-Amino-2-(3-iodbenzyl)isoindolin-1-on

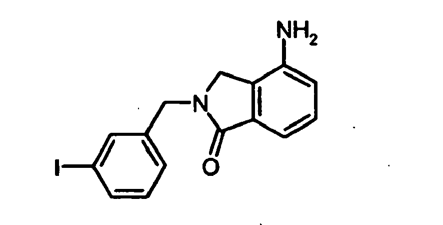

### Stufe a): 2-(Brommethyl)-3-nitrobenzoesäuremethylester

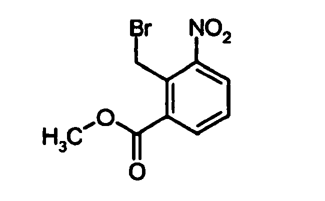

Eine Lösung aus 21 g (109 mmol) 2-Methyl-3-nitrobenzoesäuremethylester in 300 ml Tetrachlorkohlenstoff wird unter Rückfluss gerührt, mit 23 g (130 mmol, 1.2 eq.) N-Bromsuccinimid und 1.8 g (11 mmol, 0.1 eq.) 2,2'-Azobis-2-methylpropannitril versetzt und über Nacht unter Rückfluss gerührt. Nach Abkühlen auf RT wird die Reaktionsmischung mit Dichlormethan verdünnt, mehrmals mit Wasser gewaschen, über Natriumsulfat getrocknet, filtriert und im Vakuum eingeengt.
Ausbeute: 31 g (quantitativ)
HPLC (Methode 12): Rₜ = 4.33 min;
MS (ESIpos): m/z = 273 [M+H]⁺;
¹H-NMR (300 MHz, DMSO-d₆): δ = 8.16 (d, 1H), 8.11 (d, 1H), 7.74 (t, 1H), 5.03 (s, 2H), 3.92 (s, 3H).

### Stufe b): 2-(3-Iodbenzyl)-4-nitroisoindolin-1-on

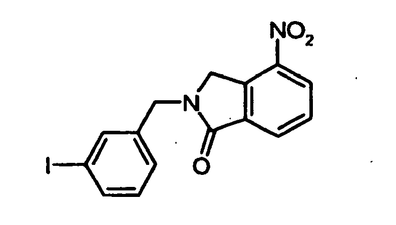

Eine Lösung aus 2.3 g (8.2 mmol) 2-(Brommethyl)-3-nitrobenzoesäuremethylester und 1.9 g (8.2 mmol, 1 eq.) 1-(3-Iodphenyl)methanamin in 40 ml Methanol wird bei RT mit 1.3 ml (9.1 mmol, 1.1 eq.) Triethylamin versetzt. Das Reaktionsgemisch wird für 3 h unter Rückfluss gerührt. Nach Abkühlen auf RT wird das Reaktionsgemisch mit gesättigter, wässriger Ammoniumchlorid-Lösung versetzt. Nach Zugabe von Dichlormethan und Phasentrennung wird die wässrige Phase mehrmals mit Dichlormethan extrahiert. Die vereinigten, organischen Phasen werden über Magnesiumsulfat getrocknet, filtriert und im Vakuum eingeengt. Die Titelverbindung wird mittels Flash-Chromatographie (Kieselgel, Dichlormethan/Cyclohexan 2:1 → Dichlormethan) isoliert.
Ausbeute: 2.8 g (87% d. Th.)
LC-MS (Methode 1): Rₜ = 2.29 min;
MS (ESIpos): m/z = 395 [M+H]⁺;
¹H-NMR (400 MHz, DMSO-d₆): δ = 8.43 (d, 1H), 8.18 (d, 1H), 7.82 (t, 1H), 7.73 (s, 1H), 7.68 (d, 1H), 7.35 (d, 1H), 7.18 (t, 1H), 4.84 (s, 2H), 4.77 (s, 2H).

### Stufe c): 4-Amino-2-(3-iodbenzyl)isoindolin-1-on

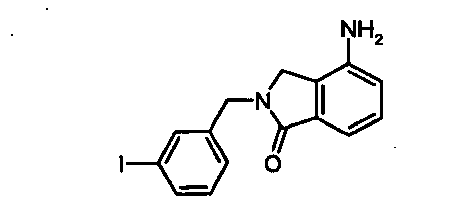

Eine Suspension aus 2.6 g (6.5 mmol) 2-(3-Iodbenzyl)-4-nitroisoindolin-1-on in 65 ml Ethanol wird bei RT mit 7.4 g (32.7 mmol, 5 eq.) Zinn(II)chlorid-dihydrat versetzt und 2.5 h bei 75°C Ölbadtemperatur gerührt (Bildung einer Lösung). Nach Abkühlen auf RT wird das Reaktionsgemisch auf Eiswasser gegossen, mit gesättigter, wässriger Natriumhydrogencarbonat-Lösung auf pH 8 eingestellt, über Celite filtriert und der Filterkuchen mehrmals mit Ethylacetat gewaschen. Nach Phasentrennung wird die wässrige Phase mit Ethylacetat extrahiert. Die vereinigten, organischen Phasen werden über Natriumsulfat getrocknet, filtriert und im Vakuum eingeengt. Die Titelverbindung wird ohne weitere Reinigung in der nächsten Reaktion eingesetzt.
Ausbeute: 2.0 g (93% d. Th.)
LC-MS (Methode 8): Rₜ = 2.05 min;
MS (ESIpos): m/z = 365 [M+H]⁺;
¹H-NMR (500 MHz, DMSO-d₆): δ = 7.70-7.62 (m, 2H), 7.26 (d, 1H), 7.22-7.15 (m, 2H), 6.91 (d, 1H), 6.76 (d, 1H), 5.40 (s, 2H), 4.69 (s, 2H), 4.11 (s, 2H).

### Beispiel 10A

### 5-Chlor-N-[2-(3-iodbenzyl)-1-oxo-2,3-dihydro-1H-isoindol-4-yl]thiophen-2-carboxamid

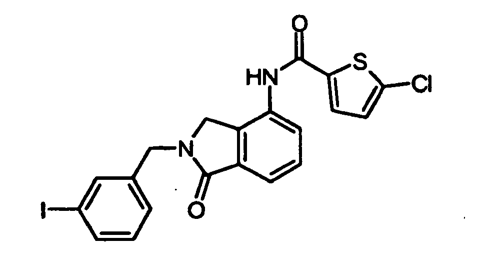

Eine Lösung aus 130 mg (0.80 mmol) 5-Chlorthiophencarbonsäure in 4 ml Dimethylformamid wird bei RT mit 335 mg (0.88 mmol, 1.1 eq.) O-(7-Azabenzotriazol-1-yl)-*N,N,N',N'*-tetramethyluronium-Hexafluoro-phosphat (HATU) und 0.28 ml (1.6 mmol, 2 eq.) *N,N*-Diisopropylethylamin versetzt, 30 min gerührt, mit 291 mg (0.80 mmol) 4-Amino-2-(3-iodbenzyl)isoindolin-1-on (Beispiel 9A) versetzt und über Nacht bei RT gerührt. Die Titelverbindung wird mittels präparativer RP-HPLC (CromSil C 18, Acetonitril/Wasser-Gradient) isoliert.
Ausbeute: 270 mg (66% d. Th.)
LC-MS (Methode 8): Rₜ = 2.64 min;
MS (ESIpos): m/z = 509 [M+H]⁺;
¹H-NMR (500 MHz, DMSO-d₆): δ = 10.39 (s, 1H), 7.85 (d, 1H), 7.70-7.64 (m, 3H), 7.61 (d, 1H), 7.55 (t, 1H), 7.32-7.25 (m, 2H), 7.16 (t, 1H), 4.70 (s, 2H), 4.41 (s, 2H).

### Beispiel 11A

### N-(2-{[tert.-Butyl(dimethyl)silyl]oxy}ethyl)phenylen-1,4-diamin

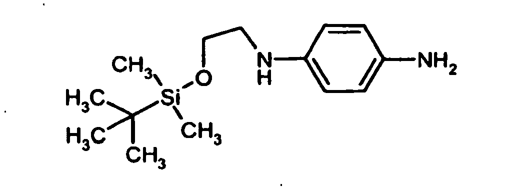

### Stufe a): 2-[(4-Nitrophenyl)amino]ethanol

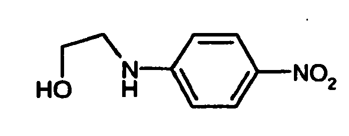

Eine Lösung aus 101 g (716 mmol) 4-Fluornitrophenol in 500 ml Ethanol wird bei RT mit 130 ml (2.15 mol, 3 eq.) 2-Aminoethanol und 274 ml (1.57 mol, 2.2 eq.) *N,N*-Diisopropylethylamin versetzt. Das Reaktionsgemisch wird bei 50°C über Nacht gerührt, anschließend mit weiteren 86 ml (1.43 mol, 2.0 eq.) 2-Aminoethanol und 249 ml (1.43 mol, 2.0 eq.) *N,N*-Diisopropylethylamin versetzt und erneut 12 h bei 50°C gerührt. Die Reaktionslösung wird im Vakuum eingeengt und der Rückstand mit 600 ml Wasser verrührt. Der entstandene Niederschlag wird filtriert, mehrmals mit Wasser gewaschen und getrocknet.
Ausbeute: 127 g (97% d. Th.)
LC-MS (Methode 5): Rₜ = 2.32 min;
MS (ESIpos): m/z = 183 [M+H]⁺;
¹H-NMR (300 MHz, DMSO-d₆): δ = 7.99 (d, 2H), 7.30 (t, 1H), 6.68 (d, 2H), 4.82 (t, 1H), 3.63-3.52 (m, 2H), 3.30-3.19 (m, 2H).

### Stufe b): N-(2-{[tert.-Butyl(dimethyl)silyl]oxy}ethyl)-4-nitroanilin

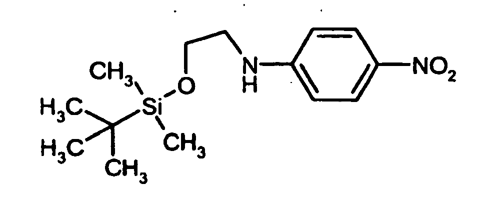

Eine Lösung aus 30.8 g (169 mmol) 2-[(4-Nitrophenyl)amino]ethanol in 300 ml DMF wird bei RT mit 30.6 g (203 mmol, 1.2 eq.) *tert*.-Butyldimethylchlorsilan und 17.3 g (254 mmol, 1.5 eq.) Imidazol versetzt und 2.5 h bei RT gerührt. Das Reaktionsgemisch wird im Vakuum eingeengt und der Rückstand in 200 ml Dichlormethan und 100 ml Wasser gelöst. Nach Phasentrennung wird die wässrige Phase dreimal mit Dichlonnethan extrahiert. Die vereinigten, organischen Phasen werden mit gesättigter, wässriger Natriumchlorid-Lösung gewaschen, über Natriumsulfat getrocknet, filtriert und im Vakuum eingeengt.
Ausbeute: 49.7 g (quantitativ)
LC-MS (Methode 3): Rₜ = 3.09 min;
MS (ESIpos): m/z = 297 [M+H]⁺;
¹H-NMR (300 MHz, DMSO-d₆): δ = 7.98 (d, 2H), 7.29 (t, 1H), 6.68 (d, 2H), 3.77-3.66 (m, 2H), 3.35-3.24 (m, 2H), 0.81 (s, 9H), 0.0 (s, 6H).

### Stufe c): N-(2-{[tert.-Butyl(dimethyl)silyl]oxy}ethyl)phenylen-1,4-diamin

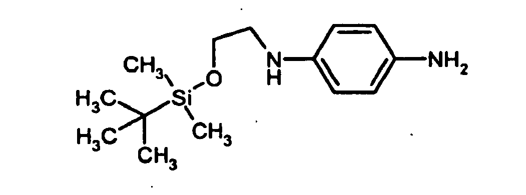

Eine Lösung aus 59.5 g (201 mmol) *N*-(2-{[*tert*.-Butyl(dimethyl)silyl]oxy}ethyl)-4-nitroanilin in 500 ml Ethanol wird unter Argon mit 4 g Palladium auf Aktivkohle (10%-ig) versetzt und in einer Wasserstoffatmosphäre bei RT und Normaldruck hydriert. Der Katalysator wird über eine Filterschicht abgetrennt, mit Ethanol gewaschen und das Filtrat im Vakuum eingeengt.
Ausbeute: 53 g (quantitativ)
LC-MS (Methode 2): Rₜ = 1.83 min;
MS (ESIpos): m/z = 267 [M+H]⁺;
¹H-NMR (300 MHz, DMSO-d₆): δ = 6.42-6.30 (m, 4H), 4.48 (t, 1H), 4.21 (br. s, 2H), 3.68-3.58 (m, 2H), 3.04-2.93 (m, 2H), 0.82 (s, 9H), 0.0 (s, 6H).

### Beispiele 12A

### 2-[(4-Aminophenyl)amino]ethanol

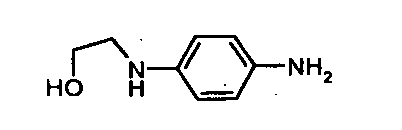

Eine Lösung aus 2.0 g (11 mmol) 2-[(4-Nitrophenyl)amino]ethanol (Beispiel 11A, Stufe a)) in 30 ml Ethanol wird unter Argon mit 200 mg Palladium auf Aktivkohle (10%-ig) versetzt und in einer Wasserstoffatmosphäre bei RT und Normaldruck hydriert. Der Katalysator wird über eine Filterschicht abgetrennt, mit Ethanol gewaschen und das Filtrat im Vakuum eingeengt.
Ausbeute: 1.7 g (97% d. Th.)
LC-MS (Methode 5): Rₜ = 0.47 min;
MS (ESIpos): m/z= 153 [M+H]⁺;
¹H-NMR (300 MHz, DMSO-d₆): δ = 6.45-6.31 (m, 4H), 4.59 (t, 1H), 4.50 (br. s, 1H), 4.21 (br. s, 2H), 3.52 (q, 2H), 2.97 (t, 2H).

### Ausführungsbeispiele

### Beispiel 1

### 5-Chlor-N-{2-[4-(2-imino-1,3-oxazolidin-3-yl)phenyl]-1,3-dioxo-2,3-dihydro-1H-isoindol-4-yl}thiophen-2-carboxamid-Methansulfonat

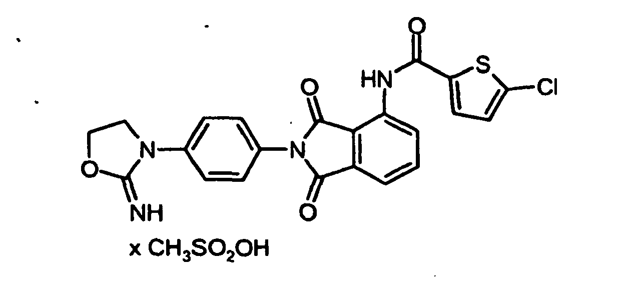

### Stufe a): N-(2-{4-[(2-{[tert.-Butyl(dimethyl)silyl]oxy}ethyl)amino]phenyl}-1,3-dioxo-2,3-dihydro-1H-isoindol-4-yl)-5-chlorthiophen-2-carboxamid

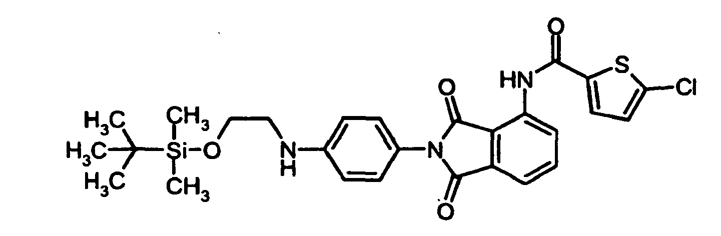

Eine Lösung aus 1.00 g (3.25 mmol) 5-Chlor-*N*-(1,3-dioxo-1,3-dihydro-2-benzofuran-4-yl)thiophen-2-carboxamid (Beispiel 3A) und 0.87 g (3.25 mmol, 1 eq.) *N*-(2-{[*tert*.-Butyl-(dimethyl)silyl]oxy}ethyl)phenylen-1,4-diamin (Beispiel 11A) in 14 ml Dioxan wird bei RT mit 2.8 ml (16.2 mmol, 5 eq.) *N,N*-Diisopropylethylamin versetzt. Das Reaktionsgemisch wird 4 h unter Rückfluss gerührt und anschließend im Eisbad gekühlt. Der entstehende Niederschlag wird filtriert, mit Dioxan gewaschen und im Vakuum getrocknet.
Ausbeute: 0.81 g (45% d. Th.)
LC-MS (Methode 1): Rₜ = 3.59 min;
MS (ESIpos): m/z = 556 [M+H]⁺;
¹H-NMR (400 MHz, DMSO-d₆): δ = 10.47 (s, 1H), 8.41 (d, 1H), 7.89 (t, 1H), 7.77 (d, 1H), 7.69 (d, 1H), 7.32 (d, 1H), 7.09 (d, 2H), 6.68 (d, 2H), 5.85 (t, 1H), 3.73 (t, 2H), 3.19 (q, 2H), 0.89 (s, 9H), 0.05 (s, 6H).

### Stufe b): N-(2-{4-[(2-{[tert.-Butyl(dimethyl)silyl]oxy}ethyl)(cyano)amino]phenyl}-1,3-dioxo-2,3-dihydro-1H-isoindol-4-yl)-5-chlorthiophen-2-carboxamid

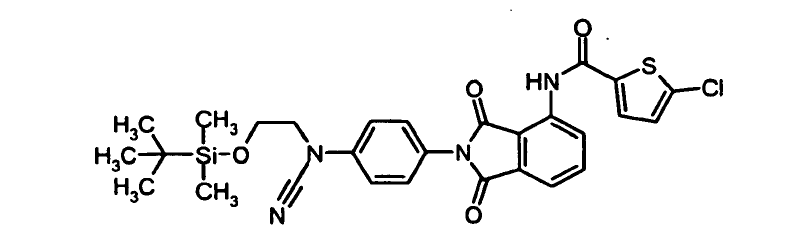

Eine Lösung aus 164 mg (0.30 mmol) *N*-(2-{4-[(2-{[*tert*.-Butyl(dimethyl)silyl]oxy}ethyl)-amino]phenyl}-1,3-dioxo-2,3-dihydro-1*H*-isoindol-4-yl)-5-chlorthiophen-2-carboxamid in 6 ml Tetrahydrofuran wird unter Argon bei RT mit 74 mg (0.89 mmol, 3 eq.) Natriumhydrogencarbonat und insgesamt 0.20 ml Bromcyan-Lösung (3 M in Dichlormethan, 0.60 mmol, 2 eq.) versetzt und 6 d bei 40°C gerührt. Nach Zugabe von Wasser/Dichlormethan und Phasentrennung wird die wässrige Phase mit Dichlormethan extrahiert. Die vereinigten, organischen Phasen werden mit gesättigter, wässriger Natriumhydrogencarbonat-Lösung gewaschen, über Natriumsulfat getrocknet, filtriert und im Vakuum eingeengt. Die Titelverbindung wird durch Verrühren des Rohproduktes in Diethylether isoliert.
Ausbeute: 0.12 g (69% d. Th.)
LC-MS (Methode 1): Rₜ = 3.30 min;
MS (ESIpos): m/z = 581 [M+H]⁺;
¹H-NMR (400 MHz, DMSO-d₆): δ = 10.48 (s, 1H), 8.41 (d, 1H), 7.91 (t, 1H), 7.79 (d, 1H), 7.74 (d, 1H), 7.50 (d, 2H), 7.36 (d, 2H), 7.34 (d, 1H), 3.89 (s, 4H), 0.86 (s, 9H), 0.0 (s, 6H).

### Stufe c): 5-Chlor-N-{2-[4-(2-imino-1,3-oxazolidin-3-yl)phenyl]-1,3-dioxo-2,3-dihydro-1H-isoindol-4-yl}thiophen-2-carboxamid-Methansulfonat

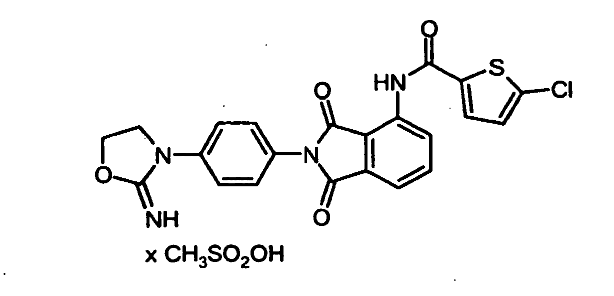

Eine Suspension aus 114 mg (0.20 mmol) *N*-(2-{4-[(2-{[*tert*.-Butyl(dimethyl)silyl]oxy}ethyl)-(cyano)amino]phenyl}-1,3-dioxo-2,3-dihydro-1*H*-isoindol-4-yl)-5-chlorthiophen-2-carboxamid in 18 ml Acetonitril wird bei RT mit 27 µl (0.41 mmol, 2.1 eq.) Methansulfonsäure versetzt (Bildung einer Lösung) und 3 d bei RT gerührt. Die Titelverbindung wird durch Filtration des entstandenen Niederschlages, Waschen mit Acetonitril und Trocknen im Vakuum isoliert.
Ausbeute: 46 mg (42% d. Th.)
HPLC (Methode 9): Rₜ = 4.30 min;
MS (ESIpos): m/z = 467 [M+H]⁺ (freie Base);
¹H-NMR (400 MHz, DMSO-d₆): δ = 10.50 (s, 1H), 9.72 (br. s, 1H), 9.10 (br. s, 1H), 8.41 (d, 1H), 7.96 (t, 1H), 7.78 (s, 2H), 7.69 (s, 4H), 7.34 (d, 1H), 4.87 (t, 2H), 4.30 (t, 2H), 2.30 (s, 3H).

### Beispiel 2

### 5-Chlor-N-{2-[4-(2-imino-1,3-oxazolidin-3-yl)phenyl]-3-oxo-2,3-dihydro-1H-isoindol-4-yl}thiophen-2-carboxamid-Methansulfonat

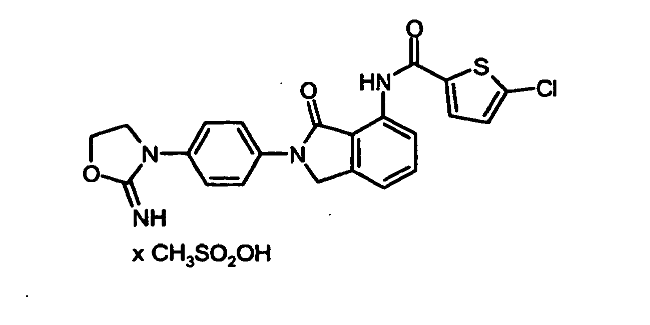

### Stufe a): 5-Chlor-N-(2-{4-[(2-hydroxyethyl)amino]phenyl}-1,3-dioxo-2,3-dihydro-1H-isoindol-4-yl)thiophen-2-carboxamid

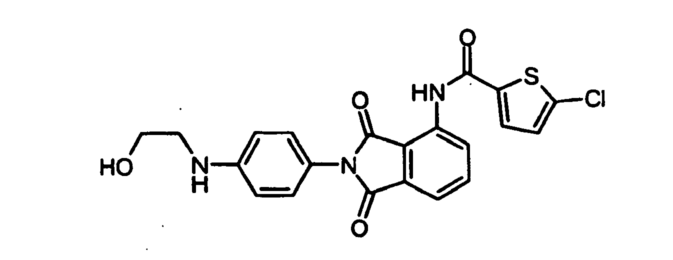

Eine Lösung aus 2.0 g (6.6 mmol) 5-Chlor-*N*-(1,3-dioxo-1,3-dihydro-2-benzofuran-4-yl)thiophen-2-carboxamid (Beispiel 3A) und 1.0 g (6.6 mmol, 1 eq.) 2-[(4-Aminophenyl)amino]ethanol (Beispiel 12A) in 35 ml Dioxan wird bei RT mit 5.7 ml (32.9 mmol, 5 eq.) *N,N*-Diisopropylethylamin versetzt. Das Reaktionsgemisch wird 5 h unter Rückfluss gerührt und anschließend im Eisbad gekühlt. Der entstehende Niederschlag wird filtriert, mit Dioxan gewaschen und im Vakuum getrocknet.
Ausbeute: 1.9 g (65% d. Th.)
LC-MS (Methode 2): Rₜ = 2.61 min;
MS (ESIpos): m/z = 442 [M+H]⁺;
¹H-NMR (400 MHz, DMSO-d₆): δ = 10.47 (s, 1H), 8.41 (d, 1H), 7.89 (t, 1H), 7.75 (d, 1H), 7.69 (d, 1H), 7.32 (d, 1H), 7.09 (d, 2H), 6.68 (d, 2H), 5.85 (t, 1H), 4.71 (br. s, 1H), 3.58 (q, 2H), 3.13 (q, 2H).

### Stufe b): Isomerengemisch aus 5-Chlor-N-(2-{4-[(2-hydroxyethyl)amino]phenyl}-3-oxo-2,3-dihydro-1H-isoindol-4-yl)thiophen-2-carboxamid

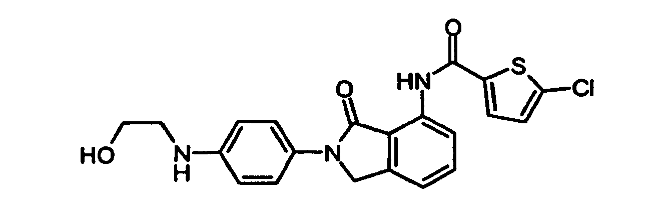

und 5-Chlor-*N*-2-(4-[(2-hydroxyethyl)amino]phenyl)-1-oxo-2,3-dihydro-1*H*-isoindol-4-yl)thiophen-2-carboxamid

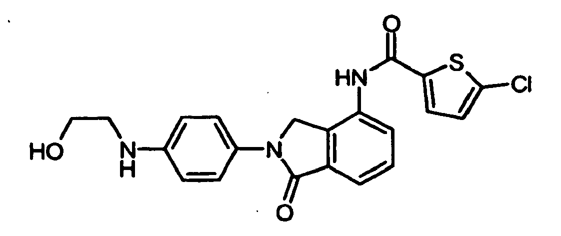

Eine Lösung aus 1.9 g (4.4 mmol) 5-Chlor-*N*-(2-{4-[(2-hydroxyethyl)amino]phenyl}-1,3-dioxo-2,3-dihydro-1*H*-isoindol-4-yl)thiophen-2-carboxamid in einem Gemisch aus 60 ml Methanol und 60 ml Dichlormethan wird unter Argon bei 0°C mit 331 mg (8.8 mmol, 2 eq.) Natriumborhydrid versetzt. Das Reaktionsgemisch wird 8 h bei RT gerührt und mit Salzsäure (1 N) auf pH 5 eingestellt. Nach Phasentrennung wird die wässrige Phase mit Dichlormethan extrahiert. Die vereinigten, organischen Phasen werden über Magnesiumsulfat getrocknet, filtriert und im Vakuum eingeengt. Das Isomerengemisch (1.75 g, 86% Reinheit, 78% d. Th.) wird ohne weitere Reinigung in der nächsten Reaktion eingesetzt.

Eine Lösung aus 1.74 g (3.9 mmol) Isomerengemisch in 24 ml Dichlormethan wird unter Argon bei RT tropfenweise mit 2.7 ml (35.3 mmol, 9 eq.) Trifluoressigsäure und 0.94 ml (5.9 mmol, 1.5 eq.) Triethylsilan versetzt. Das Reaktionsgemisch wird 5 d bei RT gerührt und mit gesättigter, wässriger Natriumhydrogencarbonat-Lösung versetzt. Nach Zugabe von Dichlormethan und Phasentrennung wird die wässrige Phase mehrmals mit Dichlormethan extrahiert. Die vereinigten, organischen Phasen werden mit gesättigter, wässriger Natriumchlorid-Lösung gewaschen und im Vakuum eingeengt. Die Isomere werden mittels präparativer RP-HPLC (Kromasil 100 C18, Acetonitril/Wasser/1%-ige Trifluoressigsäure-Gradient) isoliert.

### Isomer 1:

### 5-Chlor-N-(2-{4-[(2-hydroxyethyl)amino]phenyl}-3-oxo-2,3-dihydro-1H-isoindol-4-yl)thiophen-2-carboxamid

Ausbeute: 442 mg (25% d. Th.)
LC-MS (Methode 1): Rₜ = 2.34 min;
MS (ESIpos): m/z = 428 [M+H]⁺;
¹H-NMR (400 MHz, DMSO-d₆): δ = 11.43 (s, 1H), 8.30 (d, 1H), 7.66-7.60 (m, 2H), 7.53 (d, 2H), 7.37-7.30 (m, 2H), 6.67 (d, 2H), 5.61 (t, 1H), 4.97 (s, 2H), 4.70 (t, 1H), 3.57 (q, 2H), 3.11 (q, 2H).

### Isomer 2:

### 5-Chlor-N-(2-{4-[(2-hydroxyethyl)amino]phenyl}-1-oxo-2,3-dihydro-1H-isoindol-4-yl)thiophen-2-carboxamid

Ausbeute: 225 mg (12% d. Th.)
LC-MS (Methode 1): Rₜ = 1.75 min;
MS (ESIpos): m/z = 428 [M+H]⁺;
¹H-NMR (400 MHz, DMSO-d₆): δ = 10.45 (s, 1H), 7.87 (d, 1H), 7.71 (d, 1H), 7.60-7.49 (m, 4H), 7.28 (d, 1H), 6.64 (d, 2H), 5.52 (t, 1H), 4.88 (s, 2H), 4.69 (t, 1H), 3.55 (q, 2H), 3.10 (q, 2H).

### Stufe c): N-(2-{4-[(2-{[tert.-Butyl(dimethyl)silyl]oxy}ethyl)amino]phenyl)-3-oxo-2,3-dihydro-1H-isoindol-4-yl)-5-chlorthiophen-2-carboxamid

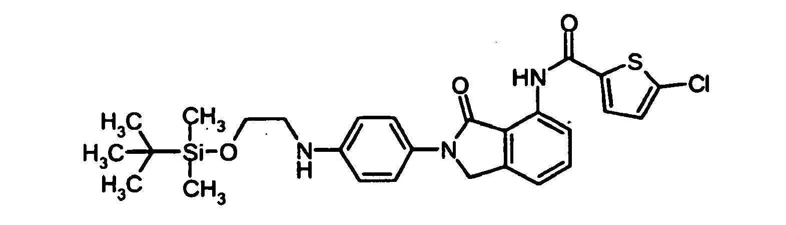

Eine Lösung aus 91 mg (0.21 mmol) 5-Chlor-*N*-(2-{4-[(2-hydroxyethyl)amino]phenyl}-3-oxo-2,3-dihydro-1*H*-isoindol-4-yl)thiophen-2-carboxamid (Isomer 1) in 12 ml Dimethylformamid wird bei 0°C mit 29 mg (0.43 mmol, 2 eq.) Imidazol und 38 mg (0.3 mmol, 1.2 eq.) *tert*.-Butyldimethylsilylchlorid versetzt. Das Reaktionsgemisch wird 19 h bei RT gerührt. Die Titelverbindung wird mittels präparativer RP-HPLC (CromSil C18, Acetonitril/Wasser-Gradient) isoliert.
Ausbeute: 32 mg (28% d. Th.)
LC-MS (Methode 1): Rₜ = 3.46 min;
MS (ESIpos): m/z = 542 [M+H]⁺;
¹H-NMR (400 MHz, DMSO-d₆): δ = 11.37 (s, 1H), 8.23 (d, 1H), 7.60-7.53 (m, 2H), 7.49 (d, 2H), 7.31-7.24 (m, 2H), 6.62 (d, 2H), 5.55 (t, 1H), 4.91 (s, 2H), 3.69 (t, 2H), 3.12 (q, 2H), 0.83 (s, 9H), 0.0 (s, 6H).

### Stufe d): N-(2-{4-[(2-{[tert.-Butyl(dimethyl)silyl]oxy}ethyl)(cyano)amino]phenyl}-3-oxo-2,3-dihydro-1H-isoindol-4-yl)-5-chlorthiophen-2-carboxamid

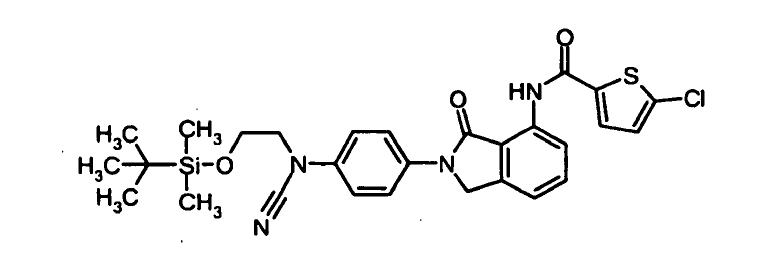

Eine Lösung aus 28 mg (0.05 mmol) *N*-(2-{4-[(2-{[*tert*.-Butyl(dimethyl)silyl]oxy}ethyl)-amino]phenyl}-3-oxo-2,3-dihydro-1*H*-isoindol-4-yl)-5-chlorthiophen-2-carboxamid in 2 ml Tetrahydrofuran wird unter Argon bei RT mit 13 mg (0.15 mmol, 3 eq.) Natriumhydrogencarbonat und insgesamt 51 µl Bromcyan-Lösung (3 M in Dichlormethan, 0.15 mmol, 3 eq.) versetzt und 6 d bei 40°C gerührt. Nach Zugabe von Wasser/Dichlormethan und Phasentrennung wird die wässrige Phase mit Dichlormethan extrahiert. Die vereinigten, organischen Phasen werden mit gesättigter, wässriger Natriumhydrogencarbonat-Lösung und mit gesättigter, wässriger Natriumchlorid-Lösung gewaschen, über Natriumsulfat getrocknet, filtriert und im Vakuum eingeengt. Die Titelverbindung wird mittels präparativer RP-HPLC (CromSil C18, Acetonitril/Wasser-Gradient) isoliert:
Ausbeute: 18 mg (62% d. Th.)
LC-MS (Methode 1): Rₜ = 3.38 min;
MS (ESIpos): m/z = 567 [M+H]⁺;
¹H-NMR (400 MHz, DMSO-d₆): δ = 11.27 (s, 1H), 8.30 (d, 1H), 7.92 (d, 2H), 7.71-7.63 (m, 2H), 7.41-7.33 (m, 2H), 7.30 (d, 2H), 5.08 (s, 2H), 3.86 (s, 4H), 0.83 (s, 9H), 0.0 (s, 6H).

### Stufe e): 5-Chlor-N-{2-[4-(2-imino-1,3-oxazolidin-3-yl)phenyl]-3-oxo-2,3-dihydro-1H-isoindol-4-yl}thiophen-2-carboxamid-Methansulfonat

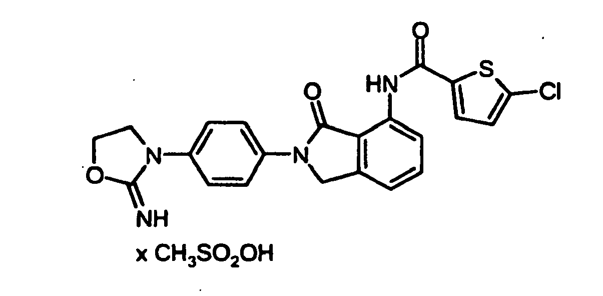

Eine Lösung aus 17 mg (0.03 mmol) *N*-(2-{4-[(2-{[*tert*.-Butyl(dimethyl)silyl]oxy}ethyl)-(cyano)amino]phenyl}-3-oxo-2,3-dihydro-1*H*-isoindol-4-yl)-5-chlorthiophen-2-carboxamid in 50 ml Acetonitril wird bei RT mit 4 µl (0.06 mmol, 2.1 eq.) Methansulfonsäure versetzt und 2 d bei RT gerührt. Die Titelverbindung wird mittels präparativer RP-HPLC (Kromasil 100 C18, Acetonitril/Wasser-Gradient) isoliert.
Ausbeute: 10 mg (59% d. Th.)
LC-MS (Methode 3): Rₜ = 1.79 min;
MS (ESIpos): m/z = 453 [M+H]⁺ (freie Base);
¹H-NMR (400 MHz, DMSO-d₆): δ = 11.21 (s, 1H), 9.40 (br. s, 1H), 8.95 (br. s, 1H), 8.32 (d, 1H), 8.09 (d, 2H), 7.75-7.61 (m, 4H), 7.42 (d, 1H), 7.39 (d, 1H), 5.12 (s, 2H), 4.82 (t, 2H), 4.25 (t, 2H), 2.29 (s, 3H).

### Beispiel 3

### 5-Chlor-N-{2-[4-(2-imino-1,3-oxazolidin-3-yl)phenyl]-1-oxo-2,3-dihydro-1H-isoindol-4-yl}thiophen-2-carboxamid-Methansulfonat

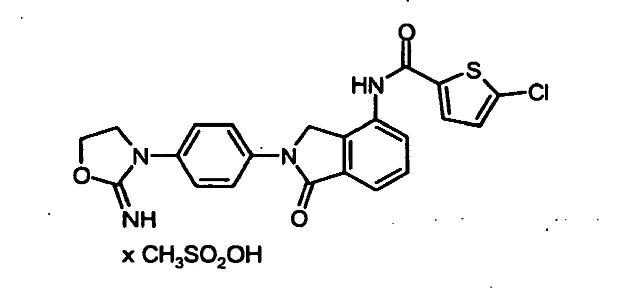

### Stufe a): N-(2-{4-[(2-{[tert.-Butyl(dimethyl)silyl]oxy}ethyl)amino]phenyl}-1-oxo-2,3-dihydro-1H-isoindol-4-yl)-5-chlorthiophen-2-carboxamid

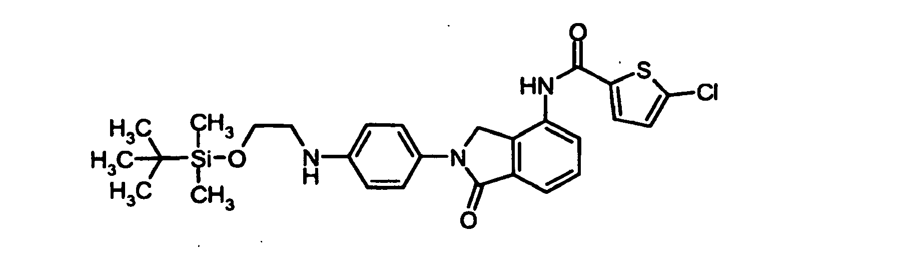

Eine Lösung aus 73 mg (0.17 mmol) 5-Chlor-*N*-(2-{4-[(2-hydroxyethyl)amino]phenyl}-1-oxo-2,3-dihydro-1*H*-isoindol-4-yl)thiophen-2-carboxamid (Beispiel 2, Stufe b), Isomer 2) in 3 ml Dimethylformamid wird bei 0°C mit 23 mg (0.34 mmol, 2 eq.) Imidazol und 31 mg (0.21 mmol, 1.2 eq.) *tert*.-Butyldimethylsilylchlorid versetzt. Das Reaktionsgemisch wird 1 d bei RT gerührt. Die Titelverbindung wird mittels präparativer RP-HPLC (CromSil C18, Acetonitril/Wasser-Gradient) isoliert.
Ausbeute: 28 mg (29% d. Th.)
LC-MS (Methode 2): Rₜ = 3.34 min;
MS (ESIpos): m/z = 542 [M+H]⁺;
¹H-NMR (400 MHz, DMSO-d₆): δ = 10.40 (s, 1H), 7.89 (d, 1H), 7.65 (d, 1H), 7.60-7.51 (m, 2H), 7.49 (d, 2H), 7.28 (d, 1H), 6.60 (d, 2H), 5.50 (t, 1H), 4.84 (s, 2H), 3.67 (t, 2H), 3.12 (q, 2H), 0.82 (s, 9H), 0.0 (s, 6H).

### Stufe b): N-(2-{4-[(2-{[tert.-Butyl(dimethyl)silyl]oxy}ethyl)(cyano)amino]phenyl}-1-oxo-2,3-dihydro-1H-isoindol-4-yl)-5-chlorthiophen-2-carboxamid

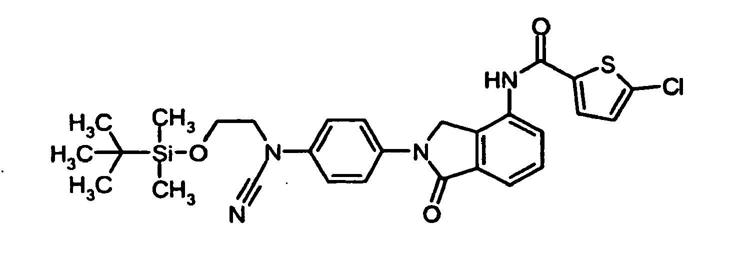

Eine Lösung aus 23 mg (0.04 mmol) *N*-(2-{4-[(2-{[*tert*.-Butyl(dimethyl)silyl]oxy}ethyl)amino]phenyl}-1-oxo-2,3-dihydro-1*H*-isoindol-4-yl)-5-hlorthiophen-2-carboxamid in 2 ml Tetrahydrofuran wird unter Argon bei RT mit 11 mg (0.13 mmol, 3 eq.) Natriumhydrogencarbonat und insgesamt 24 µl Bromcyan-Lösung (3 M in Dichlormethan, 0.07 mmol, 1.7 eq.) versetzt und 3 d bei 40°C gerührt. Nach Zugabe von Wasser/Dichlormethan und Phasentrennung wird die wässrige Phase mit Dichlormethan extrahiert. Die vereinigten, organischen Phasen werden mit gesättigter, wässriger Natriumhydrogencarbonat-Lösung und mit gesättigter, wässriger Natriumchlorid-Lösung gewaschen, über Natriumsulfat getrocknet, filtriert und im Vakuum eingeengt. Die Titelverbindung wird ohne weitere Reinigung in der nächsten Reaktion eingesetzt.
Ausbeute: 22 mg (86% d. Th.)
LC-MS (Methode 1): Rₜ = 3.01 min;
MS (ESIpos): m/z = 567 [M+H]⁺;
¹H-NMR (400 MHz, DMSO-d₆): δ = 10.49 (s, 1H), 7.95 (d, 1H), 7.93 (d, 2H), 7.75 (d, 1H), 7.68 (d, 1H), 7.60 (t, 1H), 7.33 (d, 1H), 7.28 (d, 2H), 5.00 (s, 2H), 3.83 (s, 4H), 0.83 (s, 9H), 0.0 (s, 6H).

### Stufe c): 5-Chlor-N-{2-[4-(2-imino-1,3-oxazolidin-3-yl)phenyl]-1-oxo-2,3-dihydro-1H-isoindol-4-yl}thiophen-2-carboxamid-Methansulfonat

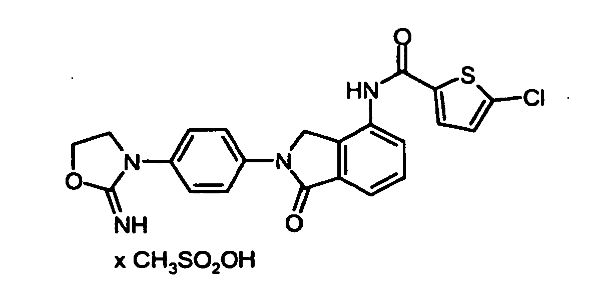

Eine Suspension aus 20 mg (0.04 mmol) *N*-(2-{4-[(2-{[*tert*.-Butyl(dimethyl)silyl]oxy}ethyl)-(cyano)amino]phenyl}-1-oxo-2,3-dihydro-1*H*-isoindol-4-yl)-5-chlorthiophen-2-carboxamid in 5 ml Acetonitril wird bei RT mit 5 µl (0.07 mmol, 2.1 eq.) Methansulfonsäure versetzt und 2 d bei RT gerührt. Die Titelverbindung wird mittels Verrühren in Diethylether isoliert.
Ausbeute: 7 mg (32% d. Th.)
LC-MS (Methode 2): Rₜ = 1.76 min;
MS (ESIpos): m/z = 453 [M+H]⁺ (freie Base);
¹H-NMR (400 MHz, DMSO-d₆): δ = 10.59 (s, 1H), 9.59 (br. s, 1H), 8.86 (br. s, 1H), 8.10 (d, 2H), 7.97 (d, 1H), 7.78-7.68 (m, 2H), 7.66-7.55 (m, 3H), 7.33 (d, 1H), 5.05 (s, 2H), 4.87 (t, 2H), 4.26 (t, 2H), 2.30 (s, 3H).

### Beispiel 4

### 5-Chlor-N-{2-[4-(2-imino-1,3-oxazolidin-3-yl)benzyl]-3-oxo-2,3-dihydro-1H-isoindol-4-yl}thiophen-2-carboxamid-Methansulfonat

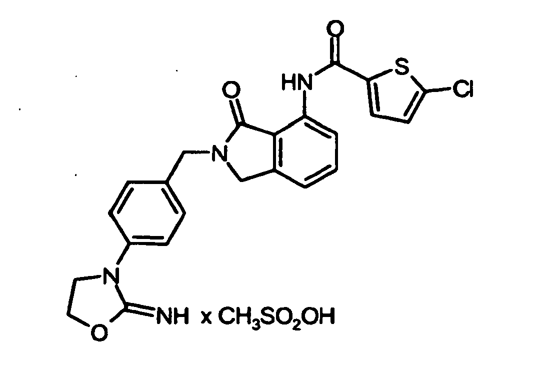

### Stufe a): 5-Chlor-N-(2-{4-[(2-hydroxyethyl)amino]benzyl}-3-oxo-2,3-dihydro-1H-isoindol-4-yl)thiophen-2-carboxamid

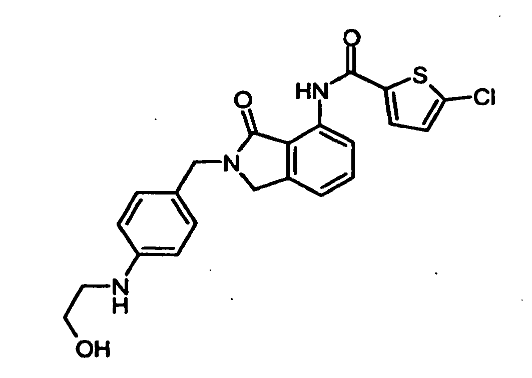

Eine Suspension aus 26 mg (0.14 mmol, 0.1 eq.) Kupfer(I)iodid und 1.17 g (5.5 mmol, 4 eq.) Kaliumphosphat in 20 ml Isopropanol wird unter Argon bei RT mit 0.31 ml (5.5 mmol, 4 eq.) 1,2-Ethandiol, 700 mg (1.38 mmol) 5-Chlor-*N*-[2-(4-iodbenzyl)-3-oxo-2,3-dihydro-1*H*-isoindol-4-yl]thiophen-2-carboxamid (Beispiel 6A) und 0.5 ml (8.3 mmol, 6 eq.) 2-Aminoethanol versetzt. Das Reaktionsgemisch wird über Nacht bei 80°C gerührt, nach Abkühlen auf RT filtriert und der Rückstand mit Isopropanol gewaschen. Die vereinigten Filtrate werden im Vakuum eingeengt. Die Titelverbindung wird mittels präparativer RP-HPLC (CromSil C18, Acetonitril/Wasser-Gradient) isoliert.
Ausbeute: 220 mg (36% d. Th.)
LC-MS (Methode 1): Rₜ = 2.33 min;
MS (ESIpos): m/z = 442 [M+H]⁺;
¹H-NMR (400 MHz, DMSO-d₆): δ = 11.37 (s, 1H), 8.24 (d, 1H), 7.66 (d, 1H), 7.58 (t, 1H), 7.34 (d, 1H), 7.27 (d, 1H), 7.04 (d, 2H), 6.56 (d, 2H), 5.54 (t, 1H), 4.66 (t, 1H), 4.58 (s, 2H), 4.37 (s, 2H), 3.52 (q, 2H), 3.06 (q, 2H).

### Stufe b): N-(2-{4-[(2-{[tert.-Butyl(dimethyl)silyl]oxy}ethyl)amino]benzyl}-3-oxo-2,3-dihydro-1H-isoindol-4-yl)-5-chlorthiophen-2-carboxamid

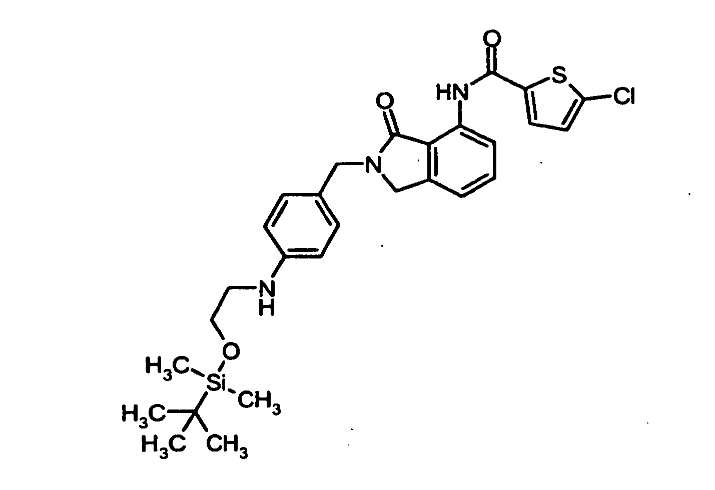

Eine Lösung aus 220 mg (0.5 mmol) 5-Chlor-*N*-(2-{4-[(2-hydroxycthyl)amino]benzyl}-3-oxo-2,3-dihydro-1*H*-isoindol-4-yl)thiophen-2-carboxamid in 8 ml Dimethylformamid wird bei 0°C mit 68 mg (1.0 mmol, 2 eq.) Imidazol und 90 mg (0.6 mmol, 1.2 eq.) *tert*.-Butyldimethylsilylchlorid versetzt. Das Reaktionsgemisch wird 2 d bei RT gerührt und im Vakuum eingeengt. Nach Zugabe von Wasser/Dichlormethan und Phasentrennung wird die wässrige Phase mehrmals mit Dichlormethan extrahiert. Die vereinigten, organischen Phasen werden über Natriumsulfat getrocknet, filtriert und im Vakuum eingeengt. Die Titelverbindung wird ohne weitere Reinigung in der nächsten Reaktion eingesetzt.
Ausbeute: 273 mg (94% d. Th.)
LC-MS (Methode 3): Rₜ = 3.63 min;
MS (ESIpos): m/z = 556 [M+H]⁺.

### Stufe c): N-(2-{4-[(2-{[tert.-Butyl(dimethyl)silyl]oxy}ethyl)(cyano)amino]benzyl}-3-oxo-2,3-dihydro-1H-isoindol-4-yl)-5-chlorthiophen-2-carboxamid

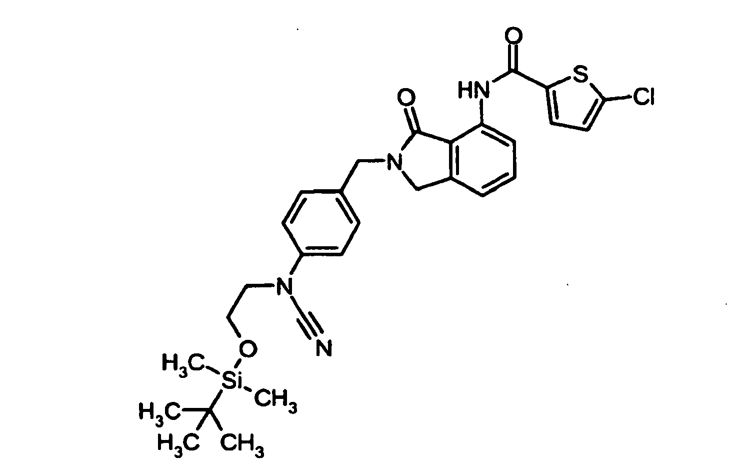

Eine Lösung aus 270 mg (0.46 mmol) *N*-(2-{4-[(2-{[*tert*.-Butyl(dimethyl)silyl]oxy}ethyl)-amino]benzyl}-3-oxo-2,3-dihydro-1*H*-isoindol-4-yl)-5-chlorthiophen-2-carboxamid in 5 ml Tetrahydrofuran wird unter Argon bei RT mit 122 mg (1.46 mmol, 3.2 eq.) Natriumhydrogencarbonat und insgesamt 0.36 ml Bromcyan-Lösung (3 M in Dichlormethan, 1.1 mmol, 2.3 eq.) versetzt und 4 d bei 40°C gerührt. Nach Zugabe von Wasser/Dichlormethan und Phasentrennung wird die wässrige Phase mit Dichlormethan extrahiert. Die vereinigten, organischen Phasen werden mit gesättigter, wässriger Natriumhydrogencarbonat-Lösung und mit gesättigter, wässriger Natriumchlorid-Lösung gewaschen, über Natriumsulfat getrocknet, filtriert und im Vakuum eingeengt. Die Titelverbindung wird mittels Flash-Chromatographie (Kieselgel, Dichlormethan/Methanol 100:1) isoliert.
Ausbeute: 228 mg (79% d. Th.)
LC-MS (Methode 3): Rₜ = 3.47 min;
MS (ESIpos): m/z = 581 [M+H]⁺;
¹H-NMR (400 MHz, DMSO-d₆): δ = 11.34 (s, 1H), 8.31 (d, 1H), 7.71 (d, 1H), 7.65 (t, 1H), 7.43 (d, 2H), 7.40 (d, 1H), 7.32 (d, 1H), 7.23 (d, 2H), 4.78 (s, 2H), 4.46 (s, 2H), 3.88 (s, 4H), 0.82 (s, 9H), 0.0 (s, 6H).

### Stufe d): 5-Chlor-N-{2-[4-(2-imino-1,3-oxazolidin-3-yl)benzyl]-3-oxo-2,3-dihydro-1H-isoindol-4-yl}thiophen-2-carboxamid-Methansulfonat

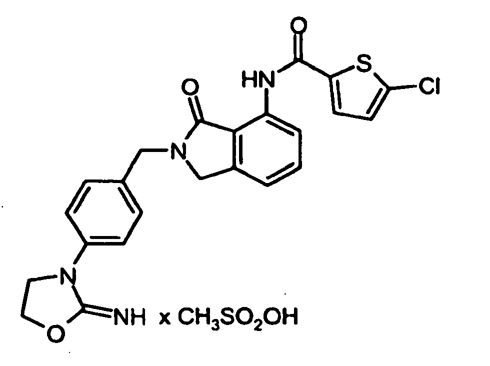

Eine Suspension aus 228 mg (0.39 mmol) *N*-(2-{4-[(2-{[*tert*.-Butyl(dimethyl)silyl]oxy}ethyl)-(cyano)amino]benzyl}-3-oxo-2,3-dihydro-1*H*-isoindol-4-yl)-5-chlorthiophen-2-carboxamid in 20 ml Acetonitril wird bei RT mit 53 µl (0.82 mmol, 2.1 eq.) Methansulfonsäure und weiteren 25 ml Acetonitril versetzt. Die Reaktionslösung wird über Nacht bei RT gerührt und im Vakuum eingeengt. Die Titelverbindung wird mittels Verrühren des Rohproduktes in Dichlormethan isoliert.
Ausbeute: 206 mg (80% d. Th.)
LC-MS (Methode 5): Rₜ = 3.24 min;
MS (ESIpos): m/z = 467 [M+H]⁺ (freie Base);
¹H-NMR (400 MHz, DMSO-d₆): δ = 11.27 (s, 1H), 9.61 (br. s, 1H), 8.80 (br. s, 1H), 8.28 (d, 1H), 7.65 (d, 1H), 7.62 (t, 1H), 7.52 (s, 4H), 7.36 (d, 1H), 7.31 (d, 1H), 4.83 (t, 2H), 4.81 (s, 2H), 4.49 (s. 2H), 4.22 (t, 2H), 2.33 (s, 3H).

### Beispiel 5

### 5-Chlor-N-{2-[3-(2-imino-1,3-oxazolidin-3-yl)benzyl]-3-oxo-2,3-dihydro-1H-isoindol-4-yl}thiophen-2-carboxamid-Methansulfonat

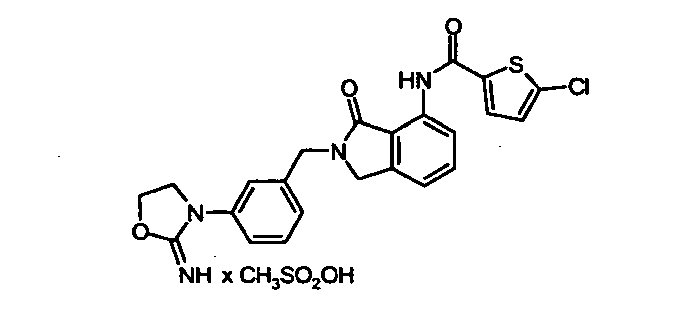

### Stufe a): 5-Chlor-N-(2-{3-[(2-hydroxyethyl)amino]benzyl}-3-oxo-2,3-dihydro-1H-isoindol-4-yl)thiophen-2-carboxamid

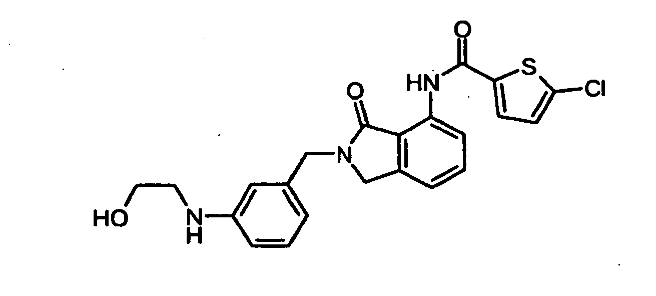

Eine Suspension aus 75 mg (0.4 mmol, 0.1 eq.) Kupfer(I)iodid und 3.4 g (16 mmol, 4 eq.) Kaliumphosphat in 50 ml Isopropanol wird unter Argon bei RT mit 0.88 ml (16 mmol, 4 eq.) 1,2-Ethandiol, 2.0 g (4.0 mmol) 5-Chlor-*N*-[2-(3-iodbenzyl)-3-oxo-2,3-dihydro-1*H*-isoindol-4-yl]thiophen-2-carboxamid (Beispiel 8A) und 1.4 ml (24 mmol, 6 eq.) 2-Aminoethanol versetzt. Das Reaktionsgemisch wird für 2.5 d bei 80°C gerührt, nach Abkühlen auf RT filtriert und der Rückstand mit Isopropanol gewaschen. Die vereinigten Filtrate werden im Vakuum eingeengt. Die Titelverbindung wird mittels Flash-Chromatographie (Kieselgel, Dichlormethan/Methanol 80:1 → 30:1) isoliert.
Ausbeute: 489 mg (83% Reinheit, 23% d. Th.)
LC-MS (Methode 1): Rₜ = 2.83 min;
MS (ESIpos): m/z = 442 [M+H]⁺;
¹H-NMR (400 MHz, DMSO-d₆): δ = 11.33 (s, 1H), 8.26 (d, 1H), 7.66 (d, 1H), 7.59 (t, 1H), 7.35 (d, 1H), 7.28 (d, 1H), 7.05 (t, 1H), 6.53-6.43 (2d, 3H), 5.58 (t, 1H), 4.64 (t, 1H), 4.62 (s, 2H), 4.42 (s, 2H), 3.52 (q, 2H), 3.06 (q, 2H).

### Stufe b): N-(2-{3-[(2-{[tert.-Butyl(dimethyl)silyl]oxy}ethyl)amino]benzyl}-3-oxo-2,3-dihydro-1H-isoindol-4-yl)-5-chlorthiophen-2-carboxamid

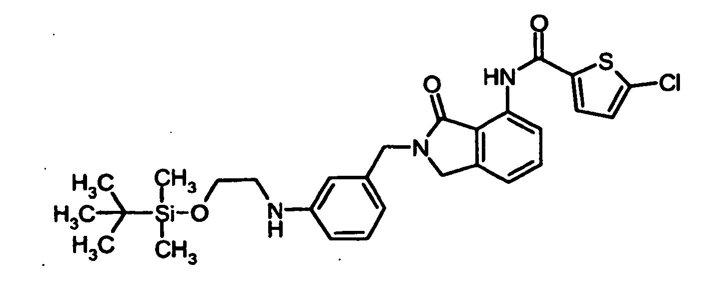

Eine Lösung aus 490 mg (83% Reinheit, 0.9 mmol) 5-Chlor-*N*-(2-{3-[(2-hydroxyethyl)amino]-benzyl}-3-oxo-2,3-dihydro-1*H*-isoindol-4-yl)thiophen-2-carboxamid in 30 ml Tetrahydrofuran wird bei RT mit 125 mg (1.8 mmol, 2 eq.) Imidazol und 166 mg (1.1 mmol, 1.2 eq.) *tert.-*Butyldimethylsilylchlorid versetzt. Das Reaktionsgemisch wird 3 d bei RT gerührt. Nach Zugabe von Wasser/Dichlormethan und Phasentrennung wird die wässrige Phase mehrmals mit Dichlormethan extrahiert. Die vereinigten, organischen Phasen werden mit Wasser und mit gesättigter, wässriger Natriumchlorid-Lösung gewaschen, über Natriumsulfat getrocknet, filtriert und im Vakuum eingeengt. Die Titelverbindung wird mittels präparativer RP-HPLC (CromSil C18, Acetonitril/Wasser-Gradient) isoliert.
Ausbeute: 343 mg (67% d. Th.)
LC-MS (Methode 7): Rₜ = 3.63 min;
MS (ESIpos): m/z = 556 [M+H]⁺;
¹H-NMR (400 MHz, DMSO-d₆): δ = 11.35 (s, 1H), 8.27 (d, 1H), 7.66 (d, 1H), 7.59 (t, 1H), 7.35 (d, 1H), 7.29 (d, 1H), 7.05 (t, 1H), 6.53-6.43 (m, 3H), 5.59 (t, 1H), 4.62 (s, 2H), 4.42 (s, 2H), 3.65 (t, 2H), 3.11 (q, 2H), 0.80 (s, 9H), 0.0 (s, 6H).

### Stufe c): N-(2-{3-[(2-{[tert.-Butyl(dimethyl)silyl]oxy}ethyl)(cyano)amino]benzyl}-3-oxo-2,3-dihydro-1H-isoindol-4-yl)-5-chlorthiophen-2-carboxamid

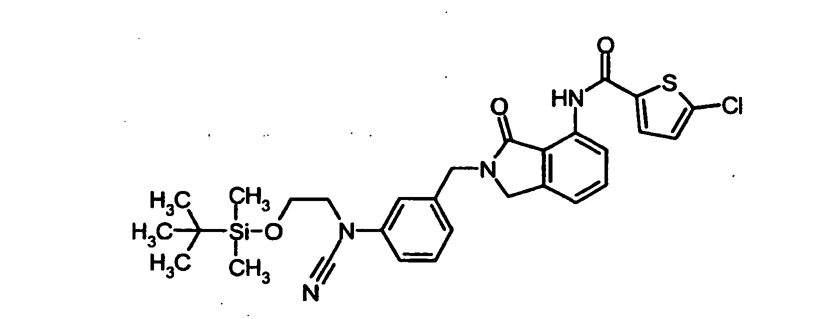

Eine Lösung aus 339 mg (0.61 mmol) *N*-(2-{3-[(2-{[*tert*.-Butyl(dimethyl)silyl]oxy}ethyl)-amino]benzyl}-3-oxo-2,3-dihydro-1*H*-isoindol-4-yl)-5-chlorthiophen-2-carboxamid in 15 ml Tetrahydrofuran wird unter Argon bei RT mit 154 mg (1.83 mmol, 3 eq.) Natriumhydrogencarbonat und insgesamt 0.43 ml Bromcyan-Lösung (3 M in Dichlormethan, 1.3 mmol, 2.1 eq.) versetzt und 6 d bei 40°C gerührt. Nach Zugabe von Wasser/Dichlormethan und Phasentrennung wird die wässrige Phase mit Dichlormethan extrahiert. Die vereinigten, organischen Phasen werden mit gesättigter, wässriger Natriumhydrogencarbonat-Lösung und mit gesättigter, wässriger Natriumchlorid-Lösung gewaschen, über Natriumsulfat getrocknet, filtriert und im Vakuum eingeengt. Die Titelverbindung wird mittels präparativer RP-HPLC (CromSil C18, Acetonitril/Wasser-Gradient) isoliert.
Ausbeute: 273 mg (73% d. Th.)
LC-MS (Methode 1): Rₜ = 3.32 min;
MS (ESIpos): m/z= 581 [M+H]⁺;
¹H-NMR (400 MHz, DMSO-d₆): δ = 11.41 (s, 1H), 8.38 (d, 1H), 7.77 (d, 1H), 7.72 (t, 1H), 7.53 (t, 1H), 7.48 (d, 1H), 7.40 (d, 1H), 7.30 (s, 1H), 7.27 (d, 1H), 7.19 (d, 1H), 4.89 (s, 2H), 4.55 (s, 2H), 3.98-3.88 (m, 4H), 0.86 (s, 9H), 0.0 (s, 6H).

### Stufe d): 5-Chlor-N-{2-[3-(2-imino-1,3-oxazolidin-3-yl)benzyl]-3-oxo-2,3-dihydro-1H-isoindol-4-yl}thiophen-2-carboxamid-Methansulfonat

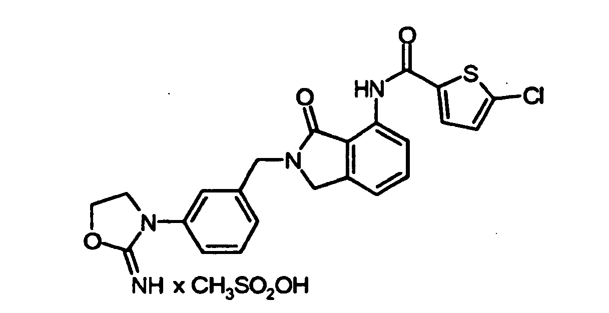

Eine Lösung aus 272 mg (0.44 mmol) *N*-(2-{3-[(2-{[*tert*.-Butyl(dimethyl)silyl]oxy}ethyl)(cyano)amino]benzyl}-3-oxo-2,3-dihydro-1*H*-isoindol-4-yl)-5-chlorthiophen-2-carboxamid in 15 ml Acetonitril wird bei RT mit 61 µl (0.93 mmol, 2.1 eq.) Methansulfonsäure versetzt und 20 h bei RT gerührt. Die Titelverbindung wird durch Filtration des entstandenen Niederschlages, Waschen mit Acetonitril und Trocknen im Vakuum isoliert.
Ausbeute: 97 mg (39% d. Th.)
HPLC (Methode 11): Rₜ = 4.48 min;
MS (DCI, NH₃): m/z = 484 [M+NH₄]⁺ (freie Base);
¹H-NMR (400 MHz, DMSO-d₆): δ = 11.29 (s, 1H), 9.58 (br. s, 1H), 8.80 (br. s, 1H), 8.28 (d, 1H), 7.64 (d, 1H), 7.63-7.53 (m, 2H), 7.49-7.41 (m, 3H), 7.36 (d, 1H), 7.31 (d, 1H), 4.82 (s, 2H und t, 2H), 4.52 (s, 2H), 4.23 (t, 2H), 2.29 (s, 3H).

### Beispiel 6

### 5-Chlor-N-{2-[3-(2-imino-1,3-oxazolidin-3-yl)benzyl]-1-oxo-2,3-dihydro-1H-isoindol-4-yl}thiophen-2-carboxamid-Methansulfonat

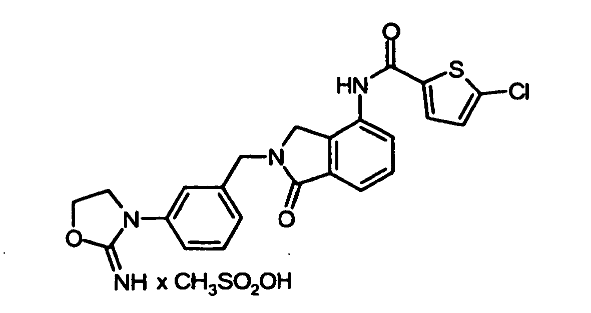

### Stufe a): 5-Chlor-N-(2-(3-[(2-hydroxyethyl)amino]benzyl)-1-oxo-2,3-dihydro-1H-isoindol-4-yl)thiophen-2-arboxamid

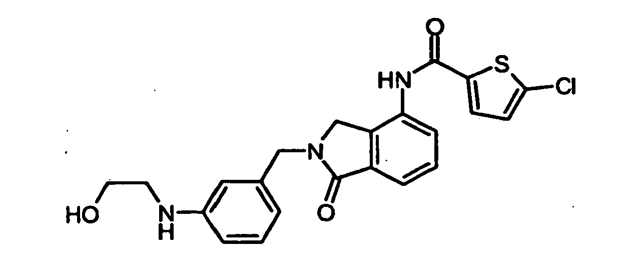

Eine Suspension aus 59 mg (0.3 mmol, 0.1 eq.) Kupfer(I)iodid und 2.6 g (12.4 mmol, 4 eq.) Kaliumphosphat in 65 ml Isopropanol wird unter Argon bei RT mit 0.69 ml (12.4 mmol, 4 eq.) 1,2-Ethandiol, 1.9 g (3.1 mmol) 5-Chlor-*N*-[2-(3-iodbenzyl)-1-oxo-2,3-dihydro-1*H*-isoindol-4-yl]-thiophen-2-carboxamid (Beispiel 10A) und 1.12ml (18.6 mmol, 6 eq.) 2-Aminoethanol versetzt. Das Reaktionsgemisch wird für 3 d bei 80°C gerührt, nach Abkühlen auf RT filtriert und der Rückstand mit Isopropanol gewaschen. Die vereinigten Filtrate werden im Vakuum eingeengt. Die Titelverbindung wird mittels Flash-Chromatographie (Kieselgel, Dichlormethan/Methanol 60:1 → 20: 1) isoliert.
Ausbeute: 780 mg (85% Reinheit, 49% d. Th.)
LC-MS (Methode 7): Rₜ = 2.07 min;
MS (ESIpos): m/z = 442 [M+H]⁺;
¹H-NMR (500 MHz, DMSO-d₆): δ = 10.39 (s, 1H), 7.87 (d, 1H), 7.65 (d, 1H), 7.61 (d, 1H), 7.54 (t, 1H), 7.28 (d, 1H), 7.03 (t, 1H), 6.50-6.43 (m, 2H), 6.41 (d, 1H), 5.57 (t, 1H), 4.65 (t, 1H), 4.60 (s, 2H), 4.38 (s, 2H), 3.51 (q, 2H), 3.04 (q, 2H).

### Stufe b): N-(2-{3-[(2-{[tert.-Butyl(dimethyl)silyl]oxy}ethyl)amino]benzyl}-1-oxo-2,3-dihydro-1H-isoindol-4-yl)-5-chlorthiophen-2-carboxamid

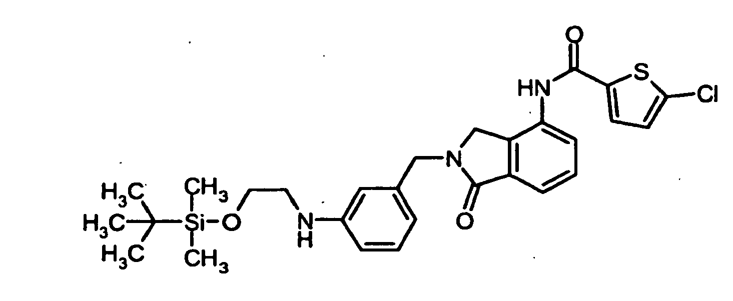

Eine Lösung aus 698 mg (85% Reinheit, 1.3 mmol) 5-Chlor-*N*-(2-{3-((2-hydroxyethyl)amino]-benzyl}-1-oxo-2,3-dihydro-1*H*-isoindol-4-yl)thiophen-2-carboxamid in 53 ml Tetrahydrofuran wird bei RT mit 183 mg (2.7 mmol, 2 eq.) Imidazol und 243 mg (1.6 mmol, 1.2 eq.) *tert.-*Butyldimethylsilylchlorid versetzt. Das Reaktionsgemisch wird 1 d bei RT gerührt, nochmals mit 162 mg (1.1 mmol, 0.8 eq.) *tert*.-Butyldimethylsilylchlorid versetzt und weitere 2 h bei RT gerührt. Nach Zugabe von Wasser/Dichlormethan und Phasentrennung wird die wässrige Phase mehrmals mit Dichlormethan extrahiert. Die vereinigten, organischen Phasen werden mit Wasser und mit gesättigter, wässriger Natriumchlorid-Lösung gewaschen, über Natriumsulfat getrocknet, filtriert und im Vakuum eingeengt. Die Titelverbindung wird mittels präparativer RP-HPLC (CromSil C18, Acetonitril/Wasser-Gradient) isoliert.
Ausbeute: 391 mg (52% d. Th.)
LC-MS (Methode 8): Rₜ = 3.14 min;
MS (ESIpos): m/z = 556 [M+H]⁺;
¹H-NMR (500 MHz, DMSO-d₆): δ = 10.41 (s, 1H), 7.39 (d, 1H), 7.68 (d, 1H), 7.65 (d, 1H), 7.59 (t, 1H), 7.30 (d, 1H), 7.06 (t, 1H), 6.53-6.47 (m, 2H), 6.55 (d, 1H), 5.61 (t, 1H), 4.62 (s, 2H), 4.49 (s, 2H), 3.69 (t, 2H), 3.12 (q, 2H), 0.83 (s, 9H), 0.0 (s, 6H).

### Stufe c): N-(2-{3-[(2-{[tert.-Butyl(dimethyl)silyl]oxy}ethyl)(cyano)amino]benzyl}-1-oxo-2,3-dihydro-1H-isoindol-4-yl)-5-chlorthiophen-2-carboxamid

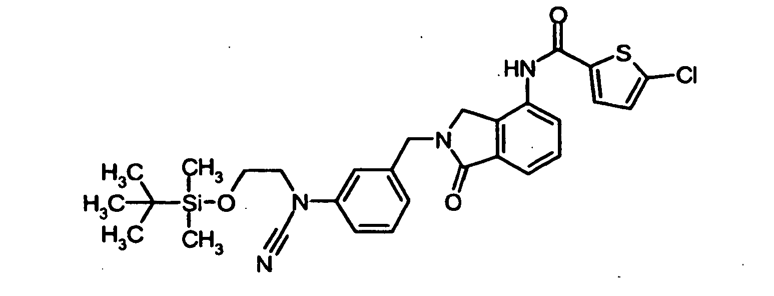

Eine Lösung aus 386mg (0.69 mmol) *N*-(2-{3-(2-{[*tert*.-Butyl(dimethyl)silyl]oxy}-ethyl)-aminobenzyl}-1-oxo-2,3-dihydro-1*H*-isoindol-4-yl)-5-chlorthiophen-2-carboxamid in 15 ml Tetrahydrofuran wird unter Argon bei RT mit 175 mg (2.08 mmol, 3 eq.) Natriumhydrogencarbonat und insgesamt 0.44 ml Bromcyan-Lösung (3 M in Dichlormethan, 1.32 mmol, 1.9 eq.) versetzt und 4 d bei 40°C gerührt. Nach Zugabe von Wasser/Dichlormethan und Phasentrennung wird die wässrige Phase mit Dichlormethan extrahiert. Die vereinigten, organischen Phasen werden mit gesättigter, wässriger Natriumhydrogencarbonat-Lösung und mit gesättigter, wässriger Natriumchlorid-Lösung gewaschen, über Natriumsulfat getrocknet, filtriert und im Vakuum eingeengt. Die Titelverbindung wird ohne weitere Reinigung in der nächsten Reaktion eingesetzt.
Ausbeute: 375 mg (93% d. Th.)
LC-MS (Methode 7): Rₜ = 3.12 min;
MS (ESIpos): m/z = 581 [M+H]⁺;
¹H-NMR (500 MHz, DMSO-d₆): δ = 10.56 (s, 1H), 7.97 (d, 1H), 7.75 (t, 2H), 7.68 (t, 1H), 7.50 (t, 1H), 7.39 (d, 1H), 7.27 (s, 1H), 7.24 (dd, 1H), 7.11 (d, 1H), 4.87 (s, 2H), 4.46 (s, 2H), 3.93 (s, 4H), 0.86 (s, 9H), 0.0 (s, 6H).

### Stufe d): 5-Chlor-N-{2-[3-(2-imino-1,3-oxazolidin-3-yl)benzyl]-1-oxo-2,3-dihydro-1H-isoindol-4-yl}thiophen-2-carboxamid-Methansulfonat

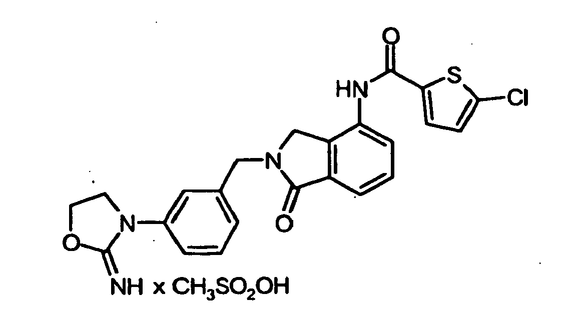

Eine Lösung aus 370 mg (0.64 mmol) *N*-(2-{3-[(2-{[*tert*.-Butyl(dimethyl)silyl]oxy}ethyl)(cyano)amino]benzyl}-1-oxo-2,3-dihydro-1*H*-isoindol-4-yl)-5-chlorthiophen-2-carboxamid in 25 ml Acetonitril wird bei RT mit 82 µl (1.34 mmol, 2.1 eq.) Methansulfonsäure versetzt und über Nacht bei RT gerührt. Das Reaktionsgemisch wird im Vakuum eingeengt und die Titelverbindung durch Verrühren des Rohproduktes in Aceton isoliert.
Ausbeute: 270 mg (74% d. Th.)
HPLC (Methode 7): Rₜ = 1.42 min;
MS (ESIpos): m/z = 467 [M+H]⁺ (freie Base);
¹H-NMR (500 MHz, DMSO-d₆): δ = 10.50 (s, 1H), 9.59 (br. s, 1H), 8.83 (br. s, 1H), 7.87 (d, 1H), 7.68-7.60 (m, 2H), 7.60-7.50 (m, 2H), 7.47-7.41 (m, 2H), 7.40 (d, 1H), 7.29 (d, 1H), 4.86-4.73 (s, 2H und t, 2H), 4.46 (s, 2H), 4.21 (t, 2H), 2.31 (s, 3H).

### B. Bewertung der pharmakologischen Wirksamkeit

Die erfindungsgemäßen Verbindungen wirken insbesondere als selektive Inhibitoren des Blutgerinnungsfaktors Xa und hemmen nicht oder erst bei deutlich höheren Konzentrationen auch andere Serinproteasen wie Plasmin oder Trypsin.

Als "selektiv" werden solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Plasmin und Trypsin, um mindestens das 100-fache kleiner sind, wobei bezüglich der Testmethoden für die Selektivität Bezug genommen wird auf die im folgenden beschriebenen Testmethoden der Beispiele B.a.1) und B.a.2).

Die vorteilhaften pharmakologischen Eigenschaften der erfindungsgemäßen Verbindungen können durch folgende Methoden festgestellt werden:

### a) Testbeschreibungen (in vitro)

### a.1) Messung der Faktor Xa-Hemmung:

Die enzymatische Aktivität von humanem Faktor Xa (FXa) wird über die Umsetzung eines für den FXa-spezifischen chromogenen Substrats gemessen. Dabei spaltet der Faktor Xa aus dem chromogenen Substrat p-Nitroanilin ab. Die Bestimmungen werden wie folgt in Mikrotiterplatten durchgeführt:

Die Prüfsubstanzen werden in unterschiedlichen Konzentrationen in DMSO gelöst und für 10 Minuten mit humanem FXa (0.5 nmol/l gelöst in 50 mmol/l Tris-Puffer [*C*,*C*,*C*-Tris(hydroxymethyl)aminomethan], 150 mmol/l NaCl, 0.1% BSA [bovine serum albumine], pH = 8.3) bei 25°C inkubiert. Als Kontrolle dient reines DMSO. Anschließend wird das chromogene Substrat (150 µmol/l Pefachrome^{®} FXa der Firma Pentapharm) hinzugefügt. Nach 20 Minuten Inkubationsdauer bei 25°C wird die Extinktion bei 405 nm bestimmt. Die Extinktionen der Testansätze mit Prüfsubstanz werden mit den Kontrollansätzen ohne Prüfsubstanz verglichen und daraus die IC₃₀-Werte berechnet.

Repräsentative Wirkdaten aus diesem Test sind in der folgenden Tabelle 1 aufgeführt:

**Tabelle 1**

| **Beispiel Nr.** | **IC₅₀ [nM]** |
|---|---|
| 5 | 1.1 |

### a.2) Bestimmung der Selektivität:

Zum Nachweis der selektiven FXa-Inhibition werden die Prüfsubstanzen auf ihre Hemmung anderer humaner Serinproteasen wie Trypsin und Plasmin hin untersucht. Zur Bestimmung der enzymatischen Aktivität von Trypsin (500 mU/ml) und Plasmin (3.2 nmol/l) werden diese Enzyme in Tris-Puffer (100 mmol/l, 20 mmol/l CaCl₂, pH = 8.0) gelöst und für 10 Minuten mit Prüfsubstanz oder Lösungsmittel inkubiert. Anschließend wird durch Zugabe der entsprechenden spezifischen chromogenen Substrate (Chromozym Trypsin^{®} und Chromozym Plasmin^{®}; Fa. Roche Diagnostics) die enzymatische Reaktion gestartet und die Extinktion nach 20 Minuten bei 405 nm bestimmt. Alle Bestimmungen werden bei 37°C durchgeführt. Die Extinktionen der Testansätze mit Prüfsubstanz werden mit den Kontrollproben ohne Prüfsubstanz verglichen und daraus die IC₅₀-Werte berechnet.

### a.3) Bestimmung der antikoagulatorischen Wirkung:

Die antikoagulatorische Wirkung der Prüfsubstanzen wird *in vitro* in Human- und Kaninchenplasma bestimmt. Dazu wird Blut unter Verwendung einer 0.11 molaren Natriumcitrat-Lösung als Vorlage in einem Mischungsverhältnis Natriumcitrat/Blut 1:9 abgenommen. Das Blut wird unmittelbar nach der Abnahme gut gemischt und 10 Minuten bei ca. 2500 g zentrifugiert. Der Überstand wird abpipettiert. Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Hemoliance^{®} RecombiPlastin, Fa. Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Kaninchen):

Nüchterne Kaninchen (Stamm: Esd: NZW) werden durch intramuskuläre Gabe einer Rompun/ Ketavet-Lösung narkotisiert (5 mg/kg bzw. 40 mg/kg). Die Thrombusbildung wird in einem arteriovenösen Shunt in Anlehnung an die von C.N. Berry et al. [Semin. Thromb. Hemost. 1996, 22, 233-241] beschriebene Methode ausgelöst. Dazu werden die linke Vena jugularis und die rechte Arteria carotis freipräpariert. Ein extracorporaler Shunt wird mittels eines 10 cm langen Venenkatheders zwischen den beiden Gefäßen gelegt. Dieser Katheder ist in der Mitte in einen weiteren, 4 cm langen Polyethylenschlauch (PE 160, Becton Dickenson), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden. Die Prüfsubstanzen werden vor Anlegung des extrakorporalen Kreislaufs entweder intravenös über eine Ohrvene oder oral mittels Schlundsonde verabreicht.

### c) Löslichkeitsassay

Benötigte Reagenzien:
- PBS-Puffer pH 7.4: 90.00 g NaCl p.a. (z.B. Merck Art. Nr. 1.06404.1000), 13.61 g KH₂PO₄ p.a. (z.B. Merck Art. Nr. 1.04873.1000) und 83.35 g 1N NaOH (z.B. Bernd Kraft GmbH Art. Nr. 01030.4000) in einen 1 1 Messkolben einwiegen, mit Wasser auffüllen und ca. 1 Stunde rühren.
- Acetatpuffer pH 4.6: 5.4 g Natriumacetat x 3 H₂O p.a. (z.B. Merck Art. Nr. 1.06267.0500) in einen 100 ml Messkolben einwiegen, in 50 ml Wasser lösen, mit 2.4 g Eisessig versetzen, auf 100 ml mit Wasser auffüllen, pH-Wert überprüfen und falls notwendig auf pH 4.6 einstellen.
- Dimethylsulfoxid (z.B. Baker Art. Nr. 7157.2500)
- destilliertes Wasser

### Herstellung der Kalibrierlösungen:

*Herstellung der Ausgangslösung für Kalibrierlösungen (Stammlösung):* In ein 2 ml Eppendorf-Safe-Lock Tube (Eppendorf Art. Nr. 0030 120.094) werden ca. 0.5 mg des Wirkstoffes genau eingewogen, zu einer Konzentration von 600 µg/ml mit DMSO versetzt (z.B. 0.5 mg Wirkstoff + 833 µl DMSO) und bis zur vollständigen Lösung mittels eines Vortexers geschüttelt.

*Kalibrierlösung 1 (20* µ*g*/*ml):* 34.4 µl der Stammlösung werden mit 1000 µl DMSO versetzt und homogenisiert.

*Kalibrierlösung 2 (2.5* µ*g*/*ml):* 100 µl der Kalibrierlösung 1 werden mit 700 µl DMSO versetzt und homogenisiert.

### Herstellung der Probenlösungen:

*Probenlösung für Löslichkeit bis 10 g*/*l in PBS-Puffer pH 7.4:* In ein 2 ml Eppendorf-Safe-Lock Tube (Eppendorf Art. Nr. 0030 120.094) werden ca. 5 mg des Wirkstoffes genau eingewogen und zu einer Konzentration von 5 g/l mit PBS-Puffer pH 7.4 versetzt (z.B. 5 mg Wirkstoff + 500 µl PBS-Puffer pH 7.4).

*Probenlösung für Löslichkeit bis 10 g*/*l in Acetatpuffer pH 4.6:* In ein 2 ml Eppendorf-Safe-Lock Tube (Eppendorf Art. Nr. 0030 120.094) werden ca. 5 mg des Wirkstoffes genau eingewogen und zu einer Konzentration von 5 g/l mit Acetatpuffer pH 4.6 versetzt (z.B. 5 mg Wirkstoff + 500 µl Acetatpuffer pH 4.6).

*Probenlösung für Löslichkeit bis 10 g*/*l inWasser:* In ein 2 ml Eppendorf-Safe-Lock Tube (Eppendorf Art. Nr. 0030 120.094) werden ca. 5 mg des Wirkstoffes genau eingewogen und zu einer Konzentration von 5 g/l mit Wasser versetzt (z.B. 5 mg Wirkstoff + 500 µl Wasser).

### Durchführung:

Die so hergestellten Probenlösungen werden 24 Stunden bei 1400 rpm mittels eines temperierbaren Schüttlers (z.B. Eppendorf Thermomixer comfort Art. Nr. 5355 000.011 mit Wechselblock Art. Nr. 5362.000.019) bei 20°C geschüttelt. Von diesen Lösungen werden jeweils 180 µl abgenommen und in Beckman Polyallomer Centrifuge Tubes (Art. Nr. 343621) überfährt. Diese Lösungen werden 1 Stunde mit ca. 223.000 *g zentrifugiert (z.B. Beckman Optima L-90K Ultracentrifuge mit Type 42.2 Ti Rotor bei 42.000 rpm). Von jeder Probenlösung werden 100 µl des Überstandes abgenommen und 1:5, 1:100 und 1:1000 mit dem jeweils verwendeten Lösungsmittel (Wasser, PBS-Puffer 7.4 oder Acetatpuffer pH 4.6) verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/l ausgedrückt.

Analysensequenz:
1. Kallibrierlösung 2.5 mg/ml
2. Kallibrierlösung 20 µg/ml
3. Probenlösung 1:5
4. Probenlösung 1:100
5. Probenlösung 1:1000

### HPLC-Methode für Säuren:

Agilent 1100 mit DAD (G1315A), quat. Pumpe (G1311A), Autosampler CTC HTS PAL, Degaser (G1322A) and Säulenthermostat (G1316A); Säule: Phenomenex Gemini C18, 50 x 2 mm, 5 µ; Temperatur: 40°C; Eluent A: Wasser/Phosphorsäure pH 2; Eluent B: Acetonitril; Flussrate: 0.7 ml/mim; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) and Säulenthermostat (G1316A); Säule: VDSoptilab Kromasil 100 C 18, 60 x 2.1 mm, 3.5 µ; Temperatur: 30°C; Eluent A: Wasser + 5 ml Perchlorsäure/l; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgenzäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel 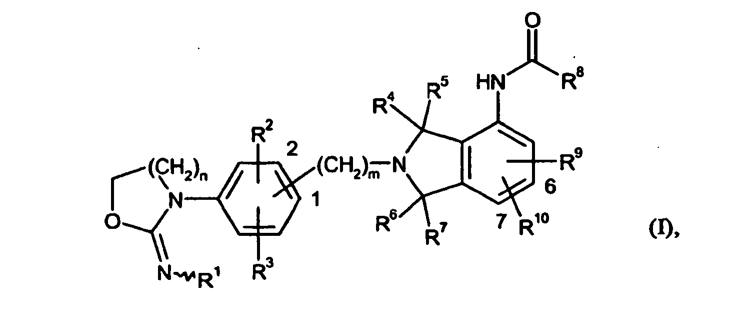 in welcher
n für die Zahl 1, 2 oder 3 steht,
m für die Zahl 0, 1 oder 2 steht, und die (CH₂)ₘ-Gruppe in 1 oder 2 Position an den Phenyl-Ring gebunden ist,
R¹ für Wasserstoff, Cyano, Hydroxy, C₁-C₄-Alkyl, C₁-C₄-Alkylcarbonyl, C₃-C₆- Cycloalkylcarbonyl, Phenylcarbonyl, 4- bis 7-gliedriges Heterocyclylcarbonyl oder 5- oder 6-gliedriges Heteroarylcarbonyl steht,
R² für Wasserstoff, Fluor, Chlor, Cyano, Hydroxy, Amino, Trifluormethyl, Trifluor- methoxy, C₁-C₄-Alkyl, C₁-C₄-Alkoxy, C₁-C₄-Alkoxymethyl, C₁-C₄-Alkylamino, C₃- C₆-Cycloalkyl, Aminocarbonyl, C₁-C₄-Alkoxycarbonyl oder C₁-C₄-Alkyl- aminocarbonyl steht,
R³ für Wasserstoff, Fluor, Chlor, Cyano, Hydroxy, Amino, Trifluormethyl, Trifluor- methoxy, C₁-C₄-Alkyl, C₁-C₄-Alkoxy, C₁-C₄-Alkoxymethyl, C₁-C₄-Alkylamino, C₃- C₆-Cycloalkyl, Aminocarbonyl, C₁-C₄-Allcoxycarbonyl oder C₁-C₄-Alkyl- aminocarbonyl steht,
R⁴ und R⁵ für Wasserstoff stehen,
und
R⁶ und R⁷ zusammen mit dem Kohlenstoffatom, an das sie gebunden sind, eine Carbonylgruppe bilden,
oder
R⁴ und R⁵ zusammen Carbonylgruppe mit dem Kohlenstoffatom, an das sie gebunden sind, eine bilden,
und
R⁶ und R⁷ für Wasserstoff stehen,
oder
R⁴ und R⁵ zusammen mit dem Kohlenstoffatom, an das sie gebunden sind, eine Carbonylgruppe bilden,
und
R⁶ und R⁷ zusammen mit dem Kohlenstoffatom, an das sie gebunden sind, eine Carbonylgruppe bilden,
R⁸ für Phenyl, Pyridyl, Pyrazinyl, Pyrimidinyl, Pyridazinyl oder Thienyl steht, wobei Phenyl, Pyridyl, Pyrazinyl, Pyrimidinyl und Pyridazinyl substituiert sind mit einem Substituenten R¹¹ und/oder einem Substituenten R¹² oder mit zwei verschiedenen Substituenten R¹¹ oder mit zwei verschiedenen Substituenten R¹²,
wobei
R¹¹ an ein Kohlenstoffatom gebunden ist, das nicht einem Stickstoffatom im Ring benachbart ist, und für Wasserstoff, Fluor, Chlor, Cyano, Ethinyl, C₁-C₄-Alkyl, C₁-C₄-Alkoxy oder C₃-C₆- Cycloalkyl steht,
R¹² an ein Kohlenstoffatom gebunden ist, das einem Stickstoffatom im Ring benachbart ist, und für Wasserstoff, Amino, C₁-C₄-Alkyl, C₁- C₄-Alkylarnino oder C₃-C₆-Cycloalkyl steht,
und
wobei Thienyl substituiert ist mit einem Substituenten R¹³ und einem Substituenten R¹⁴,
wobei
R¹³ an ein Kohlenstoffatom gebunden ist, das dem Schwefelatom im Ring benachbart ist, und für Wasserstoff, Fluor, Chlor, Cyano, Ethinyl, C₁-C₄-Alkyl, C₁-C₄-Alkoxy oder C₃-C₆-Cycloalkyl steht,
R¹⁴ für Wasserstoff, Fluor, Chlor, Amino, C₁-C₄-Alkyl, C₁-C₄- Alkylamino oder C₃-C₆-Cycloalkyl steht,
R⁹ für Wasserstoff, Fluor, Chlor, Cyano, Hydroxy, Amino, Trifluormethyl, Trifluormethoxy, C₁-C₄-Alkyl, C₁-C₄-Alkoxy, C₁-C₄-Alkylamino, C₃-C₆-Cyclo- alkyl, Aminocarbonyl, C₁-C₄-Alkoxycarbonyl oder C₁-C₄-Alkylaminocarbonyl steht,
R¹⁰ für Wasserstoff, Fluor, Chlor, Cyano, Trifluormethyl, Trifluormethoxy, C₁-C₄- Alkyl, C₁-C₄-Alkoxy oder C₃-C₆-Cycloalkyl steht,
und
wobei R⁹ an die 6-Position und R¹⁰ an die 7-Position des Isoindolin-Rings gebunden ist, oder
wobei R⁹ an die 7-Position und R¹⁰ an die 6-Position des Isoindolin-Rings gebunden ist, oder eines ihrer Salze, ihrer Solvate oder der Solvate ihrer Salze.

2. Verbindung nach Anspruch 1, **dadurch gekennzeichnet, dass**
n für die Zahl 1, 2 oder 3 steht,
m für die Zahl 0, 1 oder 2 steht, und die (CH₂)ₘ-Gruppe in 1 oder 2 Position an den Phenyl-Ring gebunden ist,
R¹ für Wasserstoff, Cyano, Hydroxy oder C₁-C₄-Alkyl steht,
R² für Wasserstoff, Fluor, Chlor, Cyano, Hydroxy, C₁-C₄-Alkyl oder C₁-C₄-Alkoxy steht,
R³ für Wasserstoff, Fluor, Chlor, Cyano, Hydroxy, C₁-C₄-Alkyl, C₁-C₄-Alkoxy, C₁-C₄- Alkoxymethyl, Cyclopropyl, Aminocarbonyl, C₁-C₄-Alkoxycarbonyl oder C₁-C₄- Alkylaminocarbonyl steht,
R⁴ und R⁵ für Wasserstoff stehen, und
R⁶ und R⁷ zusammen mit dem Kohlenstoffatom, an das sie gebunden sind, eine Carbonylgruppe bilden,
oder
R⁴ und R⁵ zusammen mit dem Kohlenstoffatom, an das sie gebunden sind, eine Carbonylgruppe bilden,
und
R⁶ und R⁷ für Wasserstoff stehen,
oder
R⁴ und R⁵ zusammen mit dem Kohlenstoffatom, an das sie gebunden sind, eine Carbonylgruppe bilden,
und
R⁶ und R⁷ zusammen mit dem Kohlenstoffatom, an das sie gebunden sind, eine Carbonylgruppe bilden,
R⁸ für eine Gruppe der Formel 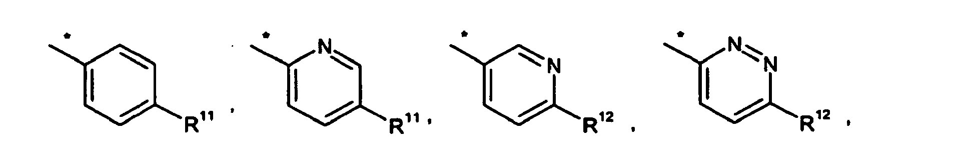 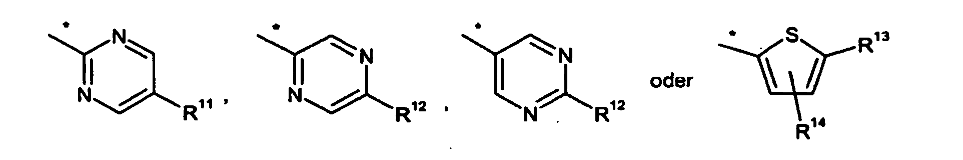 steht,
wobei
* die Anknüpfstelle an die Carbonylgruppe ist,
R¹¹ für Fluor, Chlor, Ethinyl, Methyl, Ethyl, Methoxy oder Ethoxy steht,
R¹² für Amino, Methyl, Methylamino oder Dimethylamino steht,
R¹³ für Fluor, Chlor, Ethinyl, Methyl, Ethyl, Methoxy oder Ethoxy steht,
und
R¹⁴ für Wasserstoff steht,
R⁹ für Wasserstoff, Fluor, Chlor, Cyano, Methyl, Methoxy, Aminocarbonyl, Methyl- aminocarbonyl oder Dimethylaminocarbonyl steht,
R¹⁰ für für Wasserstoff, Fluor, Chlor, Cyano, Trifluormethyl, Trifluormethoxy, Methyl Methoxy steht, und
oder
wobei R⁹ an die 6-Position und R¹⁰ an die 7-Position des Isoindolin-Rings gebunden ist,
oder
wobei R⁹ an die 7-Position und R¹⁰ an die 6-Position des Isoindolin-Rings gebunden ist.

3. Verbindung nach einem der Ansprüche 1 oder 2, **dadurch gekennzeichnet, dass**
n für die Zahl 1 oder 2 steht,
m für die Zahl 1 steht,
und die (CH₂)ₘ-Gruppe in 1 oder 2 Position an den Phenyl-Ring gebunden ist,
R¹ für Wasserstoff steht,
R² für Wasserstoff steht,
R³ für Wasserstoff, Fluor, Chlor, Cyano, Methyl, Ethyl, n-Propyl, Methoxy, Ethoxy oder Methoxymethyl steht,
R⁴ und R⁵ für Wasserstoff stehen,
und
R⁶ und R⁷ zusammen mit dem Kohlenstoffatom, an das sie gebunden sind, eine Carbonylgruppe bilden,
oder
R⁴ und R⁵ zusammen mit dem Kohlenstoffatom, an das sie gebunden sind, eine Carbonylgruppe bilden,
und
R⁶ und R⁷ für Wasserstoff stehen,
oder
R⁴ und R⁵ zusammen mit dem Kohlenstoffatom, an das sie gebunden sind, eine Carbonylgruppe bilden,
und
R⁶ und R⁷ zusammen mit dem Kohlenstoffatom, an das sie gebunden sind, eine Carbonylgruppe bilden,
R⁸ für eine Gruppe der Formel 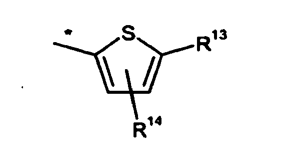 steht,
wobei
* die Anknüpfstelle an die Carbonylgruppe ist,
R¹³ für Fluor, Chlor oder Methyl steht,
und
R¹⁴ für Wasserstoff steht,
R⁹ für Wasserstoff steht,
R¹⁰ für Wasserstoff steht,
und
wobei R⁹ an die 6-Position und R¹⁰ an die 7-Position des Isoindolin-Rings gebunden ist, oder
wobei R⁹ an die 7-Position und R¹⁰ an die 6-Position des Isoindolin-Rings gebunden ist.

4. Verbindung nach einem der Ansprüche 1 bis 3, **dadurch gekennzeichnet, dass**
n für die Zahl 1 steht,
m für die Zahl 1 steht, und die (CH₂)ₘ-Gruppe in 1 oder 2 Position an den Phenyl-Ring gebunden ist,
R¹ für Wasserstoff steht,
R² für Wasserstoff steht,
R³ für Wasserstoff, Fluor, Chlor, Cyano oder Methyl steht,
R⁴ und R⁵ für Wasserstoff stehen,
und
R⁶ und R⁷ zusammen mit dem Kohlenstoffatom, an das sie gebunden sind, eine Carbonylgruppe bilden,
oder
R⁴ und R⁵ zusammen mit dem Kohlenstoffatom, an das sie gebunden sind, eine Carbonylgruppe bilden,
und
R⁶ und R⁷ für Wasserstoff stehen,
oder
R⁴ und R⁵ zusammen mit dem Kohlenstoffatom, an das sie gebunden sind, eine Carbonylgruppe bilden,
und
R⁶ und R⁷ zusammen mit dem Kohlenstoffatom, an das sie gebunden sind, eine Carbonylgruppe bilden,
R⁸ für eine Gruppe der Formel 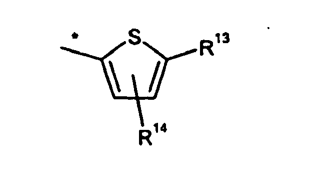 steht,
wobei
* die Anknüpfstelle an die Carbonylgruppe ist,
R¹³ für Chlor steht,
und
R¹⁴ für Wasserstoff steht,
R⁹ für Wasserstoff steht,
R¹⁰ für Wasserstoff steht,
und
wobei R⁹ an die 6-Position und R¹⁰ an die 7-Position des Isoindolin-Rings gebunden ist, oder
wobei R⁹ an die 7-Position und R¹⁰ an die 6-Position des Isoindolin-Rings gebunden ist.

5. Verfahren zur Herstellung einer Verbindung der Formel (I) oder eines ihrer Salze, ihrer Solvate oder der Solvate ihrer Salze nach Anspruch 1, **dadurch gekennzeichnet, dass**
[A] eine Verbindung der Formel 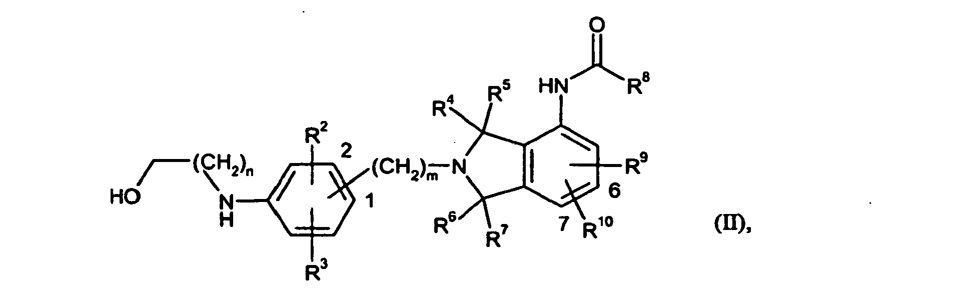 in welcher n, m, R², R³, R⁴, R⁵, R⁶, R⁷, R⁸, R⁹ und R¹⁰ die in Anspruch 1 angegebene Bedeutung haben,
in einem inerten Lösungsmittel in Gegenwart einer Säure mit Bromcyan zu einer Verbindung der Formel (I), in welcher R¹ für Wasserstoff steht, umgesetzt wird,
oder
[B] eine Verbindung der Formel 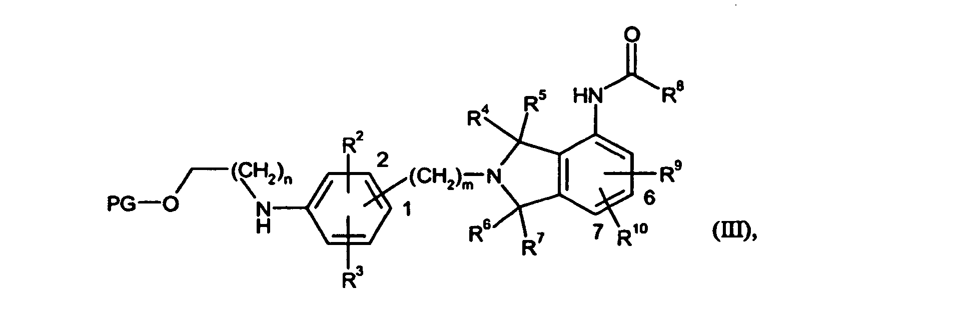 in welcher n, m, R², R³, R⁴, R⁵, R⁶, R⁷, R⁸, R⁹ und R¹⁰ die in Anspruch 1 angegebene Bedeutung haben, und
PG für eine Hydroxy-Schutzgruppe, vorzugsweise für Trimethylsilyl oder *tert*.-Butyldimethylsilyl, steht,
in einem dreistufigen Verfahren zuerst in einem inerten Lösungsmittel mit Bromcyan, vorzugsweise in Gegenwart einer Base, zu einer Verbindung der Formel 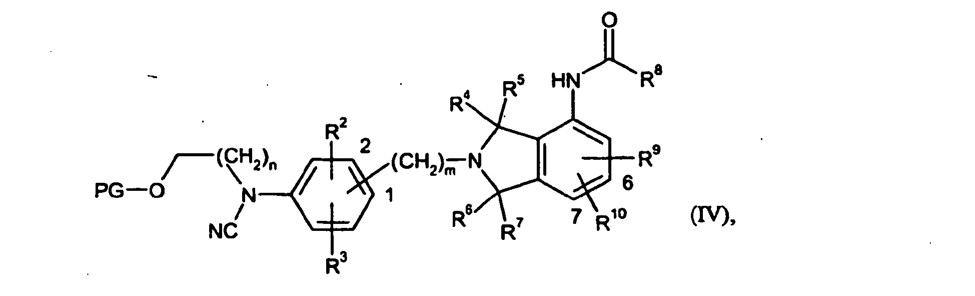 in welcher n, m, R², R³, R⁴, R⁵, R⁶, R⁷, R⁸, R⁹ und R¹⁰ die in Anspruch 1 angegebene Bedeutung haben, und
PG für eine Hydroxy-Schutzgruppe, vorzugsweise für Trimethylsilyl oder tert.-Butyldimethylsilyl, steht,
und anschließend durch Abspaltung der Schutzgruppe PG zu einer Verbindung der Formel 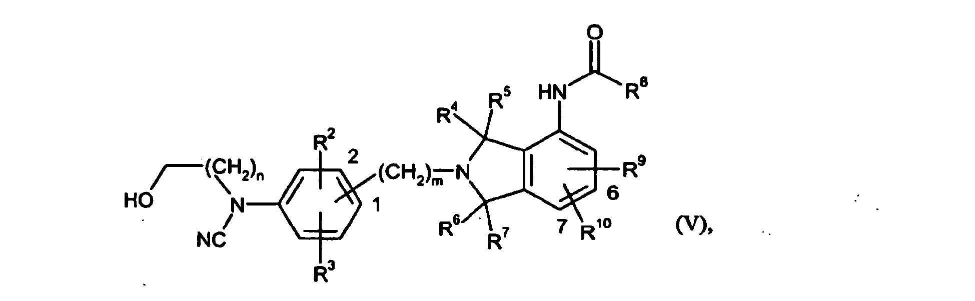 in welcher n, m, R², R³, R⁴, R⁵, R⁶, R⁷, R⁸, R⁹ und R¹⁰ die in Anspruch 1 angegebene Bedeutung haben,
umgesetzt wird und in der dritten Stufe die Verbindung der Formel (V) in einem inerten Lösungsmittel in Gegenwart einer Säure zu einer Verbindung der Formel (I), in welcher R¹ für Wasserstoff steht, cyclisiert wird,
oder
[C] die Verbindung der Formel (II) in der ersten Stufe mit einer Verbindung der Formel 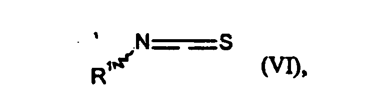 in welcher
R¹ für C₁-C₄-Alkyl, C₁-C₄-Alkylcarbonyl, C₃-C₆-Cycloalkylcarbonyl, Phenylcarbonyl, 4- bis 7-gliedriges Heterocyclylcarbonyl oder 5- oder 6-gliedriges Heteroarylcarbonyl steht,
umgesetzt wird und in der zweiten Stufe cyclisiert wird, oder
[D] die Verbindung der Formel (II) mit einer Verbindung der Formel 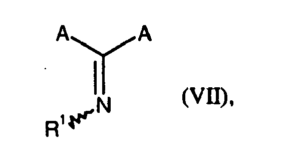 in welcher
R¹ für Cyano oder C₁-C₄-Alkyl steht, und
A für eine Abgangsgruppe, bevorzugt Phenoxy oder Methylthio, steht,
umgesetzt wird, oder
[E] die Verbindung der Formel (I), in welcher R¹ für Wasserstoff steht, mit Hydroxylamin-Hydrochlorid zu einer Verbindung der Formel (I), in welcher R¹ für Hydroxy steht, umgesetzt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von Krankheiten.

8. Verwendung einer Verbindung nach einem der Ansprüche 1 bis 4 zur Herstellung eines Arzneimittels zur Behandlung und/oder Prophylaxe von thromboembolischen Erkrankungen.

9. Arzneimittel enthaltend eine Verbindung nach einem der Ansprüche 1 bis 4 in Kombination mit einem inerten, nichttoxischen, pharmazeutisch geeigneten Hilfsstoff.

10. Arzneimittel enthaltend eine Verbindung nach einem der Ansprüche 1 bis 4 in Kombination mit einem weiteren Wirkstoff.

11. Arzneimittel nach Anspruch 9 oder 10 zur Behandlung und/oder Prophylaxe von thromboembolischen Erkrankungen.

## Claims

1. Compound of the formula 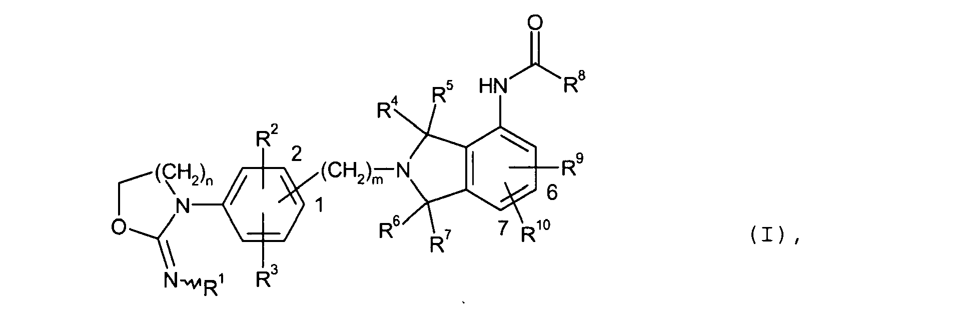 in which
n represents the number 1, 2 or 3,
m represents the number 0, 1 or 2, and the (CH₂)ₘ group is attached in the 1- or 2-position to the phenyl ring,
R¹ represents hydrogen, cyano, hydroxy, C₁-C₄- alkyl, C₁-C₄-alkylcarbonyl, C₃-C₆- cycloalkylcarbonyl, phenylcarbonyl, 4- to 7- branched heterocyclylcarbonyl or 5- or 6- branched heteroarylcarbonyl,
R² represents hydrogen, fluorine, chlorine, cyano, hydroxy, amino, trifluoromethyl, trifluoromethoxy, C₁-C₄-alkyl, C₁-C₄-alkoxy, C₁-C₄-alkoxymethyl, C₁-C₄-alkylamino, C₃-C₆-
R³ cycloalkyl, aminocarbonyl, C₁-C₄- alkoxycarbonyl or C₁-C₄-alkylaminocarbonyl, represents hydrogen, fluorine, chlorine, cyano, hydroxy, amino, trifluoromethyl, trifluoromethoxy, C₁-C₄-alkyl, C₁-C₄-alkoxy, C₁-C₄-alkoxymethyl, C₁-C₄-alkylamino, C₃-C₆- cycloalkyl, aminocarbonyl, C₁-C₄- alkoxycarbonyl or C₁-C₄-alkylaminocarbonyl,
R⁴ and R⁵ represent hydrogen,
and
R⁶ and R⁷ together with the carbon atom to which they are attached form a carbonyl group,
or
R⁴ and R⁵ together with the carbon atom to which they are attached form a carbonyl group,
and
R⁶ and R⁷ represent hydrogen,
or
R⁴ and R⁵ together with the carbon atom to which they are attached form a carbonyl group,
and
R⁶ and R⁷ together with the carbon atom to which they are attached form a carbonyl group,
R⁸ represents phenyl, pyridyl, pyrazinyl, pyrimidinyl, pyridazinyl or thienyl,
where phenyl, pyridyl, pyrazinyl, pyrimidinyl and pyridazinyl are substituted by a substituent R¹¹ and/or a substituent R¹² or by two different different substituents R¹¹ or by two substituents R¹²,
where
R¹¹ is attached to a carbon atom which is not adjacent to a nitrogen atom in the ring and represents hydrogen, fluorine, chlorine, cyano, ethynyl, C₁-C₄-alkyl, C₁-C₄-alkoxy or C₃-C₆- cycloalkyl,
R¹² is attached to a carbon atom which is adjacent to a nitrogen atom in the ring and represents hydrogen, amino, C₁-C₄-alkyl, C₁-C₄-alkylamino or C₃-C₆-cycloalkyl,
and
where thienyl is substituted by a substituent R¹³ and a substituent R¹⁴,
where
R¹³ is attached to a carbon atom which is adjacent to the sulphur atom in the ring and represents hydrogen, fluorine, chlorine, cyano, ethynyl, C₁-C₄-alkyl, C₁-C₄-alkoxy or C₃-C₆- cycloalkyl,
R¹⁴ represents hydrogen, fluorine, chlorine, amino, C₁-C₄-alkyl, C₁-C₄- alkylamino or C₃-C₆-cycloalkyl,
R⁹ represents hydrogen, fluorine, chlorine, cyano, hydroxy, amino, trifluoromethyl, trifluoromethoxy, C₁-C₄-alkyl, C₁-C₄-alkoxy, C₁-C₄-alkylamino, C₃-C₆-cycloalkyl, aminocarbonyl, C₁-C₄-alkoxycarbonyl or C₁-C₄- alkylaminocarbonyl,
R¹⁰ represents hydrogen, fluorine, chlorine, cyano, trifluoromethyl, trifluoromethoxy, C₁- C₄-alkyl, C₁-C₄-alkoxy or C₃-C₆-cycloalkyl,
and
where R⁹ is attached to the 6-position and R¹⁰ is attached to the 7-position of the isoindoline ring,
or
where R⁹ is attached to the 7-position and R¹⁰ is attached to the 6-position of the isoindoline ring, or one of its salts, its solvates or the solvates of its salts.

2. Compound according to Claim 1, **characterized in that**
n represents the number 1, 2 or 3,
m represents the number 0, 1 or 2, and the (CH₂)ₘ group is attached in the 1- or 2-position to the phenyl ring,
R¹ represents hydrogen, cyano, hydroxy or C₁-C₄- alkyl,
R² represents hydrogen, fluorine, chlorine, cyano, hydroxy, C₁-C₄-alkyl or C₁-C₄-alkoxy,
R³ represents hydrogen, fluorine, chlorine, cyano, hydroxy, C₁-C₄-alkyl, C₁-C₄-alkoxy, C₁- C₄-alkoxymethyl, cyclopropyl, aminocarbonyl, C₁-C₄-alkoxycarbonyl or C₁-C₄- alkylaminocarbonyl,
R⁴ and R⁵ represent hydrogen,
and
R⁶ and R⁷ together with the carbon atom to which they are attached form a carbonyl group,
or
R⁴ and R⁵ together with the carbon atom to which they are attached form a carbonyl group,
and
R⁶ and R⁷ represent hydrogen, or
R⁴ and R⁵ together with the carbon atom to which they are attached form a carbonyl group,
and
R⁶ and R⁷ together with the carbon atom to which they are attached form a carbonyl group,
R⁸ represents a group of the formula 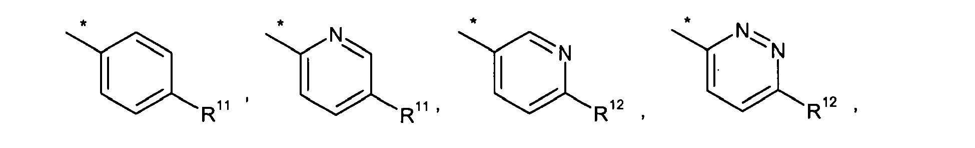 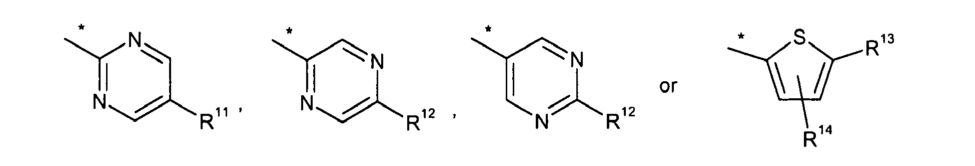
* is the point of attachment to the carbonyl group,
R¹¹ represents hydrogen, represents fluorine, chlorine, ethynyl, methyl, ethyl, methoxy or ethoxy,
R¹² represents amino, methyl, methylamino or dimethylamino,
R¹³ represents fluorine, chlorine, ethynyl, methyl, ethyl, methoxy or ethoxy,
and
R¹⁴
R⁹ represents hydrogen, fluorine, chlorine, cyano, methyl, methoxy, aminocarbonyl, methylaminocarbonyl or dimethylaminocarbonyl,
R¹⁰ represents hydrogen, fluorine, chlorine, cyano, trifluoromethyl, trifluoromethoxy, methyl or methoxy,
and
where R⁹ is attached to the 6-position and R¹⁰ to the 7-position of the isoindoline ring,
or
where R⁹ is attached to the 7-position and R¹⁰ to the 6-position of the isoindoline ring. where

3. Compound according to Claim 1 or 2, **characterized in that**
n represents the number 1 or 2,
m represents the number 1, and the (CH₂)ₘ group is attached in the 1- or 2-position to the phenyl ring,
R¹ represents hydrogen,
R² represents hydrogen,
R³ represents hydrogen, fluorine, chlorine, cyano, methyl, ethyl, n-propyl, methoxy, ethoxy or methoxymethyl,
R⁴ and R⁵ represent hydrogen,
and
R⁶ and R⁷ together with the carbon atom to which they are attached form a carbonyl group,
or
R⁴ and R⁵ together with the carbon atom to which and they are attached form a carbonyl group,
R⁶ and R⁷ represent hydrogen,
or
R⁴ and R⁵ together with the carbon atom to which and they are attached form a carbonyl group,
R⁶ and R⁷ together with the carbon atom to which they are attached form a carbonyl group,
R⁸ represents a group of the formula 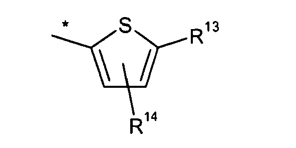 where
* is the point of attachment to the carbonyl group,
R¹³ and represents fluorine, chlorine or methyl,
and
R¹⁴ represents hydrogen,
R⁹ represents hydrogen,
R¹⁰ represents hydrogen,
and
where R⁹ is attached to the 6-position and R¹⁰ to the 7-position of the isoindoline ring,
or
where R⁹ is attached to the 7-position and R¹⁰ to the 6-position of the isoindoline ring.

4. Compound according to any of Claims 1 to 3, **characterized in that**
n represents the number 1,
m represents the number 1, and the (CH₂)ₘ group is attached in the 1- or 2-position to the phenyl ring,
R¹ represents hydrogen,
R² represents hydrogen,
R³ represents hydrogen, fluorine, chlorine, cyano or methyl,
R⁴ and R⁵ represent hydrogen,
and
R⁶ and R⁷ together with the carbon atom to which they are attached form a carbonyl group,
or
R⁴ and R⁵ together with the carbon atom to which they are attached form a carbonyl group,
and
R⁶ and R⁷ represent hydrogen,
or
R⁴ and R⁵ together with the carbon atom to which they are attached form a carbonyl group,
and
R⁶ and R⁷ together with the carbon atom to which they are attached form a carbonyl group,
R⁸ represents a group of the formula 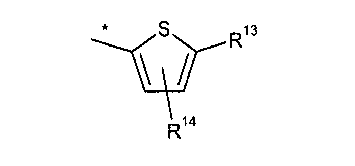 where
* is the point of attachment to the carbonyl group,
R¹³ represents chlorine,
and
R¹⁴ represents hydrogen,
R⁹ represents hydrogen,
R¹⁰ represents hydrogen,
and
where R⁹ is attached to the 6-position and R¹⁰ to the 7-position of the isoindoline ring,
or
where R⁹ is attached to the 7-position and R¹⁰ to the 6-position of the isoindoline ring.

5. Process for preparing a compound of the formula (I) or one of its salts, its solvates or the solvates of its salts according to Claim 1, **characterized in that**
[A] a compound of the formula 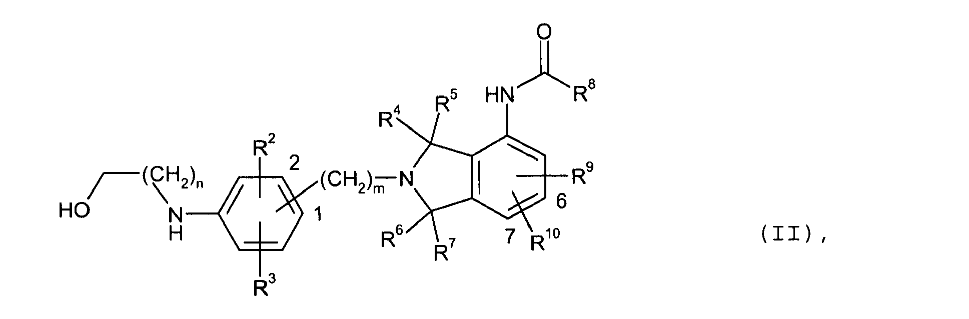 in which n, m, R², R³, R⁴, R⁵, R⁶, R⁷, R⁸, R⁹ and R¹⁰ have the meaning given in Claim 1,
is reacted with cyanogen bromide in an inert solvent in the presence of an acid to form a compound of the formula (I), in which R¹ represents hydrogen,
or
[B] a compound of the formula 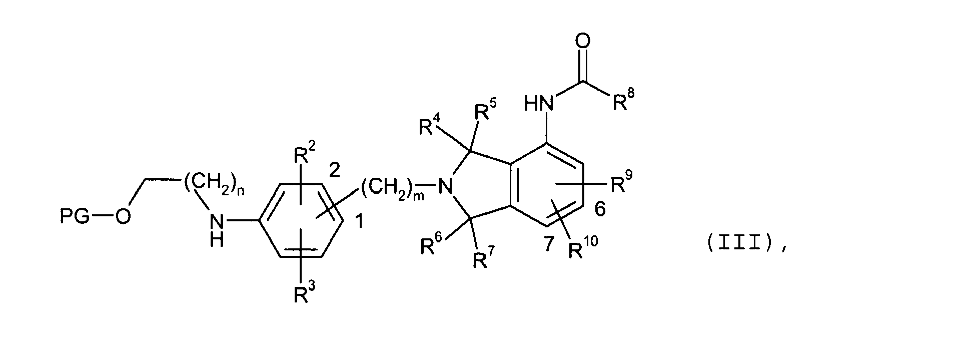 in which n, m, R², R³, R⁴, R⁵, R⁶, R⁷, R⁸, R⁹ and R¹⁰ have the meaning given in Claim 1, and
PG represents a hydroxyl protective group, preferably trimethylsilyl or *tert*- butyldimethylsilyl,
is converted in a three-step process initially in an inert solvent with cyanogen bromide, preferably in the presence of a base, into a compound of the formula 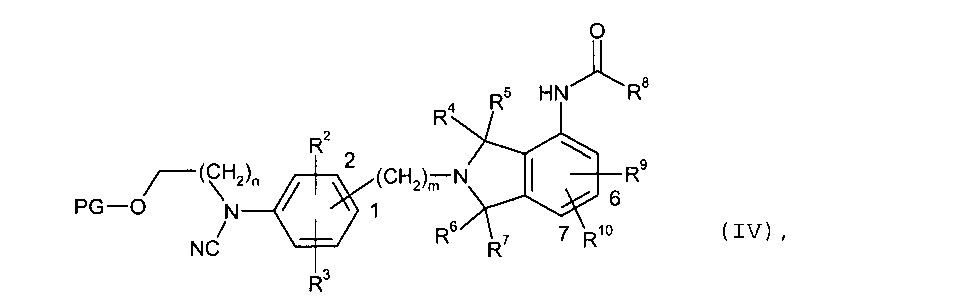 in which n, m, R², R³, R⁴, R⁵, R⁶, R⁷, R⁸, R⁹ and R¹⁰ have the meaning given in Claim 1, and
PG represents a hydroxyl protective group, preferably trimethylsilyl or *tert*- butyldimethylsilyl,
and then by removal of the protective group PG into a compound of the formula 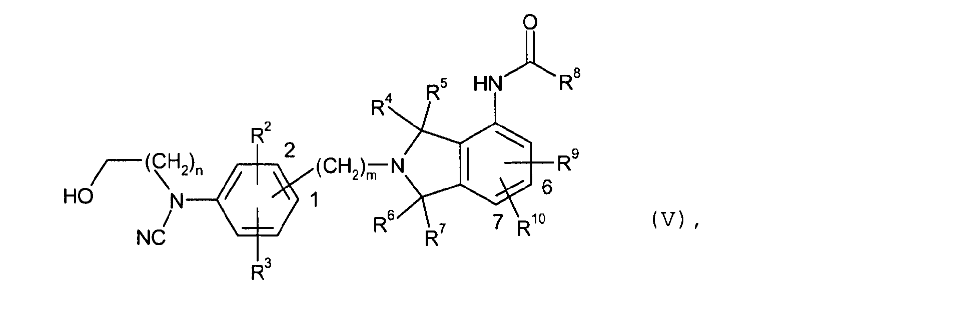 in which n, m, R², R³, R⁴, R⁵, R⁶, R⁷, R⁸, R⁹ and R¹⁰ have the meaning given in Claim 1,
and in the third step the compound of the formula (V) is cyclized in an inert solvent in the presence of an acid to a compound of the formula (I) in which R¹ represents hydrogen,
or
[C] the compound of the formula (II) is, in the first step, reacted with a compound of the formula 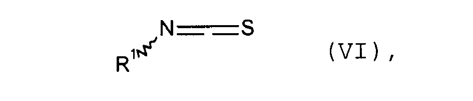 in which
R¹ represents C₁-C₄-alkyl, C₁-C₄-alkylcarbonyl, C₃-C₆-cycloalkylcarbonyl, phenylcarbonyl, 4- to 7-membered heterocyclylcarbonyl or 5- or 6-membered heteroarylcarbonyl,
and, in the second step, cyclized,
or
[D] the compound of the formula (II) is reacted with a compound of the formula 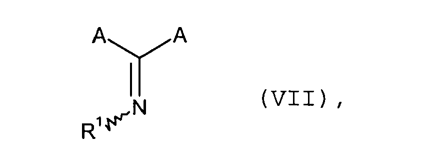 in which
R¹ represents cyano or C₁-C₄-alkyl and
A represents a leaving group, preferably phenoxy or methylthio,
or
[E] the compound of the formula (I) in which R¹ represents hydrogen is reacted with hydroxylamine hydrochloride to give a compound of the formula (I) in which R¹ represents hydroxyl.

6. Compound according to any of Claims 1 to 4 for the treatment and/or prophylaxis of diseases.

7. Use of a compound according to any of Claims 1 to 4 for preparing a medicament for the treatment and/or prophylaxis of diseases.

8. Use of a compound according to any of Claims 1 to 4 for preparing a medicament for the treatment and/or prophylaxis of thromboembolic disorders.

9. Medicament comprising a compound according to any of Claims 1 to 4 in combination with an inert nontoxic pharmaceutically acceptable auxiliary.

10. Medicament comprising a compound according to any of Claims 1 to 4 in combination with a further active compound.

11. Medicament according to Claim 9 or 10 for the treatment and/or prophylaxis of thromboembolic disorders.

## Revendications

1. Composé de formule 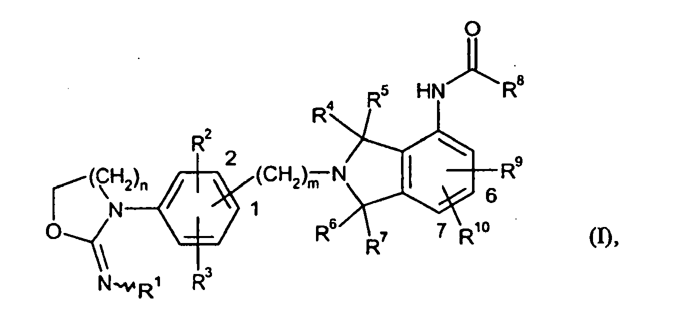 où
n représente le chiffre 1, 2 ou 3,
m représente le chiffre 0, 1 ou 2, et le groupe (CH₂)ₘ est lié à la position 1 ou 2 au cycle phényle,
R¹ représente hydrogène, cyano, hydroxy, alkyle en C₁-C₄, (alkyle en C₁-C₄)carbonyle, (cycloalkyle en C₃-C₆)carbonyle, phénylcarbonyle, hétérocyclylcarbonyle à 4 à 7 chaînons ou hétéroarylcarbonyle à 5 ou 6 chaînons,
R² représente hydrogène, fluor, chlore, cyano, hydroxy, amino, trifluorométhyle, trifluorométhoxy, alkyle en C₁-C₄, alcoxy en C₁-C₄, (alcoxy en C₁-C₄)méthyle, (alkyle en C₁-C₄)amino, cycloalkyle en C₃- C₆, aminocarbonyle, (alcoxy en C₁-C₄)carbonyle ou (alkyle en C₁-C₄)- aminocarbonyle,
R³ représente hydrogène, fluor, chlore, cyano, hydroxy, amino, trifluorométhyle, trifluorométhoxy, alkyle en C₁-C₄, alcoxy en C₁-C₄, (alcoxy en C₁-C₄)méthyle, (alkyle en C₁-C₄)amino, cycloalkyle en C₃- C₆, aminocarbonyle, (alcoxy en C₁-C₄)carbonyle ou (alkyle en C₁-C₄)- aminocarbonyle,
R⁴ et R⁵ représentent hydrogène
et
R⁶ et R⁷ forment un groupe carbonyle avec l'atome de carbone auquel ils sont liés,
ou
R⁴ et R⁵ forment un groupe carbonyle avec l'atome de carbone auquel ils sont liés et
R⁶ et R⁷ représentent hydrogène,
ou
R⁴ et R⁵ forment un groupe carbonyle avec l'atome de carbone auquel ils sont liés,
et
R⁶ et R⁷ forment un groupe carbonyle avec l'atome de carbone auquel ils sont liés,
R⁸ représente phényle, pyridyle, pyrazinyle, pyrimidinyle, pyridazinyle ou thiényle, où phényle, pyridyle, pyrazinyle, pyrimidinyle et pyridazinyle sont substitués par un substituant R¹¹ et/ou un substituant R¹² ou par deux substituants R¹¹ différents ou par deux substituants R¹² différents, étant entendu que
R¹¹ est lié à un atome de carbone qui n'est pas voisin d'un atome d'azote dans le cycle et représente hydrogène, fluor, chlore, cyano, éthinyle, alkyle en C₁-C₄, alcoxy en C₁-C₄ ou cycloalkyle en C₃-C₆,
R¹² est lié à un atome de carbone qui est proche d'un atome d'azote dans le cycle et représente hydrogène, amino, alkyle en C₁-C₄, (alkyle en C₁-C₄)amino ou cycloalkyle en C₃-C₆,
et
où thiényle est substitué par un substituant R¹³ et un substituant R¹⁴, étant entendu que
R¹³ est lié à un atome de carbone qui est proche de l'atome de soufre dans le cycle et représente hydrogène, fluor, chlore, cyano, éthinyle, alkyle en C₁-C₄, alcoxy en C₁-C₄ ou cyclo- alkyle en C₃-C₆,
R¹⁴ représente hydrogène, fluor, chlore, amino, alkyle en C₁-C₄, (alkyle en C₁-C₄)amino ou cycloalkyle en C₃-C₆, R⁹ représente hydrogène, fluor, chlore, cyano, hydroxy, amino, trifluorométhyle, trifluorométhoxy, alkyle en C₁-C₄, alcoxy en C₁-C₄, (alkyle en C₁-C₄)amino, cycloalkyle en C₃-C₆, aminocarbonyle, (alcoxy en C₁-C₄)carbonyle ou (alkyle en C₁-C₄)aminocarbonyle,
R¹⁰ représente hydrogène, fluor, chlore, cyano, trifluorométhyle, trifluorométhoxy, alkyle en C₁-C₄, alcoxy en C₁-C₄ ou cycloalkyle en C₃-C₆,
et
où R⁹ est lié à la position 6 et R¹⁰ à la position 7 du cycle isoindoline, ou
où R⁹ est lié à la position 7 et R¹⁰ à la position 6 du cycle isoindoline, ou un de ses sels, ses solvates ou des solvates de ses sels.

2. Composé selon la revendication 1, **caractérisé en ce que**
n représente le chiffre 1, 2 ou 3,
m et représente le chiffre 0, 1 ou 2, le groupe (CH₂)ₘ est lié à la position 1 ou 2 au cycle phényle,
R¹ représente hydrogène, cyano, hydroxy ou alkyle en C₁-C₄,
R² ou représente hydrogène, fluor, chlore, cyano, hydroxy, alkyle en C₁-C₄ alcoxy en C₁-C₄,
R³ représente hydrogène, fluor, chlore, cyano, hydroxy, alkyle en C₁-C₄, alcoxy en C₁-C₄, (alcoxy en C₁-C₄)méthyle, cyclopropyle, aminocarbonyle, (alcoxy en C₁-C₄)carbonyle ou (alkyle en C₁-C₄)- aminocarbonyle,
R⁴ et R⁵ représentent hydrogène et
R⁶ et R⁷ forment un groupe carbonyle avec l'atome de carbone auquel ils sont liés,
ou
R⁴ et R⁵ forment un groupe carbonyle avec l'atome de carbone auquel ils sont liés et
R⁶ et R⁷ représentent hydrogène,
ou
R⁴ et R⁵ forment un groupe carbonyle avec l'atome de carbone auquel ils sont liés,
et
R⁶ et R⁷ forment un groupe carbonyle avec l'atome de carbone auquel ils sont liés,
R⁸ représente un groupe de formule 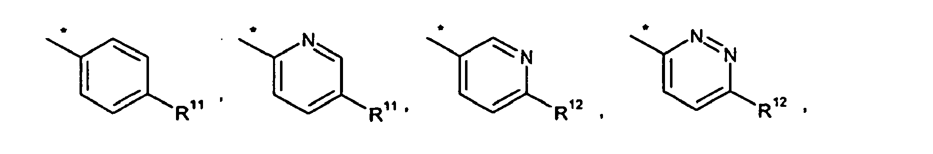 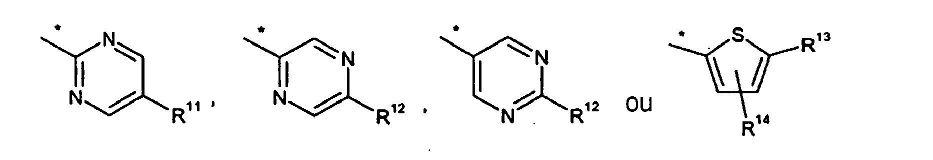 où
* est le site de liaison au groupe carbonyle,
R¹¹ représente fluor, chlore, éthinyle, méthyle, éthyle, méthoxy ou éthoxy,
R¹² représente amino, méthyle, méthylamino ou diméthylamino,
R¹³ représente fluor, chlore, éthinyle, méthyle, éthyle, méthoxy ou éthoxy,
et
R¹⁴ représente hydrogène,
R⁹ représente hydrogène, fluor, chlore, cyano, méthyle, méthoxy, aminocarbonyle, méthylaminocarbonyle ou diméthylaminocarbonyle,
R¹⁰ représente hydrogène, fluor, chlore, cyano, trifluorométhyle, trifluo- rométhoxy, méthyle ou méthoxy,
et
où R⁹ et lié à la position 6 et R¹⁰ à la position 7 du cycle isoindoline,
ou
où R⁹ est lié à la position 7 et R¹⁰ à la position 6 du cycle isoindoline.

3. Composé selon l'une des revendications 1 ou 2, **caractérisé en ce que**
n représente le chiffre 1 ou 2,
m représente le chiffre 1, et le groupe (CH₂)ₘ est lié à la position 1 ou 2 sur le cycle phényle,
R¹ représente hydrogène,
R² représente hydrogène,
R³ représente hydrogène, fluor, chlore, cyano, méthyle, éthyle, n- propyle, méthoxy, éthoxy ou méthoxyméthyle,
R⁴ et R⁵ et représentent hydrogène
R⁶ et R⁷ ou forment un groupe carbonyle avec l'atome de carbone auquel ils sont liés,
R⁴ et R⁵ forment un groupe carbonyle avec l'atome de carbone auquel ils sont liés,
et
R⁶ et R⁷ représentent hydrogène,
où
R⁴ et et R⁵ forment un groupe carbonyle avec l'atome de carbone auquel ils sont liés,
R⁶ et R⁷ forment un groupe carbonyle avec l'atome de carbone auquel ils sont liés,
R⁸ représente un groupe de formule 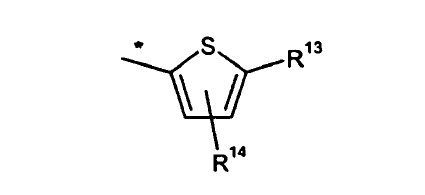 où
* lest e site de liaison au groupe carbonyle,
R¹³ représente fluor, chlore ou méthyle,
et
R¹⁴ représente hydrogène,
R⁹ représente hydrogène,
R¹⁰ représente hydrogène,
et
où R⁹ est lié à la position 6 et R¹⁰ à la position 7 du cycle isoindoline, ou
où R⁹ est lié à la position 7 et R¹⁰ à la position 6 du cycle isoindoline.

4. Composé selon une des revendications 1 à 3, **caractérisé en**
**ce que**
n représente le chiffre 1,
m représente le chiffre 1, et le groupe (CH₂)ₘ est lié à la position 1 ou 2 au cycle phényle,
R¹ représente hydrogène,
R² représente hydrogène,
R³ représente hydrogène, fluor, chlore, cyano ou méthyle,
R⁴ et R⁵ représentent hydrogène
et
R⁶ et R⁷ forment un groupe carbonyle avec l'atome de carbone auquel ils sont liés,
ou
R⁴ et R⁵ forment un groupe carbonyle avec l'atome de carbone auquel ils sont liés,
et
R⁶ et R⁷ représentent hydrogène,
où
R⁴ et R⁵ forment un groupe carbonyle avec l'atome de carbone auquel ils sont liés,
et
R⁶ et R⁷ sont forment un groupe carbonyle avec l'atome de carbone auquel ils liés,
R⁸ représente un groupe de formule 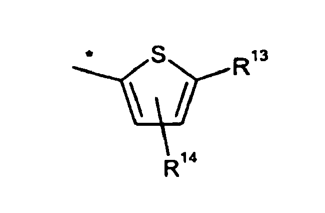 où
* est le site de liaison au groupe carbonyle,
R¹³ représente chlore,
et
R¹⁴ représente hydrogène,
R⁹ représente hydrogène,
R¹⁰ représente hydrogène,
et
où R⁹ est lié à la position 6 et R¹⁰ à la position 7 du cycle isoindoline,
ou
où R⁹ est lié à la position 7 et R¹⁰ à la position 6 du cycle isoindoline.

5. Procédé de préparation d'un composé de formule (I) ou d'un de ses sels, ses solvates ou des solvates de ses sels selon la revendication 1, **caractérisé en ce que**
on transforme
[A] un composé de formule 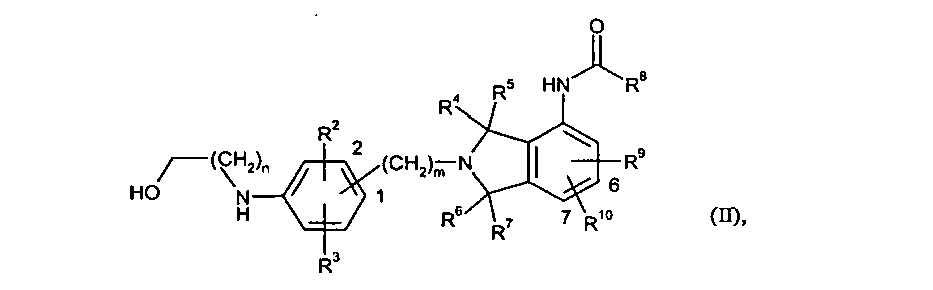 où n, m, R², R³, R⁴, R⁵, R⁶, R⁷, R⁸, R⁹ et R¹⁰ ont la signification indiquée à la revendication 1,
dans un solvant inerte en présence d'un acide avec du bromure de cyanogène en un composé de formule (I) dans laquelle R¹ représente l'hydrogène,
ou
on transforme
[B] un composé de formule 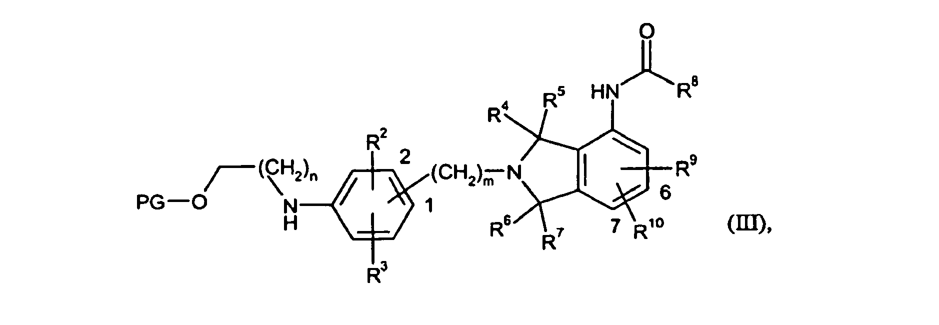 où n, m, R², R³, R⁴, R⁵, R⁶, R⁷, R⁸, R⁹ et R¹⁰ ont la signification indiquée à la revendication 1 et
PG représente un groupe protecteur d'hydroxy, de préférence triméthylsilyle ou *tert*-butyldiméthylsilyle,
selon un procédé en trois étapes tout d'abord dans un solvant inerte avec du bromure de cyanogène, de préférence en présence d'une base, en un composé de formule 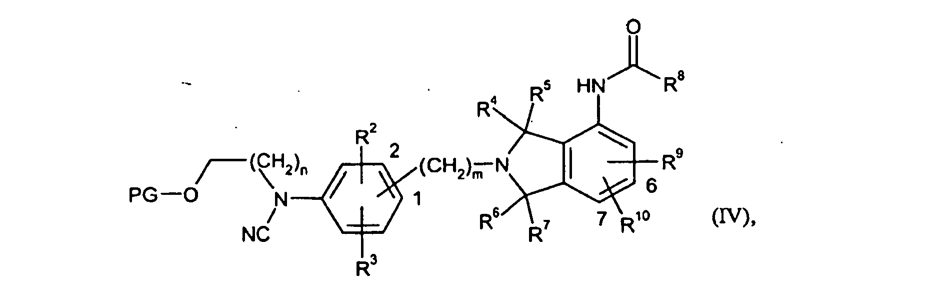 où n, m, R², R³, R⁴, R⁵, R⁶, R⁷, R⁸, R⁹ et R¹⁰ ont la signification indiquée à la revendication 1 et
PG représente un groupe protecteur d'hydroxy, de préférence triméthylsilyle ou *tert*-butyldiméthylsilyle,
et ensuite par séparation du groupe protecteur PG en un composé de formule 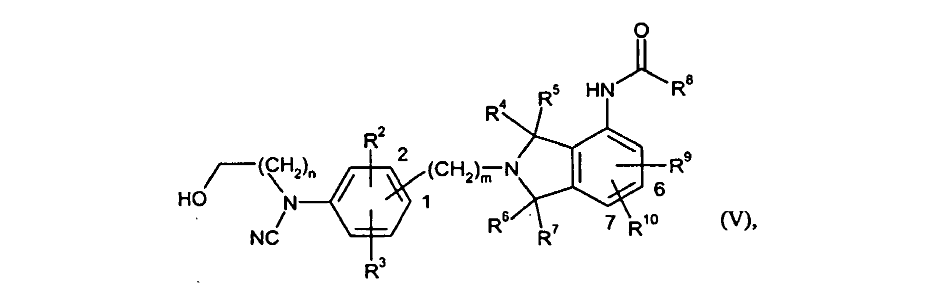 où n, m, R², R³, R⁴, R⁵, R⁶, R⁷, R⁸, R⁹ et R¹⁰ ont la signification indiquée à la revendication 1,
et au cours de la troisième étape on cyclise le composé de formule (V) dans un solvant inerte en présence d'un acide en un composé de formule (I) dans laquelle R¹ représente hydrogène,
ou
on transforme
[C] le composé de formule (II) au cours de la première étape avec un composé de formule 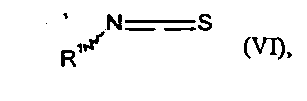 où
R¹ représente alkyle en C₁-C₄, (alkyle en C₁-C₄)carbonyle, (cycloalkyle en C₃-C₆)carbonyle, phénylcarbonyle, hétérocyclylcarbonyle à 4 à 7 chaînons ou hétéroarylcarbonyle à 5 ou 6 chaînons,
et on le cyclise au cours de la seconde étape,
ou
on transforme
[D] le composé de formule (II) avec un composé de formule 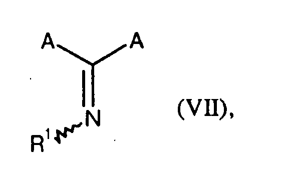 où
R¹ représente cyano ou alkyle en C₁-C₄, et
A représente un groupe qui part, de préférence phénoxy ou méthylthio,
ou
on transforme
[E] le composé de formule (I) dans laquelle R¹ représente hydrogène, avec du chlorhydrate d'hydroxylamine en un composé de formule (I) dans laquelle R¹ représente hydroxy.

6. Composé selon une des revendications 1 à 4 destiné au traitement et/ou la prévention de maladies.

7. Utilisation d'un composé selon une des revendications 1 à 4 pour la préparation d'un médicament destiné au traitement et/ou à la prévention de maladies.

8. Utilisation d'un composé selon une des revendications 1 à 4 pour la préparation d'un médicament destiné au traitement et/ou à la prévention de maladies thromboemboliques.

9. Médicament contenant un composé selon une des revendications 1 à 4 en combinaison avec un adjuvant pharmaceutiquement approprié, non toxique, inerte.

10. Médicament contenant un composé selon une des revendications 1 à 4 en association avec un autre principe actif.

11. Médicament selon la revendication 9 ou 10 destiné au traitement et/ou à la prévention de maladies thromboemboliques.
